(19) Europäisches Patentamt
European Patent Office
Office européen des brevets

(11) EP 4 190 819 A1

(12) EUROPEAN PATENT APPLICATION
published in accordance with Art. 153(4) EPC

(43) Date of publication:
07.06.2023 Bulletin 2023/23

(21) Application number: 22766354.9

(22) Date of filing: 10.03.2022

(51) International Patent Classification (IPC):
C07K 16/46 (2006.01)        C07K 16/30 (2006.01)
A61K 39/395 (2006.01)       A61P 35/00 (2006.01)

(52) Cooperative Patent Classification (CPC):
A61K 39/395; A61K 45/06; A61P 35/00;
C07K 16/30; C07K 16/46

(86) International application number:
PCT/CN2022/080107

(87) International publication number:
WO 2022/188832 (15.09.2022 Gazette 2022/37)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 12.03.2021 CN 202110270652

(71) Applicant: Akeso Biopharma, Inc.
Zhongshan, Guangdong 528437 (CN)

(72) Inventors:
• WANG, Zhongmin
Zhongshan, Guangdong 528437 (CN)
• LI, Baiyong
Zhongshan, Guangdong 528437 (CN)
• XIA, Yu
Zhongshan, Guangdong 528437 (CN)

(74) Representative: Cooley (UK) LLP
22 Bishopsgate
London EC2N 4BQ (GB)

Remarks:
The complete document including Reference
Table(s) and the Sequence Listing(s) can be
downloaded from the EPO website

(54) **PHARMACEUTICAL COMBINATION CONTAINING ANTI-PD-1-ANTI-VEGFA BISPECIFIC ANTIBODY, AND USE THEREOF**

(57)    The present invention relates to the field of tumor treatment and immunology biology. Provided are a pharmaceutical combination containing an anti-PD-1-anti-VEGFA bispecific antibody, and the use thereof. Specifically, the pharmaceutical combination comprises at least one bispecific antibody and at least one PARP inhibitor, wherein the bispecific antibody comprises a first protein functional region for targeting PD-1 and a second protein functional region for targeting VEGFA; and according to the EU numbering system, the heavy chain constant region of an immunoglobulin contained in the bispecific antibody is mutated at two sites, i.e. site 234 and site 235, and after mutation, the affinity constant of the bispecific antibody to FcγRI, FcγRIIa, FcγRIIIa and/or Clq is reduced compared with the affinity constant of the bispecific antibody thereto before mutation. The combined administration of the PARPi and the bispecific antibody has a significantly better therapeutic effect on tumors than the individual use of the PARPi or the bispecific antibody; and the present invention has good application prospects.

FIG. 38

**Description**

TECHNICAL FIELD

The present invention relates to the fields of tumor treatment and immunobiology, and particularly to a therapeutic combination comprising an anti-PD-1/anti-VEGFA bispecific antibody and use thereof. Particularly, the present invention relates to a therapeutic combination comprising an anti-human PD-1/anti-human VEGFA bispecific antibody and use thereof.

BACKGROUND

Tumors, especially malignant tumors, are serious health-threatening diseases in the world today. There are two distinct stages of tumor growth, namely, from a slow growth stage without blood vessels to a rapid proliferation stage with blood vessels. Angiogenesis enables the tumor to acquire enough nutrition to complete the blood vessel switching stage, and without angiogenesis, the size of the primary tumor will not exceed 1-2 mm, and thus the metastasis may not occur.

Vascular endothelial growth factor (VEGF) is a growth factor that can promote division and proliferation of endothelial cells, promote formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of tumor, improve vascular permeability, cause fibrin deposition in surrounding tissues, and promote infiltration of mononuclear cells, fibroblasts and endothelial cells, which facilitates the formation of tumor stroma and entry of tumor cells into new blood vessels, and promote tumor metastasis. Therefore, inhibiting tumor angiogenesis is considered as one of the most promising tumor treatment methods at present. The VEGF family includes VEGFA, VEGFB, VEGFC, VEGFD and PIGF. Vascular endothelial growth factor receptors (VEGFRs) include VEGFR1 (also known as Flt1), VEGFR2 (also known as KDR or Flk1), VEGFR3 (also known as Flt4) and Neuropilin-1 (NRP-1). The first three receptors are members of the tyrosine kinase superfamily, and are of similar structures composed of an extramembrane region, a transmembrane segment and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors. VEGFA mainly acts in combination with VEGFR1, VEGFR2 and NRP-1. VEGFR1 is the first recognized receptor, and has a higher affinity for VEGFA than VEGFR2 under normal physiological conditions but a lower tyrosinase activity in the intracellular segment than VEGFR2 (Ma Li, Chinese Journal of Birth Health and Heredity, 24(5) (2016):146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and other progresses of vascular endothelial cells, as well as the formation process of blood vessels and the permeability of blood vessels (Roskoski R Jr. et al., Crit Rev Oncol Hematol, 62(3) (2007):179-213). VEGFA, after binding to VEGFR2, mediates the transcription and expression of related intracellular protein genes through the downstream PLC-$\gamma$-PKC-Raf-MEK-MAPK signaling pathway, and thus promotes the proliferation of vascular endothelial cells (Takahashi T et al., Oncogene, 18(13) (1999):2221-2230).

VEGFR3 is one of the tyrosine kinase family members, and is mainly expressed in embryonic vascular endothelial cells and mature lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein, and is incapable of independently transducing biological signals but able to mediate signaling only after forming a complex with a VEGF tyrosine kinase receptor. (Ma Li, Chinese Journal of Birth Health and Heredity, 24(5) (2016):146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase, infiltration to peripheral tissues and other patterns of endothelial cells (Dong Hongchao et al., Sep. 2014, Journal of Modern Oncology, 22(9): 2231-3).

The programmed cell death receptor-1 (PD-1), also known as CD279, is a type I transmembrane glycoprotein membrane surface receptor and a member of the CD28 immunoglobulin superfamily, and is commonly expressed in T cells, B cells and myeloid cells. PD-1 has two natural ligands, PD-L1 and PD-L2. Both PD-L1 and PD-L2 are members of the B7 superfamily and are constitutively or inducibly expressed on the membrane surface of a variety of cells, including non-hematopoietic cells and a variety of tumor cells. PD-L1 is mainly expressed on T cells, B cells, DC and microvascular endothelial cells and a variety of tumor cells, while PD-L2 is expressed only on antigen presenting cells such as dendritic cells and macrophages. The interaction between PD-1 and its ligands can inhibit the activation of lymph, the proliferation of T cells and the secretion of cytokines such as IL-2 and IFN-$\gamma$.

A large number of researches have demonstrated that the tumor microenvironment can protect tumor cells from being damaged by immune cells, expression of PD-1 in lymphocytes infiltrating in the tumor microenvironment is up-regulated, and various primary tumor tissues are PD-L1 positive in immunohistochemical analysis, such as lung cancer, liver cancer, ovarian cancer, skin cancer, colon cancer and glioma. Meanwhile, the expression of PD-L1 in the tumor is significantly correlated with poor prognosis in cancer patients. Blocking the interaction between PD-1 and its ligands can promote the tumor-specific T cell immunity and enhance the immune elimination efficiency of tumor cells. A large number of clinical trials have shown that antibodies targeting PD-1 or PD-L1 can promote infiltration of CD8+ T cells into tumor tissues and up-regulate anti-tumor immune effector factors such as IL-2, IFN-$\gamma$, granzyme B and perforin, thereby effectively inhibiting the growth of tumors.

Due to the broad anti-tumor prospects and surprising efficacy of PD-1 antibodies, it is widely accepted in the industry that antibodies targeting the PD-1 pathway will bring breakthroughs in the treatment of a variety of tumors: for the treatment of non-small cell lung cancer, renal cell carcinoma, ovarian cancer and melanoma (Homet M. B., Parisi G., et al, Anti-PD-1 Therapy in Melanoma. Semin Oncol., Jun; 42(3) (2015):466-473), lymphoma and anemia (Held S.A., Heine A., et al, Advances in immunology of biliary muscular tissue CML. Curr. Cancer Drug Targets, Sep; 13(7) (2013):768-74), or tumors with microsatellite instability-high (MSI-H) or deficient mismatch repair (dMMR) (several anti-PD-1 antibody drugs have been approved by the FDA or other regulatory authorities for the treatment of tumors with MSI-H/dMMR characteristics).

The current combination therapies of anti-angiogenic treatment and immune checkpoint inhibitors have demonstrated good efficacy in many tumors. For example, combinations of anti-VEGF antibodies, such as bevacizumab, and anti-PD-1/PD-L1 antibodies, such as nivolumab, pembrolizumab, atezolizumab, etc., have exhibited good efficacy in ovarian cancer (Joyce F. Liu, et al., JAMA Oncol., 2019; 5(12):1731-1738.), non-small cell lung cancer (including non-small cell lung cancers with EGFR and/or ALK-sensitive mutations) (Manegold C, et al., J Thorac Oncol., 2017 Feb; 12(2):194-207), renal cell carcinoma (Dudek AZ, et al., J Clin Oncol., 2018; 36 (suppl; abstr 4558)), hepatocellular carcinoma (Stein S et al., J Clin Oncol, 2018, 36(15_suppl):4074.; the combination of bevacizumab and atezolizumab for treating hepatocellular carcinoma has bee approved by the FDA in 2020), colorectal cancer (including MSI-H/dMMR and non-MSI-H/dMMR types) (Bendell JC, et al., American Society of Clinical Oncology; May 29-June 2, 2015; Chicago, IL. 2015; abstract 704; Hochster HS, et al., American Society of Clinical Oncology Gastrointestinal Cancers Symposium; January 19-21, 2017; San Francisco, CA. 2017; abstract 673.) and breast cancer (Yukinori Ozaki, et al., AACR; Cancer Res, 2020; 80(4 Suppl):Abstract nr PD1-03.); the combination of VEGFR2 antibody and PD-1 antibody also demonstrated good antitumor efficacy in gastroesophageal junction adenocarcinoma (Herbst RS, et al., Lancet Oncol., 2019; 20(8):1109-1123.); the combination of PD-1 antibody (pembrolizumab) and angiogenesis inhibitor (lenvatinib) showed good efficacy in treating endometrial cancer, and is approved by the FDA for the treatment of endometrial cancer in 2019. For tumors such as melanoma (PD-1 antibodies nivolumab and pembrolizumab have been approved by the FDA for the treatment of melanoma), cervical cancer (Krishnansu S., et al., N Engl J Med 2014; 370:734-743.), glioma, prostate cancer (Antonarakis ES., et al., J Clin Oncol., Feb. 10, 2020; 38(5):395-405.), urothelial carcinoma (Joaquim Bellmunt, et al., N Engl J Med, 2017; 376:1015-1026; nivolumab was approved by the FDA in 2017 for treating bladder cancer), oesophageal cancer (Kato K, et al., Lancet Oncol. 2019; 20(11):1506-17.) and mesothelioma (Scherpereel A, et al., Lancet Oncol., 2019; 20(2):239-253.), PD-1/PDL-1 antibodies have exhibited good efficacy. Considering the synergism of PD-1 pathway and VEGF pathway in oncogenesis, superior anti-tumor efficacy is expected in drugs that block both PD-1 and VEGF pathways.

Poly ADP-ribose polymerases (PARPs) are members of the PARP enzyme family, and include PARP-1, PARP-2, PARP-3 and Vallt-PARP. PARP plays a key role in the DNA repair pathway. When DNA is broken, PARP is activated. As a molecular sensor of DNA damage, it participates in the DNA repair process by recognizing and binding to the cleavage site of DNA, and thus activating and catalyzing poly ADP ribosylation of the receptor protein.

PARP inhibitors can exert anti-tumor efficacy by mediating synthetic lethality. In addition to the PARP-mediated DNA repair, other DNA repair mechanisms may occur. Homologous recombination refers to recombination that occurs between non-sister chromatids or between or within DNA molecules containing homologous sequences on the same chromosome.DNA homologous recombination repair (HRR) is an important repair pattern of DNA double strand damages. BRCA1 and BRCA2 are key proteins in the process, and if BRCA gene mutation results in deficient BRCA1 and BRCA2 proteins, HRR dysfunction or HRD (homologous recombination deficiency) may occur. Abnormalities in other HRR-related genes such as PALB2, ATM, BRCA1/2, CHEK2, BARD1, BRIP1, Mre11, CDK12, RAD50, NBS1, RAD51C, RAD51D and PALB2, methylation of BRCA1 gene promoter and other unspecified factors may lead to HRD and genomic instability (Lord CJ, Ashworth A, Science, 2017; 355(6330):1152-1158.). The Cancer Genome Atlas (TCGA) has found HRD (homologous recombination deficiency) in more than 50% of high-grade serous ovarian cancer, including BRCA1/2 germline mutations (15%), BRCA1/2 somatic mutations (6%), epigenetically altered BRCA1 promoter methylation (11%), EMSY amplification (8%), PTEN mutations (7%), RAD51C methylation (3%), ATM or ATR mutations (2%) and other HRD gene mutations (5%) (Turner NC., N Engl J Med., 2017; 377(25):2490-2492).

Synthetic lethality refers to the phenomenon in which simultaneous inactivations of two non-lethal genes lead to cell death. PARP inhibitors can bind to the catalytic site of PARP1 or PARP2, resulting in failure of PARP proteins to detach from the site of DNA damage and thereby the arrest of DNA replication forks and failure of DNA replication; if PARP is inhibited and HRR-related proteins are defective, the synthetic lethality occurs.

For tumor cells carrying specific DNA repair deficiency (e.g., BRCA1 or BRCA2 germline gene mutations), PARP inhibition can hinder the DNA repair in tumor cells and promote tumor cell death through synthetic lethality.

PARP inhibitor (PARPi) has demonstrated good efficacy in clinical studies of cancers such as ovarian cancer, breast cancer, prostate cancer and pancreatic cancer, and has been approved for use in treating cancers such as ovarian cancer, fallopian tube cancer, peritoneal cancer and breast cancer carrying homologous recombination deficiency (J. Mateo et al., Ann Oncol., Sep. 1, 2019; 30(9):1437-1447.); PARPi also exhibited good efficacy in pancreatic cancer and prostate cancer. PARPi still demonstrates certain antitumor efficacy in tumors that do not carry a DNA repair deficiency (e.g., BRCA1 or BRCA2 germline gene mutations). In a NOVA clinical study, compared with the placebo, PFS of patients receiving niraparib was significantly prolonged, regardless of whether the patient carries a homologous recombination deficient gene mutation or not (Mirza MR, et al., N Engl J Med, 2016; 375(22):2154-2164.); other PARPi, such as olaparib and rucaparib, also exhibited good efficacy in tumor patients without homologous recombination deficient gene mutations (J. Mateo, et al., Ann Oncol., Sep. 1, 2019; 30(9):1437-1447.).

At present, there are four approved PARP inhibitors: olaparib (AstraZeneca), rucaparib (Clovis Oncology, USA), niraparib (Tesaro) and talazoparib (Pfizer). Fluzoparib (Hengrui Pharmaceuticals, China) has been approved in China for the treatment of patients with platinum-sensitive recurrent ovarian cancer, fallopian tube cancer or primary peritoneal cancer with germline BRCA mutations who had undergone two or more lines of previous chemotherapy. In addition, veliparib ER, ABT-472, ABT-767, Stenoparib, AST-6828, AG-PD, ANG-2864, ANG-3038, ANG-3186, AZD-5305, AZ-0108, AZD-2461, AMXI-5001, AMXI-2001, AMXI-3001, AMXI-7001, AMXI-9001, pamiparib, ZYTP-1, CK-102, XZ-120312, YHP-743, iobenguane I 131, rucaparib camsylate, CVL-218, CPH-101, CPH-102, CBX-11, CBX-15, minocycline, DB-207, DPS-102, E-7016, iobenguane I 131, MK-2512, HCX-014, HWH-340, IDX-1197, IDX-1197, senaparib, IMP-04100, IMP-04111, IMP-04149, IMP-04249, IMP-04307, IMP-04356, JPI-289, JPI-547, JPI-283, fluzoparib, GT-1620, iobenguane I 131, DR-2313, MP-124, H-10, NT-125, BGP-15, NMSP-293, NMSP-293, NMSP-118, NMSP-648, NMSP-914, DB-207, NUV-1156, NUV-1176, JPI-289, Stenoparib, OX-401, NU-1025, NU-1085, PLX-376, R-554, RBN-2397, RBN-012759, PJ-34, INO-1001, WW-46, BSI-401, iniparib, SOMCL-9112, SC-10914, HTMC-0435, SRX-3128, TSL-1502, PJ-34, CEP-8983, CK-102, THG-009, talazoparib SR, L-2286, mitoparib and WB-1340 are under development.

Bifunctional antibodies, also known as bispecific antibodies, are specific therapies that target two different antigens simultaneously, and can be produced by immunoselection purification. In addition, bispecific antibodies can also be produced by genetic engineering, and have certain advantages due to corresponding flexibility in aspects such as the optimization of binding sites, considerations of synthetic form and yield. Currently, the bispecific antibody has been demonstrated to exist in over 45 forms (Müller D, Kontermann RE. Bispecific antibodies for cancer immunotherapy: current perspectives. BioDrugs, 2010; 24:89-98). A number of bispecific antibodies have been developed in the form of IgG-scFv, namely the Morrison form (Coloma M. J., Morrison S. L. Design and production of novel tetravalent bispecific antibodies. Nat Biotechnol., 1997; 15:159-163), which has been demonstrated to be one of the ideal forms of the bispecific antibodies because of its similarity to the naturally existing IgG form and advantages in antibody engineering, expression and purification (Miller B. R., Demarest S. J., et al., Protein Eng Des Sel, 2010; 23:549-57; Fitzgerald J, Lugovskoy A. MAbs, 2011; 3:299-309).

Preclinical studies found that PARPi could trigger the recognition of tumor cells by the immune system based on both neoantigens and non-neoantigens, suggesting that its combination with immune checkpoint inhibitors might lead to better efficacy (Mateo J, et al., Ann Oncol., 2019; 30(9):1437-1447.) Inhibition of PARP induces increased expression of PD-L1 in tumor cells, resulting in immunosuppression and thus impaired efficacy of PARPi. As such, blocking PD-(L)1 while inhibiting PARP can avoid immunosuppression, resulting in stronger anti-tumor activity of PARPi and PD-(L)1 inhibitors (Jiao S, et al., Clin Cancer Res., 2017; 23(14):3711-3720.). On the other hand, for tumor cells, mutations in related genes cause specific DNA repair deficiencies, the accumulation of DNA damages causes the increased difference between tumor cells and normal cells and ease of recognizing tumor cells by the immune system. A greater amount of mutations in tumors will lead to a survival of tumor cells more dependent on the inhibition of the immune system. Therefore, the combined use of PARPi and PD-1 drugs has certain theoretical basis. Other than PD-1, for example, anti-PD-1/VEGFA bispecific antibodies also block VEGF-A which has high correlation with PD-1 expression in tumor microenvironment, and suppress tumor progression through mechanisms such as angiogenesis inhibition.

Therefore, developing a treatment or combination therapy with higher efficacy is of great meaning.

SUMMARY

Through inventive efforts, the inventor conducted in-depth studies on combination therapies of anti-PD-1/anti-VEGFA antibodies and PARP inhibitors in the treatment of tumors, and found that the combined use of the two has good efficacy in treating and/or preventing tumors, particularly malignant tumors. The present invention is detailed below.

One aspect of the present invention relates to a therapeutic combination comprising at least one bispecific antibody and at least one PARP inhibitor,

wherein, the bispecific antibody comprises:

a first protein functional region targeting PD-1, and

a second protein functional region targeting VEGFA;

wherein the first protein functional region is an immunoglobulin, and the second protein functional region is a single chain antibody; or, the first protein functional region is a single chain antibody, and the second protein functional region is an immunoglobulin;

wherein,

the immunoglobulin comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively; the single chain antibody comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively;

or,

the immunoglobulin comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the single chain antibody comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively;

the immunoglobulin is of human IgG1 subtype;

wherein, according to the EU numbering system, the immunoglobulin comprises a heavy chain constant region having mutations at any 2 or 3 of positions 234, 235 and 237, and the affinity constant of the bispecific antibody for FcγRIIIa and/or Clq is reduced after the mutation as compared to that before the mutation; preferably, the affinity constant is measured by a Fortebio Octet system.

In one or more embodiments of the present invention, for the therapeutic combination, according to the EU numbering system, the heavy chain constant region of the immunoglobulin has the following mutations:

L234A and L235A; or

L234A and G237A; or

L235A and G237A;

or

L234A, L235A and G237A.

In the present invention, letters before the position number represent amino acids before mutation, and letters after the position number represent amino acids after mutation, unless otherwise specified.

The present invention further relates to a therapeutic combination comprising at least one bispecific antibody and at least one PARP inhibitor,

wherein, the bispecific antibody comprises:

a first protein functional region targeting PD-1, and

a second protein functional region targeting VEGFA;

wherein the first protein functional region is an immunoglobulin, and the second protein functional region is a single chain antibody; or, the first protein functional region is a single chain antibody, and the second protein functional region is an immunoglobulin;

wherein,

the immunoglobulin comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively; the single chain antibody comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively;

or,

the immunoglobulin comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the single chain antibody comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively;

the immunoglobulin is of human IgG1 subtype.

The present invention further relates to a therapeutic combination comprising at least one bispecific antibody and at least one PARP inhibitor,

wherein, the bispecific antibody comprises:

a first protein functional region targeting PD-1, and

a second protein functional region targeting VEGFA;

wherein the first protein functional region is an immunoglobulin, and the second protein functional region is a single chain antibody; or, the first protein functional region is a single chain antibody, and the second protein functional region is an immunoglobulin;

wherein,

the immunoglobulin comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively; the single chain antibody comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively;

or,

the immunoglobulin comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the single chain antibody comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively;

the immunoglobulin is of human IgG1 subtype;

wherein, according to an EU numbering system, the heavy chain constant region of the immunoglobulin has the following mutations:

L234A and L235A; or

L234A and G237A; or

L235A and G237A; or

L234A, L235A and G237A.

In one or more embodiments of the present invention, for the therapeutic combination, according to the EU numbering system, the heavy chain constant region of the immunoglobulin has or further has one or more mutations selected from:

N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present invention, for the therapeutic combination,

the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence selected from SEQ ID NO: 5 and SEQ ID NO: 9, and the light chain variable region of the single chain antibody has an amino acid sequence selected from SEQ ID NO: 7, SEQ ID NO: 11 and SEQ ID NO: 17;

or,

the heavy chain variable region of the immunoglobulin has an amino acid sequence selected from SEQ ID NO: 5 and SEQ ID NO: 9, and the light chain variable region of the immunoglobulin has an amino acid sequence selected from SEQ ID NO: 7, SEQ ID NO: 11 and SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3.

In one or more embodiments of the present invention, the therapeutic combination is selected from any one of the following (1)-(12):

(1) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 7;

(2) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 11;

(3) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 17;

(4) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 9, and the light chain variable region of the single chain antibody has an amino acid sequence set forth in SEQ ID NO: 7;

(5) the heavy chain variable region of the immunoglobulin has an amino acid sequence set forth in SEQ ID NO: 1, and the light chain variable region of the immunoglobulin has an amino acid sequence set forth in SEQ ID NO: **3; the** heavy chain variable region of the single chain antibody has an amino acid sequence set forth in SEQ ID NO: 9, and the light chain variable region of the single chain antibody has an amino acid sequence set forth in SEQ ID NO: 11;

(6) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 9, and the light chain variable region of the single chain antibody has an amino acid sequence set forth in SEQ ID NO: 17;

(7) the heavy chain variable region of the immunoglobulin has an amino acid sequence set forth in SEQ ID NO: 5, and the light chain variable region of the immunoglobulin has an amino acid sequence set forth in SEQ ID NO: 7; the heavy chain variable region of the single chain antibody has an amino acid sequence set forth in SEQ ID NO: 1, and the light chain variable region of the single chain antibody has an amino acid sequence set forth in SEQ ID NO: 3;

(8) the heavy chain variable region of the immunoglobulin has an amino acid sequence set forth in SEQ ID NO: 5, and the light chain variable region of the immunoglobulin has an amino acid sequence set forth in SEQ ID NO: 11; the heavy chain variable region of the single chain antibody has an amino acid sequence set forth in SEQ ID NO: 1, and the light chain variable region of the single chain antibody has an amino acid sequence set forth in SEQ ID NO: 3;

(9) the heavy chain variable region of the immunoglobulin has an amino acid sequence set forth in SEQ ID NO: 5, and the light chain variable region of the immunoglobulin has an amino acid sequence set forth in SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3;

(10) the heavy chain variable region of the immunoglobulin has an amino acid sequence set forth in SEQ ID NO: 9, and the light chain variable region of the immunoglobulin has an amino acid sequence set forth in SEQ ID NO: 7; the heavy chain variable region of the single chain antibody has an amino acid sequence set forth in SEQ ID NO: 1, and the light chain variable region of the single chain antibody has an amino acid sequence set forth in SEQ ID NO: 3;

(11) the heavy chain variable region of the immunoglobulin has an amino acid sequence set forth in SEQ ID NO: 9, and the light chain variable region of the immunoglobulin has an amino acid sequence set forth in SEQ ID NO: 11; the heavy chain variable region of the single chain antibody has an amino acid sequence set forth in SEQ ID NO: 1, and the light chain variable region of the single chain antibody has an amino acid sequence set forth in SEQ ID NO: 3; and

(12) the heavy chain variable region of the immunoglobulin has an amino acid sequence set forth in SEQ ID NO: 9, and the light chain variable region of the immunoglobulin has an amino acid sequence set forth in SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3.

In one or more embodiments of the present invention, for the therapeutic combination:
the immunoglobulin comprises a heavy chain having an amino acid sequence set forth in SEQ ID NO: 24, and a light chain having an amino acid sequence set forth in SEQ ID NO: 26.

In one or more embodiments of the present invention, for the therapeutic combination, the bispecific antibody is in the form of IgG-scFv, i.e., the Morrison format.

In some embodiments of the present invention, for the therapeutic combination,
the heavy chain constant region of the immunoglobulin is selected from a heavy chain constant region of human IgG1, IgG2, IgG3 or IgG4, and the immunoglobulin comprises a light chain constant region selected from a light chain constant region of human IgG1, IgG2, IgG3 or IgG4.

In some embodiments of the present invention, for the therapeutic combination,
the heavy chain constant region of the immunoglobulin is human Ig gamma-1 chain C region or human Ig gamma-4 chain C region, and the light chain constant region of the immunoglobulin is human Ig kappa chain C region.

In some embodiments of the present invention, the constant regions of the immunoglobulin are humanized. For example, the heavy chain constant region is Ig gamma-1 chain C region, ACCESSION: P01857, and the light chain constant region is Ig kappa chain C region, ACCESSION: P01834; or
the heavy chain constant region of the immunoglobulin is Ig gamma-4 chain C region, ACCESSION: P01861.1, and the light chain constant region is Ig kappa chain C region, ACCESSION: P01834.

In one embodiment of the present invention, the heavy chain constant region Ig gamma-1 chain C region (ACCESSION: P01857) has the following amino acid sequence:

**ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMIS RTPEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSRDELTKNQVSLTCLVKGFYPSDIAVEWESNGQP ENNYKTTPPVLDSDGSFFLYSKLTVDKSRWQQGNVFSCSVMHEA LHNHYTQKSLSLSPGK (SEQ ID NO: 40)**

In one embodiment of the present invention, the heavy chain constant region Ig gamma-4 chain C region (ACCESSION: P01861.1) has the following amino acid sequence:

**ASTKGPSVFPLAPCSRSTSESTAALGCLVKDYFPEPVTVSWNSGAL TSGVHTFPAVLQSSGLYSLSSVVTVPSSSLGTKTYTCNVDHKPSNT KVDKRVESKYGPPCPSCPAPEFLGGPSVFLFPPKPKDTLMISRTPE VTCVVVDVSQEDPEVQFNWYVDGVEVHNAKTKPREEQFNSTYRV VSVLTVLHQDWLNGKEYKCKVSNKGLPSSIEKTISKAKGQPREPQ VYTLPPSQEEMTKNQVSLTCLVKGFYPSDIAVEWESNGQPENNYK TTPPVLDSDGSFFLYSRLTVDKSRWQEGNVFSCSVMHEALHNHYT QKSLSLSLGK (SEQ ID NO: 41)**

In one embodiment of the present invention, the light chain constant region Ig kappa chain C region (ACCESSION: P01834) has the following amino acid sequence:

**RTVAAPSVFIFPPSDEQLKSGTASVVCLLNNFYPREAKVQWKVDN ALQSGNSQESVTEQDSKDSTYSLSSTLTLSKADYEKHKVYACEVT HQGLSSPVTKSFNRGEC (SEQ ID NO: 42)**

In some embodiments of the present invention, for the therapeutic combination, the single chain antibody is linked to the C terminus of the heavy chain of the immunoglobulin. Since an immunoglobulin has two heavy chains, two single chain antibody molecules are linked to one immunoglobulin molecule. Preferably, the two single chain antibody molecules are identical.

In some embodiments of the present invention, for the therapeutic combination, two single chain antibodies are present, and one terminus of each single chain antibody is linked to the C terminus or the N terminus of one of the two heavy chains of the immunoglobulin.

In some embodiments of the present invention, a disulfide bond is present between the $V_H$ and the $V_L$ of the single chain antibody. Methods for introducing disulfide bonds between the $V_H$ and $V_L$ of an antibody are well known in the art, see, for example, US5,747,654; Rajagopal, et al., Prot. Engin., 10(1997)1453-1459; Reiter et al., Nat. Biotechnol., 14(1996)1239-1245; Reiter, et al., Protein Engineering, 8(1995)1323-1331; Webber, et al., Molecular Immunology, 32(1995)249-258; Reiter, et al., Immunity, 2(1995)281-287; Reiter, et al., JBC, 269(1994)18327-18331; Reiter, et al., Inter. J. of Cancer, 58(1994)142-149; and Reiter, et al., Cancer Res., 54(1994)2714-2718, which are incorporated herein by reference.

In one or more embodiments of the present invention, for the therapeutic combination, the first protein functional region is linked to the second protein functional region either directly or via a linker fragment; and/or the heavy chain variable region of the single chain antibody is linked to the light chain variable region of the single chain antibody either directly or via a linker fragment.

In one or more embodiments of the present invention, for the therapeutic combination, the linker fragment is (GGGGS)n; n is a positive integer, and preferably, n is 1, 2, 3, 4, 5 or 6.

In one or more embodiments of the present invention, for the therapeutic combination, the numbers of the first protein functional region and the second protein functional region are each independently 1, 2 or more.

In one or more embodiments of the present invention, for the therapeutic combination, the single chain antibody is linked to the C terminus of the heavy chain of the immunoglobulin.

The present invention further relates to a therapeutic combination comprising at least one bispecific antibody and at least one PARP inhibitor,
wherein, the bispecific antibody comprises:

a first protein functional region targeting PD-1, and
a second protein functional region targeting VEGFA;
the number of the first protein functional region is 1, and the number of the second protein functional region is 2;

wherein the first protein functional region is an immunoglobulin, and the second protein functional region is a single chain antibody;

the immunoglobulin comprises a heavy chain having an amino acid sequence set forth in SEQ ID NO: 24, and a light chain having an amino acid sequence set forth in SEQ ID NO: 26;

the single chain antibody comprises a heavy chain variable region having an amino acid sequence set forth in SEQ ID NO: 9, and a light chain variable region having an amino acid sequence set forth in SEQ ID NO: 17; or

the single chain antibody is linked to the C terminus of the heavy chain of the immunoglobulin;

the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different; preferably, amino acid sequences of the first linker fragment and the second linker fragment are independently selected from SEQ ID NO: 18 and SEQ ID NO: 19;

preferably, amino acid sequences of the first linker fragment and the second linker fragment are set forth in SEQ ID NO: 18.

In one or more embodiments of the present invention, for the therapeutic combination, the bispecific antibody or an antigen-binding fragment thereof binds to FcγRI with an affinity constant less than about $10^{-6}$ M, for example, less than about $10^{-7}$ M, $10^{-8}$ M, $10^{-9}$ M or less; preferably, the affinity constant is measured by a Fortebio Octet system.

In one or more embodiments of the present invention, for the therapeutic combination, the bispecific antibody or an antigen-binding fragment thereof binds to C1q with an affinity constant less than about $10^{-9}$ M, for example, less than about $10^{-7}$ M, $10^{-8}$ M, $10^{-9}$ M or less; preferably, the affinity constant is measured by a Fortebio Octet system.

In some embodiments of the present invention, for the therapeutic combination, the bispecific antibody or the antigen-binding fragment thereof binds to a VEGFA protein and/or a PD-1 protein with a $K_D$ less than $10^{-5}$ M, such as less than $10^{-6}$ M, $10^{-7}$ M, $10^{-8}$ M, $10^{-9}$ M or $10^{-10}$ M or less; preferably, the $K_D$ is measured by a Fortebio molecular interaction instrument.

In some embodiments of the present invention, for the therapeutic combination,

the bispecific antibody or the antigen-binding fragment thereof binds to the VEGFA protein with an $EC_{50}$ less than 1 nM, less than 0.5 nM, less than 0.2 nM, less than 0.15 nM or less than 0.14 nM; preferably, the $EC_{50}$ is measured by indirect ELISA;

and/or,

the bispecific antibody or the antigen-binding fragment thereof binds to the PD-1 protein with an $EC_{50}$ less than 1 nM, less than 0.5 nM, less than 0.2 nM, less than 0.17 nM, less than 0.16 nM or less than 0.15 nM; preferably, the $EC_{50}$ is measured by indirect ELISA.

In one or more embodiments of the present invention, the bispecific antibody is a monoclonal antibody.

In one or more embodiments of the present invention, the bispecific antibody is a humanized antibody.

In some embodiments of the present invention, for the therapeutic combination, the PARP inhibitor is selected from one or more of olaparib, rucaparib, niraparib, talazoparib, fluzoparib, veliparib ER, ABT-472, ABT-767, stenoparib, AST-6828, AG-PD, ANG-2864, ANG-3038, ANG-3186, AZD-5305, AZ-0108, AZD-2461, AMXI-5001, AMXI-2001, AMXI-3001, AMXI-7001, AMXI-9001, pamiparib, ZYTP-1, CK-102, XZ-120312, YHP-743, iobenguane I 131, rucaparib camsylate, CVL-218, CPH-101, CPH-102, CBX-11, CBX-15, minocycline, DB-207, DPS-102, E-7016, iobenguane I 131, MK-2512, HCX-014, HWH-340, IDX-1197, IDX-1197, senaparib, IMP-04100, IMP-04111, IMP-04149, IMP-04249, IMP-04307, IMP-04356, JPI-289, JPI-547, JPI-283, fluzoparib, GT-1620, iobenguane I 131, DR-2313, MP-124, H-10, NT-125, BGP-15, NMSP-293, NMSP-293, NMSP-118, NMSP-648, NMSP-914, DB-207, NUV-1156, NUV-1176, JPI-289, Stenoparib, OX-401, NU-1025, NU-1085, PLX-376, R-554, RBN-2397, RBN-012759, PJ-34, INO-1001, WW-46, BSI-401, iniparib, SOMCL-9112, SC-10914, HTMC-0435, SRX-3128, TSL-1502, PJ-34, CEP-8983, CK-102, THG-009, talazoparib SR, L-2286, mitoparib and WB-1340.

In some embodiments of the present invention, the therapeutic combination further comprises one or more anti-tumor chemotherapeutics;

preferably, the anti-tumor chemotherapeutic is selected from a topoisomerase II (TOP2) inhibitor, and preferably, the topoisomerase II (TOP2) inhibitor is etoposide;

preferably, the anti-tumor chemotherapeutic is selected from a taxane, and preferably, the taxane is selected from paclitaxel, albumin-bound paclitaxel, liposome paclitaxel and docetaxel;

preferably, the anti-tumor chemotherapeutic is selected from a platinum-based drug, preferably, the platinum-based drug is selected from cisplatin, carboplatin, and oxaliplatin;

preferably, the anti-tumor chemotherapeutic is selected from one or more of gemcitabine, pemetrexed and capecitabine.

In some embodiments of the present invention, the therapeutic combination is a fixed combination, e.g., in the form of a solid pharmaceutical composition or a liquid pharmaceutical composition; or the therapeutic combination is a non-fixed combination, e.g., the bispecific antibody, the PARP inhibitor and the anti-tumor chemotherapeutic are each in the form of a pharmaceutical composition. In some embodiments of the present invention, for the therapeutic combination, the pharmaceutical composition further comprises a pharmaceutically acceptable carrier and/or excipient.

The pharmaceutical composition of the present invention may be formulated into any dosage form known in the pharmaceutical field, such as tablet, pill, suspension, emulsion, solution, gel, capsule, powder, granule, elixir, troche, suppository, injection (including injection solution, sterile powder for injection and concentrated solution for injection), inhalant and spray. The preferred dosage form depends on the intended route of administration and therapeutic use. The pharmaceutical composition of the present invention should be sterile and stable under the conditions of manufacture and storage. One preferred dosage form is an injection. Such injections may be sterile solutions for injection. For example, sterile solutions for injection can be prepared by the following method: a necessary amount of the bispecific antibody of the present invention is added in an appropriate solvent, and optionally, other desired ingredients (including, but not limited to, pH regulators, surfactants, adjuvants, ionic strength enhancers, isotonic agents, preservatives, diluents, or any combination thereof) are added at the same time, followed by filtration and sterilization. In addition, sterile solutions for injection can be prepared as sterile lyophilized powders (e.g., by vacuum drying or lyophilizing) for ease of storage and use. Such sterile lyophilized powders may be dispersed in a suitable carrier (e.g., sterile pyrogen-free water) prior to use.

In addition, the bispecific antibody or the PARP inhibitor of the present invention may be present in the pharmaceutical composition in unit dose form for ease of administration. In some embodiments, the unit dose is at least 1 mg, at least 5 mg, at least 10 mg, at least 15 mg, at least 20 mg, at least 25 mg, at least 30 mg, at least 45 mg, at least 50 mg, at least 75 mg or at least 100 mg. When the pharmaceutical composition is in a liquid (e.g., injection) dosage form, it may comprise the bispecific antibody of the present invention at a concentration of at least 0.1 mg/mL, such as at least 0.25 mg/mL, at least 0.5 mg/mL, at least 1 mg/mL, at least 2.5 mg/mL, at least 5 mg/mL, at least 8 mg/mL, at least 10 mg/mL, at least 15 mg/mL, at least 25 mg/mL, at least 50 mg/mL, at least 75 mg/mL or at least 100 mg/mL.

The bispecific antibody or the pharmaceutical composition of the present invention may be administered by any suitable method known in the art, including, but not limited to, oral, buccal, sublingual, ocular, topical, parenteral, rectal, intrathecal, intracisternal, inguinal, intravesical, topical (e.g., powder, ointment, or drop), or nasal route. However, for many therapeutic uses, the preferred route/mode of administration is parenteral (such as intravenous injection, subcutaneous injection, intraperitoneal injection and intramuscular injection). Those skilled in the art will appreciate that the route and/or mode of administration will vary depending on the intended purpose. In a preferred embodiment, the bispecific antibody or the pharmaceutical composition of the present invention is administered by intravenous infusion or injection.

The bispecific antibody or the pharmaceutical composition provided herein can be used alone or in combination, or used in combination with additional pharmaceutically active agents (e.g., a tumor chemotherapeutic). Such an additional pharmaceutically active agent may be administered prior to, concurrently with, or subsequent to the administration of the bispecific antibody of the present invention or the pharmaceutical composition of the present invention.

In the present invention, the administration regimen may be adjusted to achieve the optimal desired response (e.g., a therapeutic or prophylactic response). For example, the regimen may be a single dose, or multiple doses over a period of time, or the dose may be reduced or increased proportionally according to the emergency of the treatment.

The bispecific antibody of the present invention is a bispecific antibody according to any embodiment of the present invention, i.e., an anti-PD-1/anti-VEGFA bispecific antibody.

Another aspect of the present invention relates to a kit product comprising the therapeutic combination according to any embodiment of the present invention, and a package insert.

Yet another aspect of the present invention relates to use of the therapeutic combination according to any embodiment of the present invention or the kit product of the present invention in preparing a medicament for treating and/or preventing a malignant tumor;

preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;
preferably, the tumor is a tumor with deficient repair function mediated by homologous recombination (e.g., a BRCA1 mutation and/or a BRCA2 mutation);

preferably, the tumor is a tumor without deficient repair function mediated by homologous recombination;

preferably, the lung cancer is non-small cell lung cancer or small cell lung cancer;

preferably, the non-small cell lung cancer is a non-small cell lung cancer with EGFR and/or ALK-sensitive mutation;

preferably, the non-small cell lung cancer is a non-small cell lung cancer without EGFR and/or ALK-sensitive mutation;

preferably, the liver cancer is hepatocellular carcinoma;

preferably, the renal cancer is renal cell carcinoma;

preferably, the breast cancer is triple negative breast cancer;

preferably, the urothelial carcinoma is bladder cancer;

preferably, the peritoneal cancer is primary peritoneal cancer;

preferably, the ovarian cancer is an ovarian cancer with deficient repair function mediated by homologous recombination (e.g., a BRCA1 mutation and/or a BRCA2 mutation);

preferably, the fallopian tube cancer is a fallopian tube cancer with deficient repair function mediated by homologous recombination (e.g., a BRCA1 mutation and/or a BRCA2 mutation);

preferably, the peritoneal cancer is a peritoneal cancer with deficient repair function mediated by homologous recombination (e.g., a BRCA1 mutation and/or a BRCA2 mutation);

preferably, the breast cancer is a breast cancer with deficient repair function mediated by homologous recombination (e.g., a BRCA1 mutation and/or a BRCA2 mutation).

Yet another aspect of the present invention relates to use of the therapeutic combination according to any embodiment of the present invention or the kit product of the present invention in preparing the following medicament:

(1)

a medicament or an agent for detecting the level of VEGFA in a sample, a medicament or an agent for blocking the binding of VEGFA to VEGFR2,

a medicament or an agent for down-regulating the activity or level of VEGFA,

a medicament or an agent for relieving the stimulation of VEGFA on vascular endothelial cell proliferation,

a medicament or an agent for inhibiting vascular endothelial cell proliferation, or

a medicament or an agent for blocking tumor angiogenesis;

and/or

(2)

a medicament or an agent for blocking the binding of PD-1 to PD-L1,

a medicament or an agent for down-regulating the activity or level of PD-1,

a medicament or an agent for relieving the immunosuppression of PD-1 in an organism,

a medicament or an agent for promoting IFN-γ secretion in T lymphocytes, or

a medicament or an agent for promoting IL-2 secretion in T lymphocytes.

In the *in vitro* experiment of the present invention, the anti-VEGFA antibody and the anti-VEGFA/anti-PD-1 bispecific antibody both can inhibit HUVEC cell proliferation, and both the anti-PD-1 antibody and the anti-VEGFA/anti-PD-1 bispecific antibody can promote the secretion of IFN-γ and/or IL-2 and activate an immune response.

Yet another aspect of the present invention relates to a method for treating and/or preventing a malignant tumor, comprising: administering to a subject in need an effective amount of the therapeutic combination according to any embodiment of the present invention or the kit product of the present invention;

preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;

preferably, the tumor is a tumor with deficient repair function mediated by homologous recombination (e.g., a BRCA1 mutation and/or a BRCA2 mutation);

preferably, the tumor is a tumor without deficient repair function mediated by homologous recombination;

preferably, the lung cancer is non-small cell lung cancer or small cell lung cancer;

preferably, the non-small cell lung cancer is a non-small cell lung cancer with EGFR and/or ALK-sensitive mutation;
preferably, the non-small cell lung cancer is a non-small cell lung cancer without EGFR and/or ALK-sensitive mutation;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the peritoneal cancer is primary peritoneal cancer;
preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

In some embodiments of the present invention, for the method for treating and/or preventing the malignant tumor, the administration is performed before or after surgery and/or before or after radiotherapy.

In some embodiments of the present invention, for the method for treating and/or preventing the malignant tumor, the bispecific antibody is administered at a unit dose of 0.1-100 mg per kg body weight, preferably 5-50 mg or 5-15 mg per kg body weight,

administered once every 3 days, 4 days, 5 days, 6 days, 10 days, 1 week, 2 weeks or 3 weeks,
and/or
administered by intravenous drip infusion or intravenous injection.

In some embodiments of the present invention, for the method for treating and/or preventing the malignant tumor, the PARP inhibitor is administered at a unit dose of 0.1-100 mg per kg body weight, preferably 5-50 mg or 5-15 mg per kg body weight,

administered once every 3 days, 4 days, 5 days, 6 days, 10 days, 1 week, 2 weeks or 3 weeks,
and/or
administered by intravenous drip infusion or intravenous injection.

In some embodiments of the present invention, for the method for treating and/or preventing the malignant tumor, the bispecific antibody and the PARP inhibitor are administered simultaneously or non-simultaneously.

A typical non-limiting range of a therapeutically or prophylactically effective amount of the bispecific antibody and/or the PARP inhibitor of the present invention is 0.02-100 mg/kg, such as 0.1-50 mg/kg, 0.1-25 mg/kg, or 1-10 mg/kg. It should be noted that the dose may vary with the type and severity of the symptom to be treated. Furthermore, those skilled in the art will appreciate that for any particular patient, the particular dose regimen may be adjusted over time according to the needs of the patient and the professional judgment of the physician; the dose ranges provided herein are for illustrative purpose only and do not limit the use or scope of the pharmaceutical composition of the present invention.

In the present invention, the subject may be a mammal, such as a human. Yet another aspect of the present invention relates to a method *in vivo* or *in vitro,* selected from:

(1)

a method for detecting the level of VEGFA in a sample,
a method for blocking the binding of VEGFA to VEGFR2,
a method for down-regulating the activity or level of VEGFA,
a method for relieving the stimulation of VEGFA on vascular endothelial cell proliferation,
a method for inhibiting vascular endothelial cell proliferation, or a method for blocking tumor angiogenesis;
and/or

(2)

a method for blocking the binding of PD-1 to PD-L1,

a method for down-regulating the activity or level of PD-1,
a method for relieving the immunosuppression of PD-1 in an organism,
a method for promoting IFN-γ secretion in T lymphocytes, or
a method for promoting IL-2 secretion in T lymphocytes.

In one or more embodiments of the present invention, the therapeutic combination or kit product is for use in treating and/or preventing a malignant tumor;

preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;
preferably, the tumor is a tumor with deficient repair function mediated by homologous recombination (e.g., a BRCA1 mutation and/or a BRCA2 mutation);
preferably, the tumor is a tumor without deficient repair function mediated by homologous recombination;
preferably, the lung cancer is non-small cell lung cancer or small cell lung cancer;
preferably, the non-small cell lung cancer is a non-small cell lung cancer with EGFR and/or ALK-sensitive mutation;
preferably, the non-small cell lung cancer is a non-small cell lung cancer without EGFR and/or ALK-sensitive mutation;
preferably, the liver cancer is hepatocellular carcinoma;
preferably, the renal cancer is renal cell carcinoma;
preferably, the breast cancer is triple negative breast cancer;
preferably, the urothelial carcinoma is bladder cancer;
preferably, the peritoneal cancer is primary peritoneal cancer;
preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

In one or more embodiments of the present invention, the therapeutic combination or kit product is for use in:

(1)

detecting the level of VEGFA in a sample,
blocking the binding of VEGFA to VEGFR2,
down-regulating the activity or level of VEGFA,
relieving the stimulation of VEGFA on vascular endothelial cell proliferation,
inhibiting vascular endothelial cell proliferation, or
blocking tumor angiogenesis;
and/or

(2)

blocking the binding of PD-1 to PD-L1,
down-regulating the activity or level of PD-1,
relieving the immunosuppression of PD-1 in an organism,
promoting IFN-γ secretion in T lymphocytes, or
promoting IL-2 secretion in T lymphocytes.

Yet another aspect of the present invention relates to use of the bispecific antibody in the therapeutic combination according to any embodiment of the present invention in preparing a medicament for treating and/or preventing a malignant tumor, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

Yet another aspect of the present invention relates to a method for treating and/or preventing a malignant tumor, comprising: administering to a subject in need an effective amount of the bispecific antibody in the therapeutic combination according to any embodiment of the present invention, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

Yet another aspect of the present invention relates to the bispecific antibody in the therapeutic combination according to any embodiment of the present invention for use in treating and/or preventing a malignant tumor, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

Antibody drugs, especially monoclonal antibodies, have achieved good efficacy in the treatment of various diseases. Conventional methods for acquiring these therapeutic antibodies include immunizing animals with an antigen and acquiring antibodies targeting the antigen in the immunized animals, or modifying those antibodies with lower affinity for the antigen by affinity maturation.

The variable regions of the light chain and the heavy chain determine the binding to antigens; the variable region of each chain comprises three hypervariable regions called complementarity determining regions (CDRs) (CDRs of the heavy chain (H Chain) are HCDR1, HCDR2, and HCDR3, and CDRs of the light chain (L Chain) are LCDR1, LCDR2, and LCDR3, which are named by Kabat et al.; see Bethesda M.d., Sequences of Proteins of Immunological Interest, Fifth Edition, NIH Publication, (1-3) 1991: 91-3242).

Preferably, CDRs may also be defined by the IMGT numbering system; see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research, 38.suppl_1 (2009): D301-D307.

The amino acid sequences of the CDR regions of the monoclonal antibody sequences in (1)-(13) below were analyzed by technical means well known to those skilled in the art, for example by VBASE2 database and especially according to the IMGT definition, and the results are as follows:

(1) Bevacizumab

The heavy chain variable region has an amino acid sequence set forth in SEQ ID NO: 1, and the light chain variable region has an amino acid sequence set forth in SEQ ID NO: 3.

The 3 CDRs of the heavy chain variable region have the following amino acid sequences:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)
the 3 CDRs of the light chain variable region have the following amino acid sequences:

LCDR1: QDISNY (SEQ ID NO: 31)
LCDR2: FTS (SEQ ID NO: 32)
LCDR3: QQYSTVPWT (SEQ ID NO: 33)

(2) 14C12, 14C12H1L1 or 14C12H1L1(M)

The 3 CDRs of the heavy chain variable region have the following amino acid sequences:

HCDR1: GFAFSSYD (SEQ ID NO: 34)
HCDR2: ISGGGRYT (SEQ ID NO: 35)
HCDR3: ANRYGEAWFAY (SEQ ID NO: 36)
the 3 CDRs of the light chain variable region have the following amino acid sequences:

LCDR1: QDINTY (SEQ ID NO: 37)
LCDR2: RAN (SEQ ID NO: 38)
LCDR3: LQYDEFPLT (SEQ ID NO: 39)

(3) VP101(hG1WT) or VP101(hG1DM)

The 9 CDRs of the heavy chain have the following amino acid sequences:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)
HCDR4: GFAFSSYD (SEQ ID NO: 34)
HCDR5: ISGGGRYT (SEQ ID NO: 35)
HCDR6: ANRYGEAWFAY (SEQ ID NO: 36)
HCDR7: QDINTY (SEQ ID NO: 37)
HCDR8: RAN (SEQ ID NO: 38)
HCDR9: LQYDEFPLT (SEQ ID NO: 39)
the 3 CDRs of the light chain variable region have the following amino acid sequences:

LCDR1: QDISNY (SEQ ID NO: 31)
LCDR2: FTS (SEQ ID NO: 32)
LCDR3: QQYSTVPWT (SEQ ID NO: 33).

For the antibody VP101(hG1DM) of the present invention, amino acid mutations are introduced into the non-variable region of VP101(hG1WT). According to the EU numbering system, amino acid mutations are introduced at positions 234 and 235:
VP101 (hG1DM) was obtained by introducing a leucine-to-alanine point mutation at position 234 (L234A) and a leucine-to-alanine point mutation at position 235 (L235A) in the hinge region of the heavy chain.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of VEGFA protein, it includes, but is not limited to, the full length of the VEGFA protein (GenBank ID: NP_001165097.1), as well as a fusion protein of VEGFA, such as a fragment fused to an Fc protein fragment of mouse or human IgG (mFc or hFc). However, those skilled in the art will appreciate that in the amino acid sequence of the VEGFA protein, mutations or variations (including but not limited to,

substitutions, deletions and/or additions) may naturally occur or can be artificially introduced without affecting biological functions thereof. Therefore, in the present invention, the term "VEGFA protein" shall include all such sequences, including their natural or artificial variants. In addition, when describing a sequence fragment of the VEGFA protein, it also includes the corresponding sequence fragments in their natural or artificial variants. In one embodiment of the present invention, the VEGFA protein has an amino acid sequence set forth in the underlined part of SEQ ID NO: 33 (a total of 302 amino acids excluding the last 6 His).

As used herein, when referring to the amino acid sequence of VEGFR2 protein (also known as KDR), it includes, but is not limited to, the full length of the VEGFR2 protein (GenBank ID: NP_002244), or the extracellular fragment VEGFR2-ECD of VEGFR2, or a fragment comprising VEGFR2-ECD, and it also includes a fusion protein of VEGFR2-ECD, such as a fragment fused to an Fc protein fragment of mouse or human IgG (mFc or hFc). However, those skilled in the art will appreciate that in the amino acid sequence of the VEGFR2 protein, mutations or variations (including but not limited to, substitutions, deletions and/or additions) may naturally occur or can be artificially introduced without affecting biological functions thereof. Therefore, in the present invention, the term "VEGFR2 protein" shall include all such sequences, including their natural or artificial variants. In addition, when describing a sequence fragment of the VEGFR2 protein, it also includes the corresponding sequence fragments in their natural or artificial variants. In one embodiment of the present invention, the extracellular fragment VEGFR2-ECD of VEGFR2 has an amino acid sequence set forth in SEQ ID NO: 34 (766 amino acids).

As used herein, unless otherwise specified, the VEGFR is VEGFR1 and/or VEGFR2; specific protein sequence thereof is a sequence known in the prior art, and reference may be made to the sequence disclosed in the existing literature or GenBank. For example, VEGFR1 (VEGFR1, NCBI Gene ID: 2321); VEGFR2 (VEGFR2, NCBI Gene ID: 3791).

As used herein, when referring to the amino acid sequence of PD-1 protein (programmed cell death protein 1), it includes the full length of the PD-1 protein (NCBI GenBank: NP_005009.2), or the extracellular fragment PD-1ECD of PD-1 or a fragment comprising PD-1ECD, and it also includes a fusion protein of PD-1ECD, such as a fragment fused to an Fc protein fragment of a mouse or human IgG (mFc or hFc). However, it will be appreciated by those skilled in the art that in the amino acid sequence of PD-1 protein, mutations or variations (including but not limited to substitutions, deletions and/or additions) may naturally occur or can be artificially introduced without affecting biological functions thereof. Therefore, in the present invention, the term "PD-1 protein" shall include all such sequences, including their natural or artificial variants. In addition, when describing a sequence fragment of the PD-1 protein, it also includes the corresponding sequence fragments in their natural or artificial variants.

As used herein, the term $EC_{50}$ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair consisting of one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains can be classified into κ and λ light chains. Heavy chains are generally classified into μ, δ, γ, α and ε, and the antibodies are defined as IgM, IgD, IgG, IgA and IgE isotypes, respectively. In light chains and heavy chains, the variable region and constant region are linked by a "J" region of about 12 or more amino acids, and the heavy chain further comprises a "D" region of about 3 or more amino acids. Each heavy chain consists of a heavy chain variable region ($V_H$) and a heavy chain constant region ($C_H$). The heavy chain constant region consists of 3 domains ($C_{H1}$, $C_{H2}$, and $C_{H3}$). Each light chain consists of a light chain variable region ($V_L$) and a light chain constant region ($C_L$). The light chain constant region consists of one domain $C_L$. The constant region of the antibody can mediate the binding of immunoglobulins to host tissues or factors, including the binding of various cells of the immune system (e.g., effector cells) to the first component (C1q) of classical complement system. The $V_H$ and $V_L$ regions can be further subdivided into hypervariable regions (called complementarity determining regions, or CDRs) and conservative regions called framework regions (FRs) that are distributed between the CDRs. Each $V_H$ and $V_L$ consists of 3 CDRs and 4 FRs arranged from the amino terminus to the carboxyl terminus in the following order: FR1, CDR1, FR2, CDR2, FR3, CDR3, FR4. The variable regions ($V_H$ and $V_L$) of each heavy chain/light chain pair form an antibody binding site. The assignment of amino acids to the regions or domains may be defined by Kabat Sequences of Proteins of Immunological Interest (National Institutes of Health, Bethesda, Md. (1987 and 1991)), or Chothia & Lesk J. Mol. Biol., 196(1987): 901-917; Chothia et al., Nature, 342(1989): 878-883 or the IMGT numbering system (see Ehrenmann, Francois, Quentin Kaas, and Marie-Paule Lefranc. "IMGT/3Dstructure-DB and IMGT/Domain-GapAlign: a database and a tool for immunoglobulins or antibodies, T cell receptors, MHC, IgSF and MhcSF." Nucleic acids research, 38.suppl_1 (2009): D301-D307). In particular, the heavy chain may further comprise more than 3 CDRs, such as 6, 9, or 12. For example, in the bispecific antibody of the present invention, the heavy chain may be an scFv with the C-terminus of the heavy chain of IgG antibody linked to another antibody, and in this case, the heavy chain comprises 9 CDRs. The term "antibody" is not limited by any specific method for producing the antibody. For example, the antibody includes, in particular, a recombinant antibody, a monoclonal antibody and a polyclonal antibody. The antibody can be antibodies of different isotypes, such as IgG (e.g., subtypes IgG1, IgG2, IgG3 or IgG4), IgA1, IgA2, IgD, IgE or IgM.

As used herein, the term "antigen binding fragment", also known as the "antigen binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989)). An antigen-binding fragment of an antibody can be produced by recombinant DNA technique or by enzymatic or chemical cleavage of the intact antibody. In some cases, the antigen binding fragment includes Fab, Fab', F (ab')$_2$, Fd, Fv, dAb, and complementarity determining region (CDR) fragment, single chain antibody fragment (e.g., scFv), chimeric antibody, diabody and a polypeptide that comprises at least a portion of an antibody sufficient to impart specific antigen binding ability to the polypeptide. As used herein, the term "Fd fragment" refers to an antibody fragment consisting of $V_H$ and $C_{H1}$ domains; the term "Fv fragment" refers to an antibody fragment consisting of the $V_L$ and $V_H$ domains of a single arm of an antibody; the term "dAb fragment" refers to an antibody fragment consisting of a $V_H$ domain (Ward et al., Nature, 341 (1989):544-546); the term "Fab fragment" refers to an antibody fragment consisting of $V_L$, $V_H$, $C_L$ and $C_{H1}$ domains; the term "F(ab')$_2$ fragment" refers to an antibody fragment comprising two Fab fragments linked by disulfide bridges in the hinge region.

In some cases, the antigen binding fragment of the antibody is a single chain antibody (e.g., scFv) in which the $V_L$ and $V_H$ domains are paired to form a monovalent molecule via a linker that enables the production of a single polypeptide chain (see, e.g., Bird et al., Science 242 (1988):423-426 and Huston et al., Proc. Natl. Acad. Sci. USA, 85 (1988):5879-5883). Such scFv molecules may have a general structure: $NH_2$-$V_L$-linker-$V_H$-COOH or $NH_2$-$V_H$-linker-$V_L$-COOH. An appropriate linker in prior art consists of GGGGS amino acid sequence repeats or a variant thereof. For example, a linker having the amino acid sequence (GGGGS)$_4$ can be used, and variants thereof can also be used (Holliger et al., Proc. Natl. Acad. Sci. USA, 90 (1993):6444-6448). Other linkers useful in the present invention are described by Alfthan et al., Protein Eng. 8 (1995): 725-731, Choi et al., Eur. J. Immunol. 31 (2001): 94-106, Hu et al., Cancer Res. 56 (1996): 3055-3061, Kipriyanov et al., J. Mol. Biol. 293 (1999): 41-56, and Roovers et al., Cancer Immunol. (2001).

In some cases, the antigen binding fragment of the antibody is a diabody, that is, a bivalent antibody, in which the $V_H$ and $V_L$ domains are expressed on a single polypeptide chain. However, the linker used is too short to allow the pairing of the two domains on the same chain. Thus the domains are forced to pair with the complementary domains on the other chain and two antigen binding sites are generated (see, e.g., Holliger P. et al., Proc. Natl. Acad. Sci. USA 90 (1993):6444-6448, and Poljak R.J. et al., Structure 2 (1994):1121-1123).

Antigen-binding fragments (e.g., the above mentioned antibody fragments) of antibodies can be obtained from given antibodies by using conventional techniques known to those skilled in the art (e.g., DNA recombination, or enzymatic or chemical cleavage), and the antigen-binding fragments of the antibodies are screened for specificity in the same way as for intact antibodies.

As used herein, unless otherwise clearly defined in the context, when referring to the term "antibody", it includes not only intact antibodies but also antigen-binding fragments of antibodies.

As used herein, the terms "mAb" and "monoclonal antibody" refer to an antibody or a fragment of an antibody that is derived from a group of highly homologous antibodies, i.e. from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained by hybridoma technique first reported by Kohler et al. (Nature, 256:495, 1975), and can also be obtained by recombinant DNA technique (for example, see U.S. Patent No. 4,816,567).

As used herein, the term "chimeric antibody" refers to an antibody of which a part of the light or/and heavy chains is derived from an antibody (which may be derived from a specific species or belong to a specific antibody isotype or subtype), and the other part of the light or/and heavy chains are derived from another antibody (which may be derived from the same or different species or belong to the same or different antibody isotype or subtype). But in any case, it retains the binding activity for the target antigen (U.S. Patent 4,816,567 Cabilly et al.; Morrison et al., Proc. Natl. Acad. Sci. USA, 81 (1984):6851-6855).

As used herein, the term "humanized antibody" refers to an antibody or antibody fragment obtained when all or a part of CDRs of a human immunoglobulin (receptor antibody) are replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 321 (1986): 522-525; Reichmann et al., Nature, (1988) 332:323-329; Presta, Curr. Op. Struct. Biol., 2 (1992): 593-596, and Clark, Immunol. Today 21 (2000): 397-402.

As used herein, the term "epitope" refers to a site on the antigen that an immunoglobulin or antibody specifically binds to. "Epitope" is also referred to in the art as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14 or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *Escherichia coli* or *bacillus subtilis*, fungal cells such as yeast cells or *Aspergillus*, insect cells such as S2 drosophila cells or Sf9, or animal cells such as fibroblasts, CHO cells, COS cells, NSO cells, HeLa cells, BHK cells, HEK 293 cells or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity ($K_D$) less than about $10^{-5}$ M, such as less than about $10^{-6}$ M, $10^{-7}$ M, $10^{-8}$ M, $10^{-9}$ M or $10^{-10}$ M or less. In some embodiments of the present invention, the term "target" refers to specific binding.

As used herein, the term "$K_D$" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, an antibody binds to an antigen with a dissociation equilibrium constant ($K_D$) less than about $10^{-5}$ M, such as less than about $10^{-6}$ M, $10^{-7}$ M, $10^{-8}$ M, $10^{-9}$ M or $10^{-10}$ M or less, for example, as measured by surface plasmon resonance (SPR) on a BIACORE system or by a Fortebio system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, as used herein, amino acids are generally represented by single-letter and three-letter abbreviations known in the art. For example, alanine can be represented by A or Ala.

As used herein, the term "pharmaceutically acceptable excipient" refers to a carrier and/or vehicle that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g.,

aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials.

As used herein, the term "effective amount" refers to an amount sufficient to obtain or at least partially obtain a desired effect. For example, a prophylactically effective amount (e.g., for a disease associated with PD-1 binding to PD-L1 or overexpression of VEGF, such as a tumor) is an amount sufficient to prevent, stop or delay the onset of the disease (e.g., a disease associated with PD-L1 binding to PD-L1 or overexpression of VEGF, such as a tumor); a therapeutically effective amount is an amount sufficient to cure or at least partially stop the disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

Beneficial Effects

The present invention achieves any one or more of the following technical effects (1) to (6):

(1) In the present invention, the modifications at the Fc fragment of the antibody completely eliminate the binding activity of VP101(hG1WT) to $F_C$ receptors FcγRI and FcγRIIIa_F158, thereby completely eliminating the ADCC activity.

(2) In the present invention, the modifications at the Fc fragment of the antibody completely eliminate the binding activity of VP101(hG1WT) to complement C1q, thereby eliminating the CDC activity.

(3) The bispecific antibody of the present invention can specifically bind to VEGFA, effectively block the binding of VEGFA to VEGFR2, and specifically relieve the promotion effect of VEGFA on the immune suppression and the angiogenesis.

(4) The bispecific antibody of the present invention can specifically bind to PD-1, effectively block the binding of PD-1 to PD-L1, and specifically relieve the immunosuppression of PD-1 to the organism and activate immune responses.

(5) The combined use of PARPi and the bispecific antibody of the present invention has significantly superior efficacy in tumors, particularly breast or ovarian cancer, to the PARPi or the bispecific antibody alone.

(6) The PARPi and the bispecific antibody of the present invention have a synergistic effect in treating or preventing tumors.

BRIEF DESCRIPTION OF THE DRAWINGS

FIG. 1: Affinity constant assay of VP101(hG1DM) to FcγRI. The antibody concentrations for the curve pairs from top to bottom are 50 nM, 25 nM, 12.5 nM, 6.25 nM and 3.12 nM, respectively.

FIG. 2: Affinity constant assay of bevacizumab to FcγRI. The antibody concentrations for the curve pairs from top to bottom are 50 nM, 25 nM, 12.5 nM, 6.25 nM and 3.12 nM, respectively.

FIG. 3: Affinity constant assay of nivolumab to FcγRI. The antibody concentrations for the curve pairs from top to bottom are 50 nM, 25 nM, 12.5 nM, 6.25 nM and 3.12 nM, respectively.

FIG. 4: Affinity constant assay of VP101(hG1WT) to FcγRI. The antibody concentrations for the curve pairs from top to bottom are 50 nM, 25 nM, 12.5 nM, 6.25 nM and 3.12 nM, respectively.

FIG. 5: Affinity constant assay of VP101(hG4WT) to FcγRI. The antibody concentrations for the curve pairs from top to bottom are 50 nM, 25 nM, 12.5 nM, 6.25 nM and 3.12 nM, respectively.

FIG. 6: Affinity constant assay of VP101(hG1DM) to FcγRIIa_H131. The antibody concentrations for the curve pairs from top to bottom are 200 nM, 100 nM, 50 nM, 25 nM and 12.5 nM, respectively.

FIG. 7: Affinity constant assay of bevacizumab to FcγRIIa_H131. The antibody concentrations for the curve pairs from top to bottom are 200 nM, 100 nM, 50 nM, 25 nM and 12.5 nM, respectively.

FIG. 8: Affinity constant assay of nivolumab to FcγRIIa_H131. The antibody concentrations for the curve pairs from top to bottom are 200 nM, 100 nM, 50 nM, 25 nM and 12.5 nM, respectively.

FIG. 9: Affinity constant assay of VP101(hG1WT) to FcγRIIa_H131. The antibody concentrations for the curve pairs from top to bottom are 200 nM, 100 nM, 50 nM, 25 nM and 12.5 nM, respectively.

FIG. 10: Affinity constant assay of VP101(hG4WT) to FcγRIIa_H131. The antibody concentrations for the curve pairs from top to bottom are 200 nM, 100 nM, 50 nM, 25 nM and 12.5 nM, respectively.

FIG. 11: Affinity constant assay of VP101(hG1DM) to FcγRIIa_R131. The antibody concentrations for the curve pairs from top to bottom are 200 nM, 100 nM, 50 nM, 25 nM and 12.5 nM, respectively.

FIG. 12: Affinity constant assay of bevacizumab to Fc$\gamma$RIIa_RI31. The antibody concentrations for the curve pairs from top to bottom are 200 nM, 100 nM, 50 nM, 25 nM and 12.5 nM, respectively.

FIG. 13: Affinity constant assay of nivolumab to Fc$\gamma$RIIa_RI31. The antibody concentrations for the curve pairs from top to bottom are 200 nM, 100 nM, 50 nM, 25 nM and 12.5 nM, respectively.

FIG. 14: Affinity constant assay of VP101(hG1WT) to Fc$\gamma$RIIa_RI31. The antibody concentrations for the curve pairs from top to bottom are 200 nM, 100 nM, 50 nM, 25 nM and 12.5 nM, respectively.

FIG. 15: Affinity constant assay of VP101(hG4WT) to Fc$\gamma$RIIa_RI31. The antibody concentrations for the curve pairs from top to bottom are 200 nM, 100 nM, 50 nM, 25 nM and 12.5 nM, respectively.

FIG. 16: Affinity constant assay of VP101(hG1DM) to Fc$\gamma$RIIIa_V158. The antibody concentrations for the curve pairs from top to bottom are 500 nM, 250 nM, 125 nM, 62.5 nM and 31.25 nM, respectively.

FIG. 17: Affinity constant assay of bevacizumab to Fc$\gamma$RIIIa_V158.The antibody concentrations for the curve pairs from top to bottom are 500 nM, 250 nM, 125 nM, 62.5 nM and 31.25 nM, respectively.

FIG. 18: Affinity constant assay of nivolumab to Fc$\gamma$RIIIa_V158.The antibody concentrations for the curve pairs from top to bottom are 500 nM, 250 nM, 125 nM, 62.5 nM and 31.25 nM, respectively.

FIG. 19: Affinity constant assay of VP101(hG1WT) to Fc$\gamma$RIIIa_V158. The antibody concentrations for the curve pairs from top to bottom are 500 nM, 250 nM, 125 nM, 62.5 nM and 31.25 nM, respectively.

FIG. 20: Affinity constant assay of VP101(hG4WT) to Fc$\gamma$RIIIa_V158. The antibody concentrations for the curve pairs from top to bottom are 500 nM, 250 nM, 125 nM, 62.5 nM and 31.25 nM, respectively.

FIG. 21: Affinity constant assay of VP101(hG1DM) to Fc$\gamma$RIIIa_F158. The antigen concentrations for the curve pairs from top to bottom are 500 nM, 250 nM, 125 nM, 62.5 nM and 31.25 nM, respectively.

FIG. 22: Affinity constant assay of bevacizumab to Fc$\gamma$RIIIa_F158. The antibody concentrations for the curve pairs from top to bottom are 500 nM, 250 nM, 125 nM, 62.5 nM and 31.25 nM, respectively.

FIG. 23: Affinity constant assay of nivolumab to Fc$\gamma$RIIIa_F158. The antibody concentrations for the curve pairs from top to bottom are 500 nM, 250 nM, 125 nM, 62.5 nM and 31.25 nM, respectively.

FIG. 24: Affinity constant assay of VP101(hG1WT) to Fc$\gamma$RIIIa_F158. The antibody concentrations for the curve pairs from top to bottom are 500 nM, 250 nM, 125 nM, 62.5 nM and 31.25 nM, respectively.

FIG. 25: Affinity constant assay of VP101(hG4WT) to Fc$\gamma$RIIa_F158. The antibody concentrations for the curve pairs from top to bottom are 500 nM, 250 nM, 125 nM, 62.5 nM and 31.25 nM, respectively.

FIG. 26: Affinity constant assay of VP101(hG1DM) to C1q. The antibody concentrations for the curve pairs from top to bottom are 10 nM, 5 nM, 2.5 nM, 1.25 nM and 0.625 nM, respectively.

FIG. 27: Affinity constant assay of bevacizumab to C1q. The antibody concentrations for the curve pairs from top to bottom are 10 nM, 5 nM, 2.5 nM, 1.25 nM and 0.625 nM, respectively.

FIG. 28: Affinity constant assay of nivolumab to C1q. The antibody concentrations for the curve pairs from top to bottom are 10 nM, 5 nM, 2.5 nM, 1.25 nM and 0.625 nM, respectively.

FIG. 29: Affinity constant assay of VP101(hG1WT) to C1q. The antibody concentrations for the curve pairs from top to bottom are 10 nM, 5 nM, 2.5 nM, 1.25 nM and 0.625 nM, respectively.

FIG. 30: Affinity constant assay of VP101(hG4WT) to C1q. The antigen concentrations for the curve pairs from top to bottom are 10 nM, 5 nM, 2.5 nM, 1.25 nM and 0.625 nM, respectively.

FIG. 31: ADCC activity assay of VP101(hG1WT) and VP101(hG1DM) in a CHO-K1-PD1 target cell system expressing PD-1 antigen.

FIG. 32: CDC activity assay of VP101(hG1WT) and VP101(hG1DM) in a CHO-K1-PD1 target cell system expressing PD-1 antigen.

FIG. 33: Effect of antibody VP101(hG1DM) on secretion of cytokine IFN-$\gamma$ induced by mixed lymphocyte reaction of PBMCs and Raji-PDL1 cells by ELISA.

FIG. 34: Effect of antibody VP101(hG1DM) on secretion of cytokine IL-2 induced by mixed lymphocyte reaction of PBMCs and Raji-PDL1 cells by ELISA.

FIG. 35: ADCP activity assay of VP101(hG1DM) in a CHO-K1-PD1 target cell system expressing PD-1 antigen.

FIG. 36: Inhibition on proliferative activity of breast cancer tumor cells in a breast pad tumor xenograft model by VP101(hG1DM).

FIG. 37: Inhibition on migration of ovarian cancer cells by combined use of PARPi and VP101(hG1DM).

FIG. 38: Effect of PARPi and VP101(hG1DM) combination on tumor volume in a breast cancer subcutaneous xenograft tumor mouse model.

FIG. 39: Effect of PARPi and VP101(hG1DM) combination on body weight in a breast cancer subcutaneous xenograft tumor mouse model.

FIG. 40: Effect of PARPi and VP101(hG1DM) combination on tumor volume in an ovarian cancer subcutaneous xenograft tumor mouse model.

FIG. 41: Effect of PARPi and VP101(hG1DM) combination on body weight in an ovarian cancer subcutaneous xenograft tumor mouse model.

DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Guide to Molecular Cloning Experiments, authored by J. Sambrook et al., and translated by Huang Peitang et al., third edition, Science Press) or according to the product instruction. Reagents or instruments used are all commercially available conventional products if the manufacturers thereof are not specified.

In the following examples of the present invention, the approved antibody bevacizumab (trade name Avastin®) for the same target was purchased from Roche as a reference antibody, or was prepared according to Preparation Example 1.

In the following examples of the present invention, the approved antibody nivolumab for the same target (trade name Opdivo®) was purchased from BMS as a reference antibody.

In the following examples of the present invention, the PARP inhibitor olaparib used was purchased from Selleckchem.

The variable region sequence of the isotype control antibody in the examples of the present invention, human anti-hen egg lysozyme IgG (anti-HEL, or human IgG, abbreviated as hIgG), is derived from Acierno et al., "Affinity maturation increases the stability and plasticity of the Fv domain of anti-protein antibodies" (Acierno et al., J Mol Biol., 2007; 374(1):130-46). The hIgG1DM and hIgG4WT used in the examples are isotype control antibodies with anti-HEL having an hG1DM and hG4WT constant region sequence, respectively, prepared in Akeso Biopharma, Inc.

In the following examples of the present invention, MDA-MB-231 breast cancer cells, SNU-251 ovarian cancer cells and SK-OV-3 cells contain BRCA1 mutation and BRCA2 mutation.

Preparation Example 1: Preparation of anti-VEGFA antibody bevacizumab

For the amino acid sequences of the heavy chain variable region and the light chain variable region of the commercially available anti-VEGFA monoclonal antibody Avastin (bevacizumab), refer to the Chinese Patent Publication No. CN1259962A. Genscript was entrusted to synthesize the nucleic acid sequences encoding the heavy chain variable region and the light chain variable region.

Amino acid sequence of bevacizumab heavy chain variable region (Bevacizumab-Hv): (123 aa)

**EVQLVESGGGLVQPGGSLRLSCAASGYTFTNYGMNWVRQAPGK GLEWVGWINTYTGEPTYAADFKRRFTFSLDTSKSTAYLQMNSLR AEDTAVYYCAKYPHYYGSSHWYFDVWGQGTLVTVSS (SEQ ID NO: 1)**

Nucleic acid sequence encoding bevacizumab heavy chain variable region: (369 bp)

GAGGTGCAGCTGGTCGAGTCCGGGGGGGGGGCTGGTGCAGCCA
GGCGGGTCTCTGAGGCTGAGTTGCGCCGCTTCAGGGTACACC
TTCACAAACTATGGAATGAATTGGGTGCGCCAGGCACCAGGAA
AGGGACTGGAGTGGGTCGGCTGGATCAACACTTACACCGGGG
AACCTACCTATGCAGCCGACTTTAAGCGGCGGTTCACCTTCAG
CCTGGATACAAGCAAATCCACTGCCTACCTGCAGATGAACAGC
CTGCGAGCTGAGGACACCGCAGTCTACTATTGTGCTAAATATC
CCCACTACTATGGGAGCAGCCATTGGTATTTTGACGTGTGGGG
GCAGGGGACTCTGGTGACAGTGAGCAGC (SEQ ID NO: 2)

Amino acid sequence of bevacizumab light chain variable region (Bevacizumab-Lv): (107 aa)

DIQMTQSPSSLSASVGDRVTITCSASQDISNYLNWYQQKPGKAPKV
LIYFTSSLHSGVPSRFSGSGSGTDFTLTISSLQPEDFATYYCQQYST
VPWTFGQGTKVEIK (SEQ ID NO: 3)

Nucleic acid sequence encoding bevacizumab light chain variable region: (321 bp)

GATATTCAGATGACTCAGAGCCCCTCCTCCCTGTCCGCCTCTG
TGGGCGACAGGGTCACCATCACATGCAGTGCTTCACAGGATAT
TTCCAACTACCTGAATTGGTATCAGCAGAAGCCAGGAAAAGCA
CCCAAGGTGCTGATCTACTTCACTAGCTCCCTGCACTCAGGAG
TGCCAAGCCGGTTCAGCGGATCCGGATCTGGAACCGACTTTAC
TCTGACCATTTCTAGTCTGCAGCCTGAGGATTTCGCTACATAC
TATTGCCAGCAGTATTCTACCGTGCCATGGACATTTGGCCAGG
GGACTAAAGTCGAGATCAAG (SEQ ID NO: 4)

The heavy chain constant regions were all Ig gamma-1 chain C region, ACCESSION: P01857; the light chain constant regions were all Ig kappa chain C region, ACCESSION: P01834.

The heavy chain cDNA and the light chain cDNA of bevacizumab were cloned into vector pcDNA3.1, and the recombinant expression plasmid of the antibody bevacizumab was obtained. The recombinant plasmid was used to transfect 293F cells. The 293F cell culture was purified and then detected.

The anti-VEGFA monoclonal antibody Avastin (bevacizumab) was thus obtained.

Preparation Example 2: Sequence design of anti-PD-1 antibody 14C12, humanized antibody 14C12H1L1 thereof and mutant 14C12H1L1(M)

The amino acid sequences and encoding nucleic acid sequences of the heavy and light chains of anti-PD-1 antibody 14C12 and its humanized antibody 14C12H1L1 are identical to those of 14C12 and 14C12H1L1 in Chinese Patent Publication No. CN106967172A, respectively.

(1) Heavy and light chain variable region sequences of 14C12

Amino acid sequence of 14C12 heavy chain variable region: (118 aa)

**EVKLVESGGGLVKPGGSLKLSCAASGFAFSSYDMSWVRQTPEKR LEWVATISGGGRYTYYPDSVKGRFTISRDNARNTLYLQMSSLRSE DTALYYCANRYGEAWFAYWGQGTLVTVSA (SEQ ID NO: 5)**

Nucleic acid sequence encoding 14C12 heavy chain variable region: (354 bp)

**GAGGTCAAACTGGTGGAGAGCGGCGGCGGGCTGGTGAAGCCC GGCGGGTCACTGAAACTGAGCTGCGCCGCTTCCGGCTTCGCCT TTAGCTCCTACGACATGTCATGGGTGAGGCAGACCCCTGAGAA GCGCCTGGAATGGGTCGCTACTATCAGCGGAGGCGGGCGATA CACCTACTATCCTGACTCTGTCAAAGGGAGATTCACAATTAGT CGGGATAACGCCAGAAATACTCTGTATCTGCAGATGTCTAGTC TGCGGTCCGAGGATACAGCTCTGTACTATTGTGCAAACCGGTA CGGCGAAGCATGGTTTGCCTATGGGGACAGGGCACCCTGGT GACAGTCTCTGCC (SEQ ID NO: 6)**

Amino acid sequence of 14C12 light chain variable region: (107 aa)

**DIKMTQSPSSMYASLGERVTFTCKASQDINTYLSWFQQKPGKSPK TLIYRANRLVDGVPSRFSGSGSGQDYSLTISSLEYEDMGIYYCLQY DEFPLTFGAGTKLELK (SEQ ID NO: 7)**

Nucleic acid sequence encoding 14C12 light chain variable region: (321 bp)

**GACATTAAGATGACACAGTCCCCTTCCTCAATGTACGCTAGCC**

TGGGCGAGCGAGTGACCTTCACATGCAAAGCATCCCAGGACATCAACACATACCTGTCTTGGTTTCAGCAGAAGCCAGGCAAAAGCCCCAAGACCCTGATCTACCGGGCCAATAGACTGGTGGACGGGGTCCCCAGCAGATTCTCCGGATCTGGCAGTGGGCAGGATTACTCCCTGACCATCAGCTCCCTGGAGTATGAAGACATGGGCATCTACTATTGCCTGCAGTATGATGAGTTCCCTCTGACCTTTGGAGCAGGCACAAAACTGGAACTGAAG (SEQ ID NO: 8)

(2) Heavy and light chain variable region and heavy and light chain sequences of humanized monoclonal antibody 14C12H1L1

Amino acid sequence of 14C12H1L1 heavy chain variable region: (118 aa)

EVQLVESGGGLVQPGGSLRLSCAASGFAFSSYDMSWVRQAPGKGLDWVATISGGGRYTYYPDSVKGRFTISRDNSKNNLYLQMNSLRAEDTALYYCANRYGEAWFAYWGQGTLVTVSS (SEQ ID NO: 9)

Nucleic acid sequence encoding 14C12H1L1 heavy chain variable region: (354 bp)

GAAGTGCAGCTGGTCGAGTCTGGGGGAGGGCTGGTGCAGCCCGGCGGGTCACTGCGACTGAGCTGCGCAGCTTCCGGATTCGCCTTTAGCTCCTACGACATGTCCTGGGTGCGACAGGCACCAGGAAAGGGACTGGATTGGGTCGCTACTATCTCAGGAGGCGGGAGATACACCTACTATCCTGACAGCGTCAAGGGCCGGTTCACAATCTCTAGAGATAACAGTAAGAACAATCTGTATCTGCAGATGAACAGCCTGAGGGCTGAGGACACCGCACTGTACTATTGTGCCAACCGCTACGGGGAAGCATGGTTTGCCTATTGGGGGCAGGGAACCCTGGTGACAGTCTCTAGT (SEQ ID NO: 10)

Amino acid sequence of 14C12H1L1 light chain variable region: (107 aa)

DIQMTQSPSSMSASVGDRVTFTCRASQDINTYLSWFQQKPGKSPKTLIYRANRLVSGVPSRFSGSGSGQDYTLTISSLQPEDMATYYCLQYDEFPLTFGAGTKLELK (SEQ ID NO: 11)

Nucleic acid sequence encoding 14C12H1L1 light chain variable region: (321 bp)

GACATTCAGATGACTCAGAGCCCCTCCTCCATGTCCGCCTCTG TGGGCGACAGGGTCACCTTCACATGCCGCGCTAGTCAGGATAT CAACACCTACCTGAGCTGGTTTCAGCAGAAGCCAGGGAAAAGC CCCAAGACACTGATCTACCGGGCTAATAGACTGGTGTCTGGAG TCCCAAGTCGGTTCAGTGGCTCAGGGAGCGGACAGGACTACA CTCTGACCATCAGCTCCCTGCAGCCTGAGGACATGGCAACCTA CTATTGCCTGCAGTATGATGAGTTCCCACTGACCTTTGGCGCC GGGACAAAACTGGAGCTGAAG (SEQ ID NO: 12)

Amino acid sequence of 14C12H1L1 heavy chain (14C12H1): (448 aa)

EVQLVESGGGLVQPGGSLRLSCAASGFAFSSYDMSWVRQAPGKG LDWVATISGGGRYTYYPDSVKGRFTISRDNSKNNLYLQMNSLRAE DTALYYCANRYGEAWFAYWGQGTLVTVSSASTKGPSVFPLAPSS KSTSGGTAALGCLVKDYFPEPVTVSWNSGALTSGVHTFPAVLQSS GLYSLSSVVTVPSSSLGTQTYICNVNHKPSNTKVDKKVEPKSCDKT HTCPPCPAPELLGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALPAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG K (SEQ ID NO: 13)

Nucleic acid sequence encoding 14C12H1L1 heavy chain (14C12H1): (1344 bp)

GAAGTGCAGCTGGTCGAGTCTGGGGGAGGGCTGGTGCAGCCC
GGCGGGTCACTGCGACTGAGCTGCGCAGCTTCCGGATTCGCC
TTTAGCTCCTACGACATGTCCTGGGTGCGACAGGCACCAGGAA
AGGGACTGGATTGGGTCGCTACTATCTCAGGAGGCGGGAGAT
ACACCTACTATCCTGACAGCGTCAAGGGCCGGTTCACAATCTC
TAGAGATAACAGTAAGAACAATCTGTATCTGCAGATGAACAGC
CTGAGGGCTGAGGACACCGCACTGTACTATTGTGCCAACCGCT
ACGGGGAAGCATGGTTTGCCTATTGGGGGCAGGGAACCCTGG
TGACAGTCTCTAGTGCCAGCACCAAAGGACCTAGCGTGTTTCC
TCTCGCCCCTCCTCCAAAAGCACCAGCGGAGGAACCGCTGCT
CTCGGATGTCTGGTGAAGGACTACTTCCCTGAACCCGTCACCG
TGAGCTGGAATAGCGGCGCTCTGACAAGCGGAGTCCATACATT
CCCTGCTGTGCTGCAAAGCAGCGGACTCTATTCCCTGTCCAGC
GTCGTCACAGTGCCCAGCAGCAGCCTGGGCACCCAGACCTAC
ATCTGTAACGTCAACCACAAGCCCTCCAACACCAAGGTGGACA
AGAAAGTGGAGCCCAAATCCTGCGACAAGACACACACCTGTCC
CCCCTGTCCTGCTCCCGAACTCCTCGGAGGCCCTAGCGTCTTC
CTCTTTCCTCCCAAACCCAAGGACACCCTCATGATCAGCAGAA
CCCCTGAAGTCACCTGTGTCGTCGTGGATGTCAGCCATGAGGA
CCCCGAGGTGAAATTCAACTGGTATGTCGATGGCGTCGAGGTG
CACAACGCCAAAACCAAGCCCAGGGAGGAACAGTACAACTCC
ACCTACAGGGTGGTGTCCGTGCTGACAGTCCTCCACCAGGACT
GGCTGAACGGCAAGGAGTACAAGTGCAAGGTGTCCAACAAGG
CTCTCCCTGCCCCCATTGAGAAGACCATCAGCAAGGCCAAAGG
CCAACCCAGGGAGCCCAGGTCTATACTGCCTCCCTCCAGG
GACGAACTCACCAAGAACCAGGTGTCCCTGACCTGCCTGGTCA
AGGGCTTTTATCCCAGCGACATCGCCGTCGAGTGGGAGTCCAA
CGGACAGCCCGAGAATAACTACAAGACCACCCCTCCTGTCCTC

GACTCCGACGGCTCCTTCTTCCTGTACAGCAAGCTGACCGTGG ACAAAAGCAGGTGGCAGCAGGGAAACGTGTTCTCCTGCAGCG TGATGCACGAAGCCCTCCACAACCACTACACCCAGAAAAGCCT GTCCCTGAGCCCCGGCAAA (SEQ ID NO: 14)

Amino acid sequence of 14C12H1L1 light chain (14C12L1): (214 aa)

DIQMTQSPSSMSASVGDRVTFTCRASQDINTYLSWFQQKPGKSPK TLIYRANRLVSGVPSRFSGSGSGQDYTLTISSLQPEDMATYYCLQY DEFPLTFGAGTKLELKRTVAAPSVFIFPPSDEQLKSGTASVVCLLN NFYPREAKVQWKVDNALQSGNSQESVTEQDSKDSTYSLSSTLTLS KADYEKHKVYACEVTHQGLSSPVTKSFNRGEC (SEQ ID NO: 15)

Nucleic acid sequence encoding 14C12H1L1 light chain (14C12L1): (642 bp)

GACATTCAGATGACTCAGAGCCCCTCCTCCATGTCCGCCTCTG TGGGCGACAGGGTCACCTTCACATGCCGCGCTAGTCAGGATAT CAACACCTACCTGAGCTGGTTTCAGCAGAAGCCAGGGAAAAGC CCCAAGACACTGATCTACCGGGCTAATAGACTGGTGTCTGGAG TCCCAAGTCGGTTCAGTGGCTCAGGGAGCGGACAGGACTACA CTCTGACCATCAGCTCCTGCAGCCTGAGGACATGGCAACCTA CTATTGCCTGCAGTATGATGAGTTCCCACTGACCTTTGGCGCC GGGACAAAACTGGAGCTGAAGCGAACTGTGGCCGCTCCCTCC GTCTTCATTTTTCCCCCTTCTGACGAACAGCTGAAATCAGGCA CAGCCAGCGTGGTCTGTCTGCTGAACAATTTCTACCCTAGAGA GGCAAAAGTGCAGTGGAAGGTCGATAACGCCCTGCAGTCCGG CAACAGCCAGGAGAGTGTGACTGAACAGGACTCAAAAGATAG CACCTATTCCCTGTCTAGTACACTGACTCTGTCCAAGGCTGAT TACGAGAAGCACAAAGTGTATGCATGCGAAGTGACACATCAGG GACTGTCAAGCCCCGTGACTAAGTCTTTTAACCGGGGCGAATG T (SEQ ID NO: 16)

(3) Heavy and light chain variable region sequences of 14C12H1L1(M) 14C12H1L1(M) was obtained by a mutation on an amino acid in the framework region (light chain) of 14C12H1L1.

Heavy chain variable region 14C12H1(M) of 14C12H1L1(M):
The sequence is identical to the heavy chain variable region 14C12H1 of 14C12H1L1. That is, the amino acid sequence is set forth in SEQ ID NO: 9.
Light chain variable region 14C12L1(M) of 14C12H1L1(M): (108 aa; the mutant position on the basis of 14C12H1L1 underlined in the amino acid sequence)

# DIQMTQSPSSMSASVGDRVTFTCRASQDINTYLSWFQQKPGKSPK TLIYRANRLVSGVPSRFSGSGSGQDYTLTISSLQPEDMATYYCLQY DEFPLTFGAGTKLELKR (SEQ ID NO: 17)

Preparation Example 3: Sequence design of bispecific antibodies

1. Sequence design

The structure of the bispecific antibody described herein is in the Morrison form (IgG-scFv), i.e., the C-termini of the two heavy chains of one IgG antibody are each linked to the scFv fragments of the other antibody, and the design of the heavy and light chains is as shown in Table 1 below.
On the basis of the bevacizumab described above, the VP101 antibody comprises the amino acid sequences of the heavy chain variable region and the light chain variable region of the 14C12H1L1(M) as the scFv fragments, and is referred to as VP101(M). Compared with 14C12H1L1, 14C12H1L1(M) demonstrated effectively optimized bispecific antibody structure and improved efficacy.

Table 1: Design of heavy and light chains of VP101M and VP101(G4M)

| Bispecific antibody No. | Immunoglobulin moiety | | Linker fragment | scFv moiety |
| --- | --- | --- | --- | --- |
| | Heavy chain | Light chain | | |
| VP101(M) | Bevacizumab-H | Bevacizumab-L | Linker1 | 14C12H1(M)$_V$-Linker1-14C12L1(M)$_V$ |
| VP101(G4M) | Bevacizumab-G4H | Bevacizumab-L | Linker1 | 14C12H1(M)$_V$-Linker1-14C12L1(M)$_V$ |

In Table 1 above:

(1) Those with "V" label at lower right corner refer to the variable region of the corresponding heavy chain or the variable region of the corresponding light chain. For those without "V" label, the corresponding heavy or light chain is the full length comprising the constant region. The corresponding sequences described in the above preparation examples are referred to for the amino acid sequences of these variable regions or the full lengths and the nucleic acid sequences encoding them.
(2) The amino acid sequence of Linker1 is GGGGSGGGGSGGGGSG GGGS (SEQ ID NO: 18)
Optionally, amino acid sequence GGGGSGGGGSGGGGS (SEQ ID NO: 19) may be used as Linker2 for replacing the aforementioned Linker1.
(3) Bevacizumab-H comprises Ig gamma-1 chain C region (ACCESSION: P01857) as the heavy chain constant region.
(4) Bevacizumab-G4H comprises Ig gamma-4 chain C region (ACCESSION: P01861.1) as the heavy chain constant region.

2. Expression and purification of antibody VP101(M)

The heavy chain cDNA sequence and the light chain cDNA sequence of VP101(M) were each cloned into vector pUC57simple (provided by Genscript) to obtain plasmids pUC57simple-VP101H and pUC57simple-VP101L, respectively.

Plasmids pUC57simple-VP101H and pUC57simple-VP101L were enzymatically digested (HindIII&EcoRI). Heavy and light chains isolated by electrophoresis were subcloned into vector pcDNA3.1, and the recombinant plasmids were extracted to co-transfect 293F cells. After 7 days of cell culture, the culture medium was centrifuged at high speed, and the supernatant was concentrated and loaded onto a HiTrap MabSelect SuRe column. The protein was further subjected to a one-step elution with an elution buffer, and the target sample antibody VP101 was isolated and transferred into PBS by buffer exchange.

3. Detection of antibody VP101(M)

The purified sample was added to both a reduced protein electrophoresis loading buffer and a non-reduced protein electrophoresis loading buffer, boiled and subjected to SDS-PAGE electrophoresis analysis.

For differentiation from the mutant antibody in Preparation Example 4, VP101(M) is also referred to as VP101(hG1WT) in the present invention. VP101(M) described above is a "wild-type", comprising an Ig gamma-1 chain C region (ACCESSION: P01857) as the heavy chain constant region and an Ig kappa chain C region (ACCESSION: P01834) as the light chain constant region.

Amino acid sequence of the heavy chain of the immunoglobulin moiety in VP101(hG1WT): (453 aa)

**EVQLVESGGGLVQPGGSLRLSCAASGYTFTNYGMNWVRQAPGK GLEWVGWINTYTGEPTYAADFKRRFTFSLDTSKSTAYLQMNSLR AEDTAVYYCAKYPHYYGSSHWYFDVWGQGTLVTVSSASTKGPSV FPLAPSSKSTSGGTAALGCLVKDYFPEPVTVSWNSGALTSGVHTFP AVLQSSGLYSLSSVVTVPSSSLGTQTYICNVNHKPSNTKVDKKVEP KSCDKTHTCPPCPAPELLGGPSVFLFPPKPKDTLMISRTPEVTCVV VDVSHEDPEVKFNWYVDGVEVHNAKTKPREEQYNSTYRVVSVLT VLHQDWLNGKEYKCKVSNKALPAPIEKTISKAKGQPREPQVYTL PPSRDELTKNQVSLTCLVKGFYPSDIAVEWESNGQPENNYKTTPP VLDSDGSFFLYSKLTVDKSRWQQGNVFSCSVMHEALHNHYTQKS LSLSPGK (SEQ ID NO: 20)**

Nucleic acid sequence encoding the heavy chain of the immunoglobulin moiety in VP101(hG1WT): (1359 bp)

GAGGTGCAGCTGGTCGAGTCCGGGGGGGGGGCTGGTGCAGCCA
GGCGGGTCTCTGAGGCTGAGTTGCGCCGCTTCAGGGTACACC
TTCACAAACTATGGAATGAATTGGGTGCGCCAGGCACCAGGAA
AGGGACTGGAGTGGGTCGGCTGGATCAACACTTACACCGGGG
AACCTACCTATGCAGCCGACTTTAAGCGGCGGTTCACCTTCAG
CCTGGATACAAGCAAATCCACTGCCTACCTGCAGATGAACAGC
CTGCGAGCTGAGGACACCGCAGTCTACTATTGTGCTAAATATC
CCCACTACTATGGGAGCAGCCATTGGTATTTTGACGTGTGGGG
GCAGGGGACTCTGGTGACAGTGAGCAGCGCAAGCACCAAAGG
GCCCAGCGTGTTTCCTCTCGCCCCCTCCTCCAAAAGCACCAGC
GGAGGAACCGCTGCTCTCGGATGTCTGGTGAAGGACTACTTCC
CTGAACCCGTCACCGTGAGCTGGAATAGCGGCGCTCTGACAA
GCGGAGTCCATACATTCCCTGCTGTGCTGCAAAGCAGCGGACT
CTATTCCCTGTCCAGCGTCGTCACAGTGCCCAGCAGCAGCCTG
GGCACCCAGACCTACATCTGTAACGTCAACCACAAGCCCTCCA

ACACCAAGGTGGACAAGAAAGTGGAGCCCAAATCCTGCGACA
AGACACACCTGTCCCCCCTGTCCTGCTCCCGAACTCCTCGG
AGGCCCTAGCGTCTTCCTCTTTCCTCCCAAACCCAAGGACACC
CTCATGATCAGCAGAACCCCTGAAGTCACCTGTGTCGTCGTGG
ATGTCAGCCATGAGGACCCCGAGGTGAAATTCAACTGGTATGT
CGATGGCGTCGAGGTGCACAACGCCAAAACCAAGCCCAGGGA
GGAACAGTACAACTCCACCTACAGGGTGGTGTCCGTGCTGACA
GTCCTCCACCAGGACTGGCTGAACGGCAAGGAGTACAAGTGC
AAGGTGTCCAACAAGGCTCTCCCTGCCCCCATTGAGAAGACCA
TCAGCAAGGCCAAAGGCCAACCCAGGGAGCCCAGGTCTATA
CACTGCCTCCCTCCAGGGACGAACTCACCAAGAACCAGGTGTC
CCTGACCTGCCTGGTCAAGGGCTTTTATCCCAGCGACATCGCC
GTCGAGTGGGAGTCCAACGGACAGCCCGAGAATAACTACAAG
ACCACCCCTCCTGTCCTCGACTCCGACGGCTCCTTCTTCCTGT
ACAGCAAACTGACCGTCGATAAATCTAGGTGGCAGCAGGGCA
ACGTGTTCTCTTGTTCCGTGATGCATGAAGCACTGCACAACCA
TTATACCCAGAAGTCTCTGAGCCTGTCCCCCGGCAAG (SEQ ID NO: 21)

For differentiation from the mutant antibody in Preparation Example 4, VP101(G4M) is also referred to as VP101(hG4WT) in the present invention. VP101(G4M) described above is a "wild-type", comprising an Ig gamma-4 chain C region (ACCESSION: P01861.1) as the heavy chain constant region and an Ig kappa chain C region (ACCESSION: P01834) as the light chain constant region.

Amino acid sequence of the heavy chain of the immunoglobulin moiety in VP101(hG4WT): (450 aa)

EVQLVESGGGLVQPGGSLRLSCAASGYTFTNYGMNWVRQAPGK

GLEWVGWINTYTGEPTYAADFKRRFTFSLDTSKSTAYLQMNSLR
AEDTAVYYCAKYPHYYGSSHWYFDVWGQGTLVTVSSASTKGPSV
FPLAPCSRSTSESTAALGCLVKDYFPEPVTVSWNSGALTSGVHTFP
AVLQSSGLYSLSSVVTVPSSSLGTKTYTCNVDHKPSNTKVDKRVES
KYGPPCPPCPAPEFLGGPSVFLFPPKPKDTLMISRTPEVTCVVVDV
SQEDPEVQFNWYVDGVEVHNAKTKPREEQFNSTYRVVSVLTVLH
QDWLNGKEYKCKVSNKGLPSSIEKTISKAKGQPREPQVYTLPPSQ
EEMTKNQVSLTCLVKGFYPSDIAVEWESNGQPENNYKTTPPVLDS
DGSFFLYSRLTVDKSRWQEGNVFSCSVMHEALHNHYTQKSLSLSL
GK (SEQ ID NO: 22)

The nucleic acid sequence encoding the heavy chain of the immunoglobulin moiety in VP101 (hG4WT): (1350 bp)

GAGGTGCAGCTGGTCGAGTCCGGGGGGGGGCTGGTGCAGCCA
GGCGGGTCTCTGAGGCTGAGTTGCGCCGCTTCAGGGTACACC
TTCACAAACTATGGAATGAATTGGGTGCGCCAGGCACCAGGAA
AGGGACTGGAGTGGGTCGGCTGGATCAACACTTACACCGGGG
AACCTACCTATGCAGCCGACTTTAAGCGGCGGTTCACCTTCAG
CCTGGATACAAGCAAATCCACTGCCTACCTGCAGATGAACAGC
CTGCGAGCTGAGGACACCGCAGTCTACTATTGTGCTAAATATC
CCCACTACTATGGGAGCAGCCATTGGTATTTTGACGTGTGGGG
GCAGGGGACTCTGGTGACAGTGAGCAGCGCAAGCACCAAAGG
GCCCTCGGTCTTCCCCCTGGCGCCTGCTCCAGGAGCACCTCC
GAGAGCACAGCCGCCCTGGGCTGCCTGGTCAAGGACTACTTC
CCCGAACCGGTGACGGTGTCGTGGAACTCAGGCGCCCTGACC
AGCGGCGTGCACACCTTCCCGGCTGTCCTACAGTCCTCAGGAC
TCTACTCCCTCAGCAGCGTGGTGACCGTGCCCTCCAGCAGCTT
GGGCACGAAGACCTACACCTGCAACGTAGATCACAAGCCCAG
CAACACCAAGGTGGACAAGAGAGTTGAGTCCAAATATGGTCCC

CCATGCCCACCATGCCCAGCACCTGAGTTCCTGGGGGGACCAT
CAGTCTTCCTGTTCCCCCAAAACCCAAGGACACTCTCATGAT
CTCCCGGACCCCTGAGGTCACGTGCGTGGTGGTGGACGTGAG
CCAGGAAGACCCCGAGGTCCAGTTCAACTGGTACGTGGATGG
CGTGGAGGTGCATAATGCCAAGACAAAGCCGCGGGAGGAGCA
GTTCAACAGCACGTACCGTGTGGTCAGCGTCCTCACCGTCCTG
CACCAGGACTGGCTGAACGGCAAGGAGTACAAGTGCAAGGTC
TCCAACAAAGGCCTCCCGTCCTCCATCGAGAAAACCATCTCCA
AAGCCAAAGGGCAGCCCCGAGAGCCACAGGTGTACACCCTGC
CCCCATCCCAGGAGGAGATGACCAAGAACCAGGTCAGCCTGA
CCTGCCTGGTCAAAGGCTTCTACCCCAGCGACATCGCCGTGGA
GTGGGAGAGCAATGGGCAGCCGGAGAACAACTACAAGACCAC
GCCTCCCGTGCTGGACTCCGACGGCTCCTTCTTCCTCTACAGC
AGGCTAACCGTGGACAAGAGCAGGTGGCAGGAGGGGAATGTC
TTCTCATGCTCCGTGATGCATGAGGCTCTGCACAACCACTACA
CACAGAAGTCTCTGAGCCTGTCCCTCGGCAAG (SEQ ID NO: 23)

Preparation Example 4: Non-variable region amino acid mutation design based on humanized bispecific antibody VP101(hG1WT)

On the basis of VP101(hG1WT) obtained in Preparation Example 3, VP101(hG1DM) was obtained by introducing a leucine-to-alanine point mutation at position 234 (L234A) and a leucine-to-alanine point mutation at position 235 (L235A) in the heavy chain.

Amino acid sequence of the heavy chain of the immunoglobulin moiety in VP101(hG1DM): (453 aa, mutation positions underlined)

EVQLVESGGGLVQPGGSLRLSCAASGYTFTNYGMNWVRQAPGK
GLEWVGWINTYTGEPTYAADFKRRFTFSLDTSKSTAYLQMNSLR
AEDTAVYYCAKYPHYYGSSHWYFDVWGQGTLVTVSSASTKGPSV

FPLAPSSKSTSGGTAALGCLVKDYFPEPVTVSWNSGALTSGVHTFP
AVLQSSGLYSLSSVVTVPSSSLGTQTYICNVNHKPSNTKVDKKVEP
KSCDKTHTCPPCPAPE__AA__GGPSVFLFPPKPKDTLMISRTPEVTCVV
VDVSHEDPEVKFNWYVDGVEVHNAKTKPREEQYNSTYRVVSVLT
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGK (SEQ ID NO: 24)

Nucleic acid sequence encoding the heavy chain of the immunoglobulin moiety in VP101(hG1DM): (1359 bp, mutation positions underlined)

GAGGTGCAGCTGGTCGAGTCCGGGGGGGGGCTGGTGCAGCCA
GGCGGGTCTCTGAGGCTGAGTTGCGCCGCTTCAGGGTACACC
TTCACAAACTATGGAATGAATTGGGTGCGCCAGGCACCAGGAA
AGGGACTGGAGTGGGTCGGCTGGATCAACACTTACACCGGGG
AACCTACCTATGCAGCCGACTTTAAGCGGCGGTTCACCTTCAG
CCTGGATACAAGCAAATCCACTGCCTACCTGCAGATGAACAGC
CTGCGAGCTGAGGACACCGCAGTCTACTATTGTGCTAAATATC
CCCACTACTATGGGAGCAGCCATTGGTATTTTGACGTGTGGGG
GCAGGGGACTCTGGTGACAGTGAGCAGCGCAAGCACCAAAGG
GCCCAGCGTGTTTCCTCTCGCCCCCTCCTCCAAAAGCACCAGC
GGAGGAACCGCTGCTCTCGGATGTCTGGTGAAGGACTACTTCC
CTGAACCCGTCACCGTGAGCTGGAATAGCGGCGCTCTGACAA
GCGGAGTCCATACATTCCCTGCTGTGCTGCAAAGCAGCGGACT
CTATTCCCTGTCCAGCGTCGTCACAGTGCCCAGCAGCAGCCTG
GGCACCCAGACCTACATCTGTAACGTCAACCACAAGCCCTCCA
ACACCAAGGTGGACAAGAAGTGGAGCCCAAATCCTGCGACA
AGACACACACCTGTCCCCCTGTCCTGCTCCCGAA__GCTGCTGG__
AGGCCCTAGCGTCTTCCTCTTTCCTCCCAAACCCAAGGACACC

CTCATGATCAGCAGAACCCCTGAAGTCACCTGTGTCGTCGTGG ATGTCAGCCATGAGGACCCCGAGGTGAAATTCAACTGGTATGT CGATGGCGTCGAGGTGCACAACGCCAAAACCAAGCCCAGGGA GGAACAGTACAACTCCACCTACAGGGTGGTGTCCGTGCTGACA GTCCTCCACCAGGACTGGCTGAACGGCAAGGAGTACAAGTGC AAGGTGTCCAACAAGGCTCTCCCTGCCCCCATTGAGAAGACCA TCAGCAAGGCCAAGGCCAACCCAGGGAGCCCCAGGTCTATA CACTGCCTCCCTCCAGGGACGAACTCACCAAGAACCAGGTGTC CCTGACCTGCCTGGTCAAGGGCTTTTATCCCAGCGACATCGCC GTCGAGTGGGAGTCCAACGGACAGCCCGAGAATAACTACAAG ACCACCCCTCCTGTCCTCGACTCCGACGGCTCCTTCTTCCTGT ACAGCAAACTGACCGTCGATAAATCTAGGTGGCAGCAGGGCA ACGTGTTCTCTTGTTCCGTGATGCATGAAGCACTGCACAACCA TTATACCCAGAAGTCTCTGAGCCTGTCCCCCGGCAAG (SEQ ID NO: 25)

The amino acid sequences of the light chains of the immunoglobulin moieties of VP101(hG1DM), VP101(hG1WT) and VP101(hG4WT) are identical, and the encoding nucleic acid sequences are also identical. Amino acid sequence of the light chain of the immunoglobulin moiety in VP101(hG1DM): (214 aa)

DIQMTQSPSSLSASVGDRVTITCSASQDISNYLNWYQQKPGKAPKV LIYFTSSLHSGVPSRFSGSGSGTDFTLTISSLQPEDFATYYCQQYST VPWTFGQGTKVEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGEC (SEQ ID NO: 26)

Nucleic acid sequence encoding the light chain of the immunoglobulin moiety in VP101(hG1DM): (642 bp)

GATATTCAGATGACTCAGAGCCCCTCCTCCCTGTCCGCCTCTG

TGGGCGACAGGGTCACCATCACATGCAGTGCTTCACAGGATAT
TTCCAACTACCTGAATTGGTATCAGCAGAAGCCAGGAAAAGCA
CCCAAGGTGCTGATCTACTTCACTAGCTCCCTGCACTCAGGAG
TGCCAAGCCGGTTCAGCGGATCCGGATCTGGAACCGACTTTAC
TCTGACCATTTCTAGTCTGCAGCCTGAGGATTTCGCTACATAC
TATTGCCAGCAGTATTCTACCGTGCCATGGACATTTGGCCAGG
GGACTAAAGTCGAGATCAAGCGGACCGTGGCCGCTCCCAGTG
TCTTCATTTTTCCCCCTAGCGACGAACAGCTGAAATCCGGGAC
AGCCTCTGTGGTCTGTCTGCTGAACAACTTCTACCCTAGAGAG
GCAAAAGTGCAGTGGAAGGTCGATAACGCCCTGCAGAGTGGC
AATTCACAGGAGAGCGTGACAGAACAGGACTCCAAAGATTCTA
CTTATAGTCTGTCAAGCACACTGACTCTGAGCAAGGCTGACTA
CGAAAAGCATAAAGTGTATGCATGTGAGGTCACCCACCAGGG
GCTGAGCAGTCCAGTCACCAAGTCATTCAACAGAGGCGAGTGC
**(SEQ ID NO: 27)**

Example 1: Affinity constant assay of FcγRI to VP101(hG1WT) and VP101(hG1DM)

The Fc receptor FcγRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody.

The affinity constants of VP101(hG1WT) and VP101(hG1DM) to FcγRI were measured using a Fortebio Octet system to evaluate the ADCC activity of the antibodies.

The method for determining the affinity constant of the antibodies to FcγRI by the Fortebio Octet system is briefly described as follows: The sample dilution buffer was a solution of 0.02% Tween-20 and 0.1% BSA in PBS, pH 7.4. 1 μg/mL FcγRIa was immobilized on the HISIK sensor for 50 s. The sensor was equilibrated in a buffer for 60 s, and the binding of the immobilized CD64 on the sensor to the antibodies at concentrations of 3.12-50 nM (two-fold dilution) was determined for 120 s. The antibody was dissociated in the buffer for 120 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

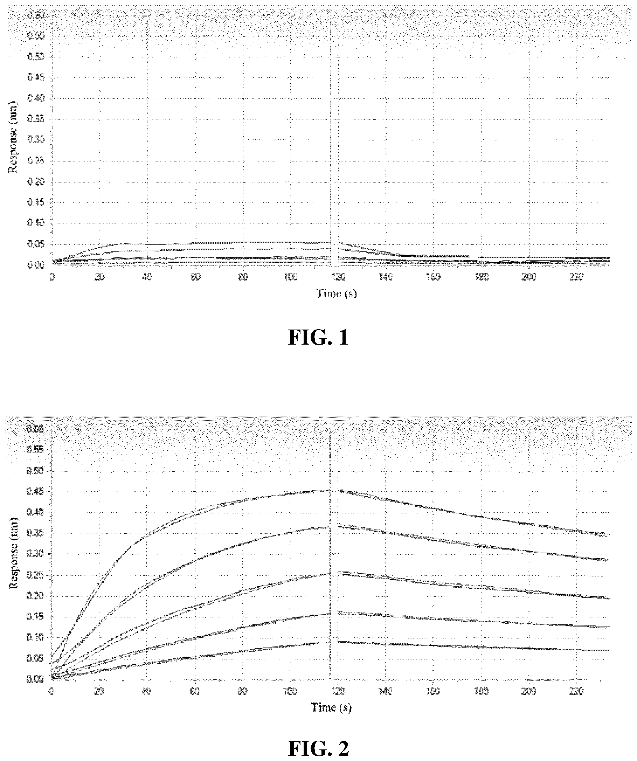

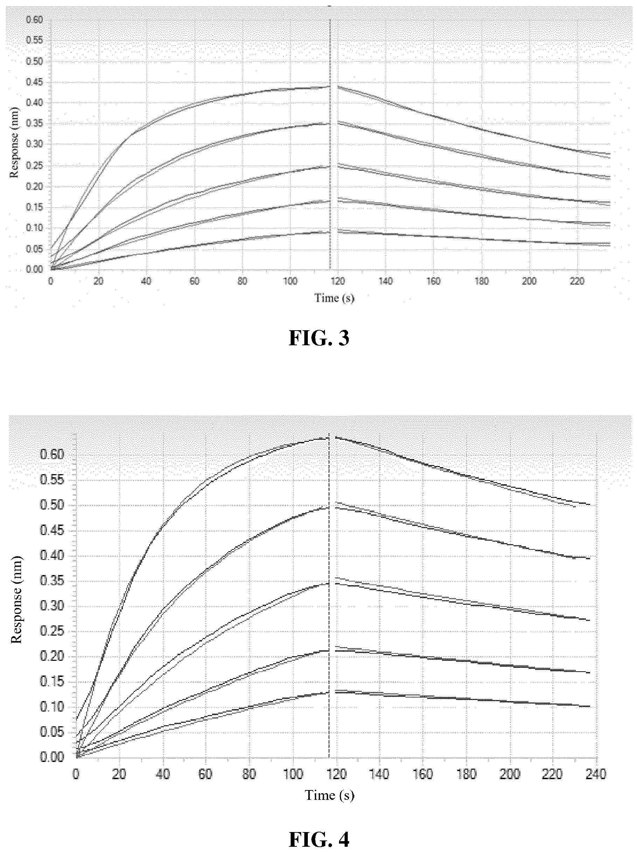

The results of affinity constant assay of FcγRI to VP101(hG1WT), VP101(hG4WT) and VP101(hG1DM), and reference antibodies nivolumab and bevacizumab are shown in Table 1 and FIGs. 1 to 5.

Table 1: Kinetics for binding of VP101(hG1WT), VP101(hG1DM) and subtypes thereof to FcγRI

| Antibody | $K_D$ (M) | kon(1/Ms) | SE (kon) | kdis(1/s) | SE(kdis) | Rmax(nm) |
|---|---|---|---|---|---|---|
| VP101(hG1DM) | N/A | N/A | N/A | N/A | N/A | N/A |
| Bevacizumab | 3.68E-09 | 6.61E+05 | 2.07E+04 | 2.44E-03 | 9.02E-05 | 0.46-0.49 |
| Nivolumab | 6.20E-09 | 6.98E+05 | 2.22E+04 | 4.32E-03 | 9.88E-05 | 0.48-0.53 |
| VP101(hG1WT) | 3.95E-09 | 5.67E+05 | 1.82E+04 | 2.24E-03 | 9.02E-05 | 0.68-0.80 |

(continued)

| Antibody | $K_D$ (M) | kon(1/Ms) | SE (kon) | kdis(1/s) | SE(kdis) | Rmax(nm) |
|---|---|---|---|---|---|---|
| VP101(hG4WT) | 8.52E-09 | 6.28E+05 | 2.12E+04 | 5.35E-03 | 1.07E-04 | 0.61-0.65 |

N/A indicates that the antibody had no binding or an extremely weak binding signal to the antigen. Thus the results were not analyzed and no corresponding data was obtained.

The results showed that VP101(hG1WT) bound to FcγRI with an affinity constant of 3.95E-09M; VP101(hG4WT) bound to FcγRI with an affinity constant of 8.52E-09M; bevacizumab bound to FcγRI with an affinity constant of 3.68E-09M; nivolumab bound to FcγRI with an affinity constant of 6.20E-09M; VP101(hG1DM) had no binding or an extremely weak binding signal to FcγRI, and thus the results were not analyzed and no corresponding data was obtained.

The results demonstrated that the antibodies other than VP101(hG1DM) that has no binding to FcγRI have similar bindings to FcγRI. The binding activity of VP101(hG1DM) was effectively eliminated.

Example 2: Affinity constant assay of FcγRIIa_H131 to VP101(hG1WT) and VP101(hG1DM)

The Fc receptor FcγRIIa_H131 (also known as CD32a_H131), can bind to the Fc fragment of IgG antibodies and mediate ADCC effects.

The affinity constants of VP101(hG1WT) and VP101(hG1DM) to FcγRIIa_H131 were measured using a Fortebio Octet system to evaluate the ADCC activity of the antibodies.

The method for determining the affinity constant of VP101(hG1WT) and VP101(hG1DM) to FcγRIIa_H131 by the Fortebio Octet system is briefly described as follows: The immobilization dilution buffer was a solution of 0.02% Tween-20 and 0.1% BSA in PBS, pH 7.4, and the analyte dilution buffer was a solution of 0.02% Tween-20, 0.02% casein and 0.1% BSA in PBS, pH 7.4. 5 μg/mL FcγRIIa_H131 was immobilized on the NTA sensor for an immobilization time of about 60 nm. The sensor was equilibrated in a buffer of 0.02% Tween-20, 0.02% casein and 0.1% BSA in PBS pH 7.4 for 600 s for blocking, and the binding of the immobilized FcγRIIa_H131 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

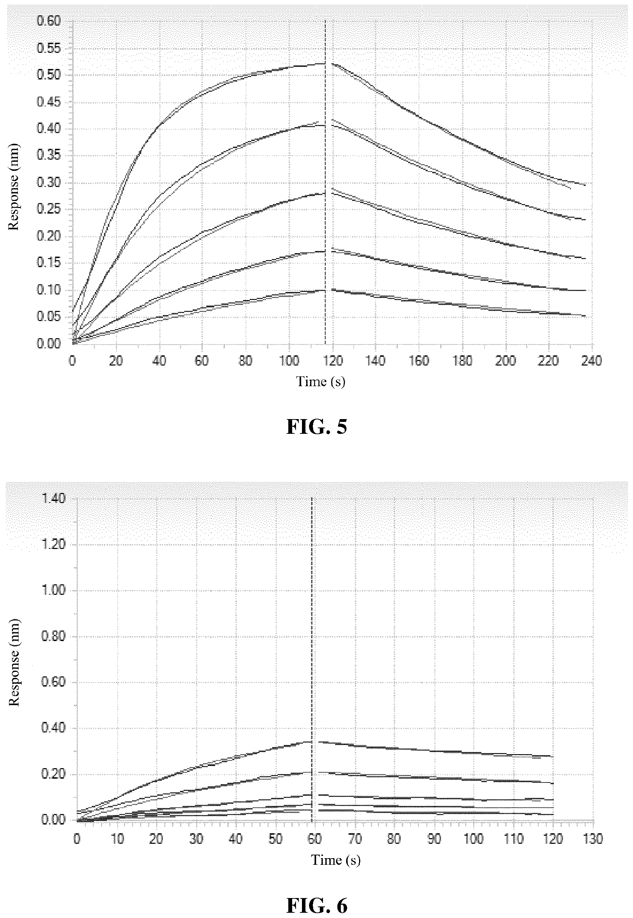

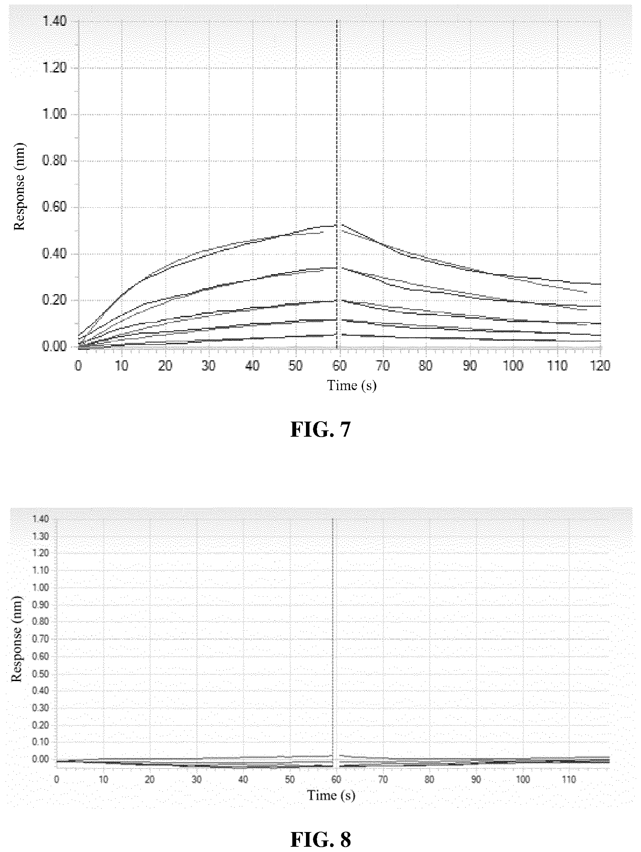

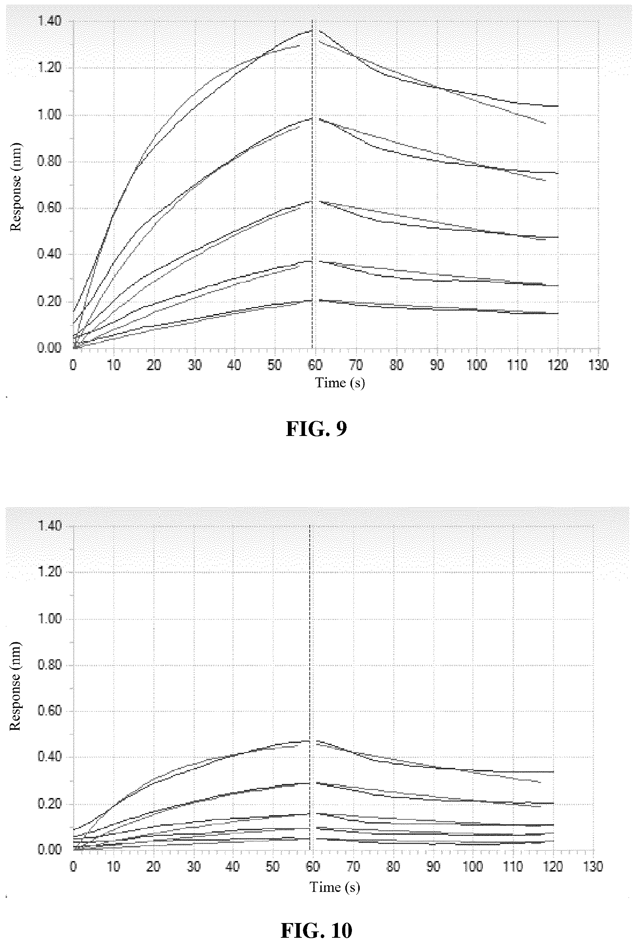

The results of affinity constant assay of FcγRIIa_H131 to VP101(hG1WT), VP101(hG4WT) and VP101(hG1DM), and reference antibodies nivolumab and bevacizumab are shown in Table 2 and FIGs. 6 to 10.

Table 2: Kinetics for binding of VP101(hG1WT), VP101(hG1DM) and subtypes thereof to FcRIIa H131

| Antibody | $K_D$ (M) | kon(1/Ms) | SE (kon) | kdis(1/s) | SE(kdis) | Rmax(nm) |
|---|---|---|---|---|---|---|
| VP101(hG1DM) | 3.57E-08 | 1.15E+05 | 1.01E+04 | 4.11E-03 | 3.27E-04 | 0.41-0.53 |
| Bevacizumab | 6.44E-08 | 2.10E+05 | 1.78E+04 | 1.36E-02 | 5.14E-04 | 0.49-0.68 |
| Nivolumab | N/A | N/A | N/A | N/A | N/A | N/A |
| VP101(hG1WT) | 2.28E-08 | 2.41E+05 | 1.48E+04 | 5.50E-03 | 3.49E-04 | 1.40-1.52 |
| VP101(hG4WT) | 3.68E-08 | 2.13E+05 | 2.43E+04 | 7.83E-03 | 6.65E-04 | 0.41-0.57 |

N/A indicates that the antibody had no binding or an extremely weak binding signal to the antigen. Thus the results were not analyzed and no corresponding data was obtained.

The results showed that VP101(hG1WT) bound to FcγRIIa_H131 with an affinity constant of 2.28E-08M; VP101(hG4WT) bound to FcγRIIa_H131 with an affinity constant of 3.68E-08M; bevacizumab bound to FcγRIIa_H131 with an affinity constant of 6.44E-08M; VP101(hG1DM) bound to FcγRIIa_H131 with an affinity constant of 3.57E-08M; nivolumab had no binding or an extremely weak binding signal to FcγRIIa_H131, and thus the results were not analyzed and no corresponding data was obtained.

The results demonstrated that other than nivolumab that has no binding to FcγRIIa_HI31, the antibodies exhibit binding affinity to FcγRIIa_H131 in a descending order of VP101(hG1WT), VP101(hG1DM), VP101(hG4WT), bevacizumab.

Example 3: Affinity constant assay of FcγRIIa_R131 to VP101(hG1WT) and VP101(hG1DM)

The Fc receptor FcγRIIa_RI31 (also known as CD32a_R131), can bind to the Fc fragment of IgG antibodies and mediate ADCC effects.

The affinity constants of VP101(hG1WT) and VP101(hG1DM) to FcγRIIa_RI31 were measured using a Fortebio Octet system to evaluate the ADCC activity of the antibodies.

The method for determining the affinity constant of VP101(hG1WT) and VP101(hG1DM) by the Fortebio Octet system is briefly described as follows: The immobilization dilution buffer was a solution of 0.02% Tween-20 and 0.1% BSA in PBS, pH 7.4, and the analyte dilution buffer was a solution of 0.02% Tween-20, 0.02% casein and 0.1% BSA in PBS, pH 7.4. 5 μg/mL FcγRIIa_RI31 was immobilized on the NTA sensor for an immobilization time of about 60 nm. The sensor was equilibrated in a buffer of 0.02% Tween-20, 0.02% casein and 0.1% BSA in PBS pH 7.4 for 600 s for blocking, and the binding of the immobilized FcγRIIa_RI31 on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

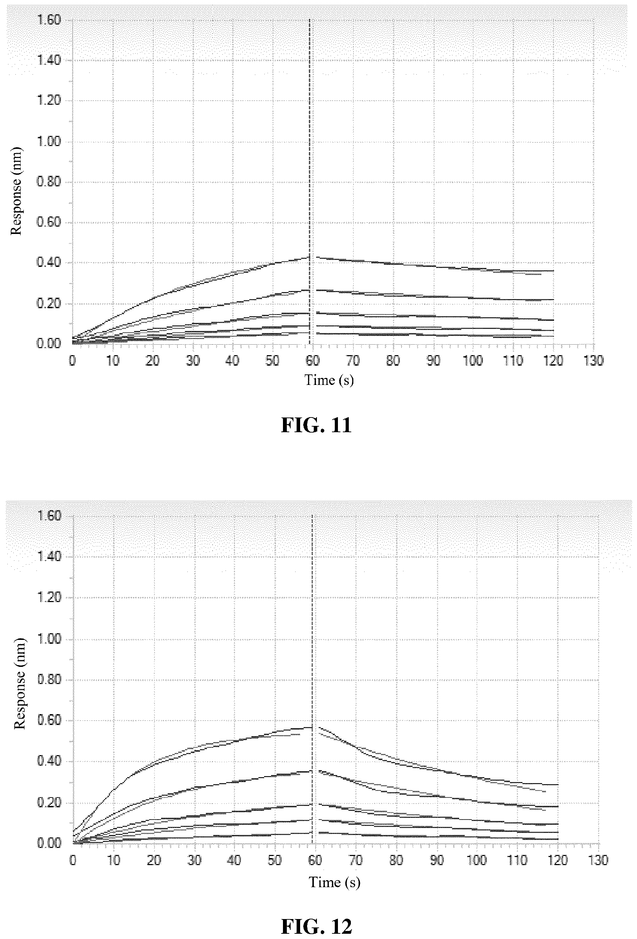

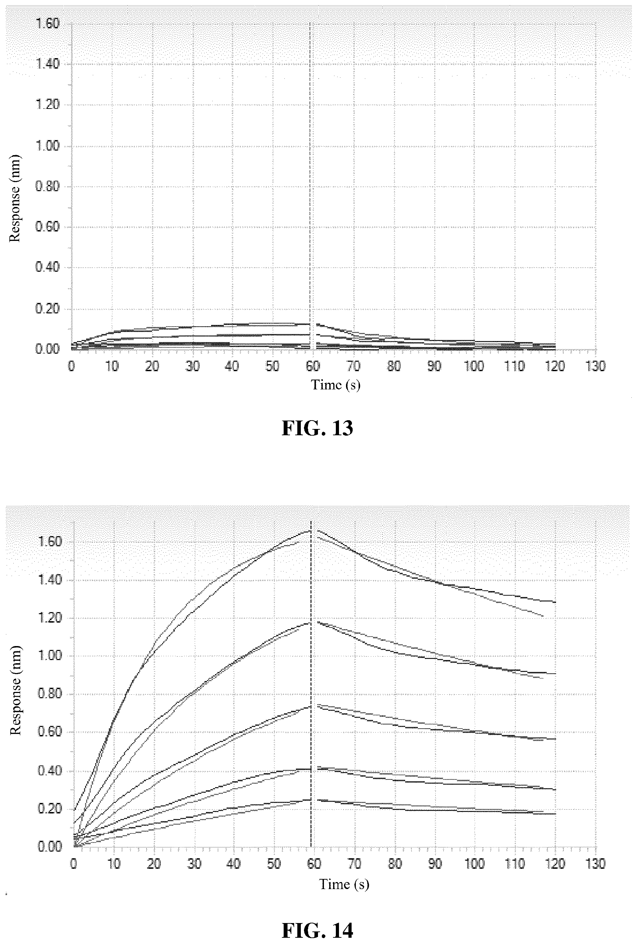

The results of affinity constant assay of FcγRIIa_RI31 to VP101(hG1WT), VP101(hG4WT) and VP101(hG1DM), and reference antibodies nivolumab and bevacizumab are shown in Table 3 and FIGs. 11 to 15.

Table 3: Kinetics for binding of VP101(hG1WT), VP101(hG1DM) and subtypes thereof to FcγRIIa R131

| Antibody | KD (M) | kon(1/Ms) | SE (kon) | kdis(1/s) | SE(kdis) | Rmax(nm) |
|---|---|---|---|---|---|---|
| VP101(hG1DM) | 3.35E-08 | 1.20E+05 | 9.72E+03 | 4.03E-03 | 3.08E-04 | 0.57-0.69 |
| Bevacizumab | 5.16E-08 | 2.59E+05 | 1.72E+04 | 1.33E-02 | 4.52E-04 | 0.42-0.69 |
| Nivolumab | 6.93E-08 | 4.78E+05 | 1.09E+05 | 3.31E-02 | 2.54E-03 | 0.08-0.16 |
| VP101(hG1WT) | 2.42E-08 | 2.14E+05 | 1.30E+04 | 5.17E-03 | 3.28E-04 | 1.75-1.92 |
| VP101(hG4WT) | 3.57E-08 | 1.99E+05 | 1.23E+04 | 7.09E-03 | 3.40E-04 | 0.81-1.05 |
| N/A indicates that the antibody had no binding or an extremely weak binding signal to the antigen. Thus the results were not analyzed and no corresponding data was obtained. | | | | | | |

The results showed that VP101(hG1WT) bound to FcγRIIa_RI31 with an affinity constant of 2.42E-08M; VP101(hG4WT) bound to FcγRIIa_RI31 with an affinity constant of 3.57E-08M; bevacizumab bound to FcγRIIa_RI31 with an affinity constant of 5.16E-08M; nivolumab bound to FcγRIIa_RI31 with an affinity constant of 6.93E-08M; VP101(hG1DM) bound to FcγRIIa_RI31 with an affinity constant of 3.35E-08M.

The results demonstrated that the antibodies exhibit binding affinity to FcγRIIa_RI31 in a descending order of: VP101(hG1WT), VP101(hG1DM), VP101(hG4WT), bevacizumab, nivolumab.

Example 4: Affinity constant assay of FcγRIIb to VP101(hG1WT) and VP101(hG1DM)

The Fc receptor FcyRIIb (also known as CD32b), can bind to the Fc fragment of IgG antibodies and is involved in regulating functions of immune cells.

The affinity constants of VP101(hG1WT) and VP101(hG1DM) to FcyRIIb were measured using a Fortebio Octet system to evaluate the binding capacity of VP101(hG1WT) and VP101(hG1DM) to Fc receptors.

The method for determining the affinity constant of VP101(hG1WT) and VP101(hG1DM) to FcyRIIb by the Fortebio Octet system is briefly described as follows: The immobilization dilution buffer was a solution of 0.02% Tween-20 and 0.1% BSA in PBS, pH 7.4, and the analyte dilution buffer was a solution of 0.02% Tween-20, 0.02% casein and 0.1% BSA in PBS, pH 7.4. 5 μg/mL hFCGR2B-his was immobilized on the NTA sensor for an immobilization time of about 60 nm. The sensor was equilibrated in a buffer of 0.02% Tween-20, 0.02% casein and 0.1% BSA in PBS pH 7.4 for 600 s for blocking, and the binding of the immobilized hFCGR2B-his on the sensor to the antibodies at concentrations of 12.5-200 nM (serial two-fold dilution) was determined for 60 s. The antibody was dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

Example 5: Affinity constant assay of FcγRrIIIa_V158 to VP101(hG1WT) and VP101(hG1DM)

The Fc receptor FcγRIIIa_V158 (also known as CD16a_V158), can bind to the Fc fragment of IgG antibodies and mediate ADCC effects.

The affinity constants of VP101(hG1WT) and VP101(hG1DM) to FcγRIIIa_V158 were measured using a Fortebio Octet system to evaluate the ADCC activity of the antibodies.

The method for determining the affinity constant of the antibodies to FcγRIIIa_V158 by the Fortebio Octet system is briefly described as follows: The sample dilution buffer was a solution of 0.02% Tween-20 and 0.1% BSA in PBS, pH 7.4. 5 μg/mL FcγRIIIa_V158 was immobilized on the HIS1K sensor for 120 s. The sensor was equilibrated in a buffer for 60 s, and the binding of the immobilized hFcGR3A(V158)-his on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

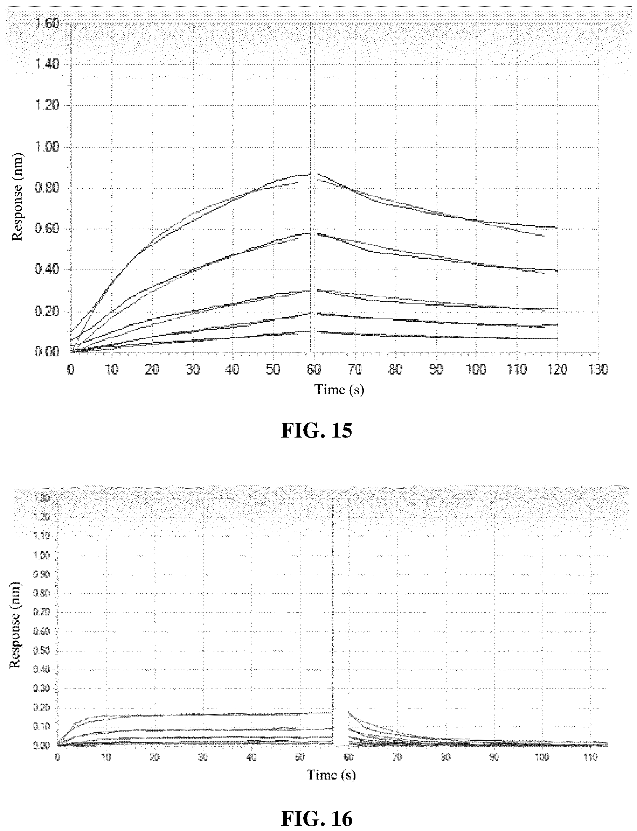

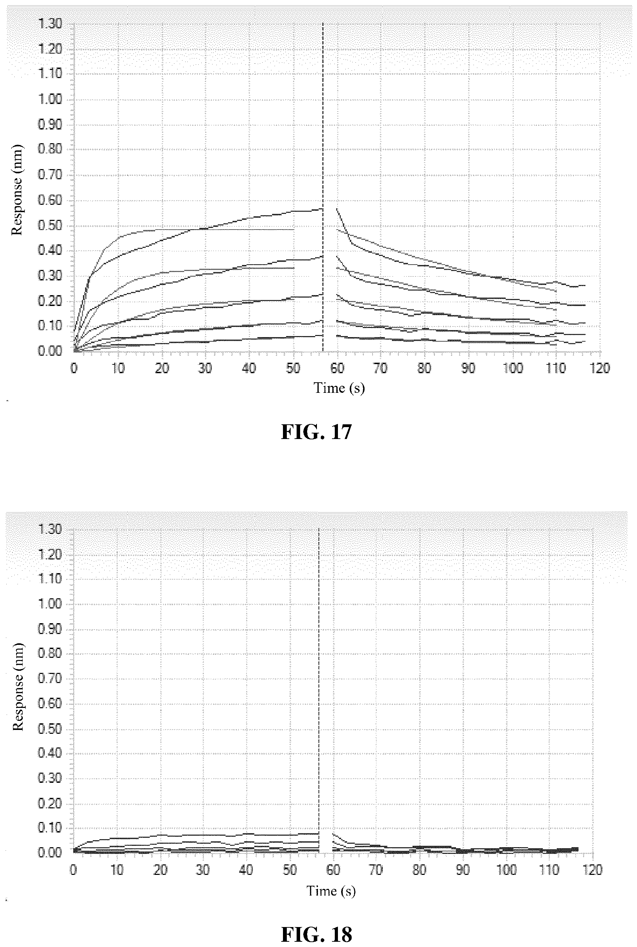

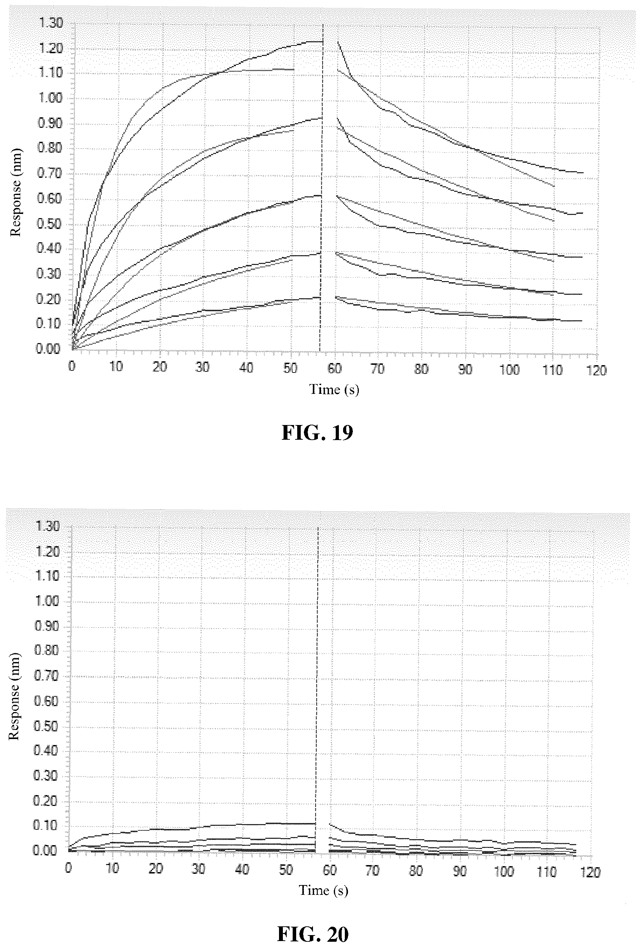

The results of affinity constant assay of FcγRIIIa_V158 to VP101(hG1WT), VP101(hG4WT) and VP101(hG1DM), and reference antibodies nivolumab and bevacizumab are shown in Table 4 and FIGs. 16 to 20.

Table 4: Kinetics for binding of VP101(hG1WT), VP101(hG1DM) and subtypes thereof to FcRIIIa V158

| Antibody | $K_D$ (M) | kon(1/Ms) | SE (kon) | kdis(1/s) | SE(kdis) | Rmax(nm) |
|---|---|---|---|---|---|---|
| VP101(hG1DM) | 1.34E-07 | 6.05E+05 | 2.36E+05 | 8.11E-02 | 7.42E-03 | 0.07-0.21 |
| Bevacizumab | 2.76E-08 | 5.06E+05 | 1.14E+05 | 1.39E-02 | 1.41E-03 | 0.13-0.51 |
| Nivolumab | N/A | N/A | N/A | N/A | N/A | N/A |
| VP101(hG1WT) | 4.35E-08 | 2.39E+05 | 3.14E+04 | 1.04E-02 | 8.73E-04 | 0.80-1.22 |
| VP101(hG4WT) | N/A | N/A | N/A | N/A | N/A | N/A |
| N/A indicates that the antibody had no binding or an extremely weak binding signal to the antigen. Thus the results were not analyzed and no corresponding data was obtained. | | | | | | |

The results showed that VP101(hG1WT) bound to FcγRIIIa_V158 with an affinity constant of 4.35E-08M; VP101(hG1DM) bound to FcγRIIIa_V158 with an affinity constant of 1.34E-07M; bevacizumab bound to FcγRIIIa_V158 with an affinity constant of 2.76E-08M; nivolumab and VP101(hG4WT) had no binding or an extremely weak binding signal to FcγRIIIa_V158, and thus the results were not analyzed and no corresponding data was obtained.

The results demonstrated that other than nivolumab and VP101(hG4WT) that have no binding to FcγRIIIa_V158, the antibodies exhibit binding affinity to FcγRIIIa_V158 in a descending order of: bevacizumab, VP101(hG1WT), VP101(hG1DM).

Example 6: Affinity constant assay of FcγRIIIa_F158 to VP101(hG1WT) and VP101(hG1DM)

The Fc receptor FcγRIIIa_F158 (also known as CD16a_F158), can bind to the Fc fragment of IgG antibodies and mediate ADCC effects.

The affinity constants of VP101(hG1WT) and VP101(hG1DM) to FcγRIIIa_F158 were measured using a Fortebio Octet system to evaluate the ADCC activity of the antibodies.

The method for determining the affinity constant of VP101(hG1WT) and VP101(hG1DM) to FcγRIIIa_F158 by the Fortebio Octet system is briefly described as follows: The sample dilution buffer was a solution of 0.02% Tween-20 and 0.1% BSA in PBS, pH 7.4. 5 μg/mL FcγRIIIa_F158 was immobilized on the HIS1K sensor for 120 s. The sensor was equilibrated in a buffer for 60 s, and the binding of the immobilized hFcGR3A(F158)-his on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

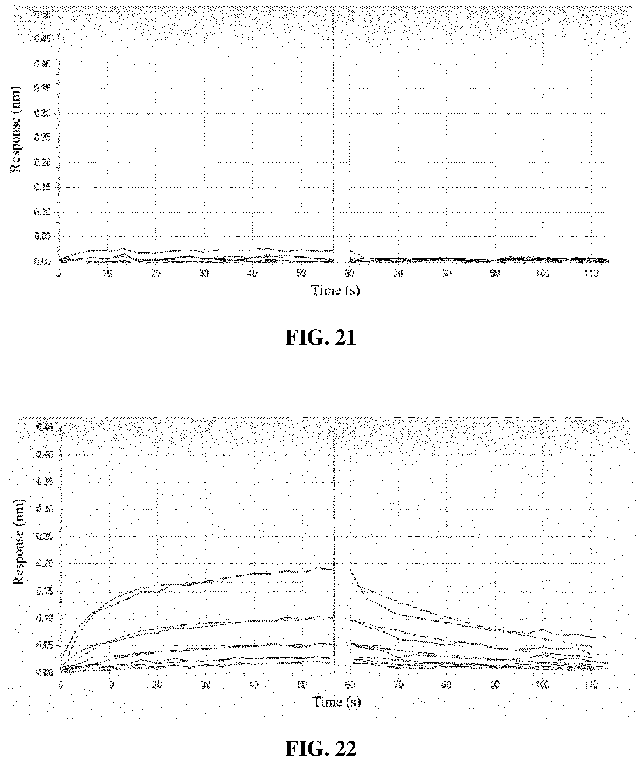

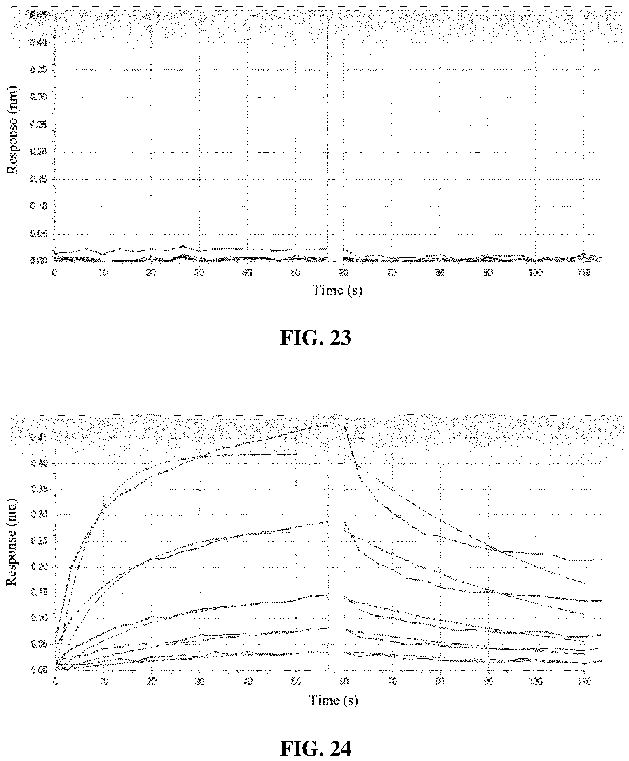

The results of affinity constant assay of FcγRIIIa_F158 to VP101(hG1WT), VP101(hG4WT) and VP101(hG1DM), and reference antibodies nivolumab and bevacizumab are shown in Table 5 and FIGs. 21 to 25.

Table 5: Kinetics for binding of VP101(hG1WT), VP101(hG1DM) and subtypes thereof to FcγRIIIa F158

| Antibody | $K_D$ (M) | kon(1/Ms) | SE (kon) | kdis(1/s) | SE(kdis) | Rmax(nm) |
|---|---|---|---|---|---|---|
| VP101(hG1DM) | N/A | N/A | N/A | N/A | N/A | N/A |
| Bevacizumab | 9.32E-08 | 2.64E+05 | 7.16E+04 | 2.46E-02 | 2.09E-03 | 0.08-0.20 |

(continued)

| Antibody | $K_D$ (M) | kon(1/Ms) | SE (kon) | kdis(1/s) | SE(kdis) | Rmax(nm) |
|---|---|---|---|---|---|---|
| Nivolumab | N/A | N/A | N/A | N/A | N/A | N/A |
| VP101(hG1WT) | 7.41E-08 | 2.47E+05 | 5.20E+04 | 1.83E-02 | 1.55E-03 | 0.15-0.48 |
| VP101(hG4WT) | N/A | N/A | N/A | N/A | N/A | N/A |
| N/A indicates that the antibody had no binding or an extremely weak binding signal to the antigen. Thus the results were not analyzed and no corresponding data was obtained. | | | | | | |

The results showed that VP101(hG1WT) bound to FcγRIIIa_F158 with an affinity constant of 7.41E-08M; bevacizumab bound to FcyRIIIa_F158 with an affinity constant of 9.32E-08M; nivolumab, VP101(hG4WT) and VP101(hG1DM) had no binding or an extremely weak binding signal to FcγRIIIa_F158, and thus the results were not analyzed and no corresponding data was obtained.

The results demonstrated that other than nivolumab, VP101(hG4WT) and VP101(hG1DM) that have no binding to FcγRIIIa_F158, the antibodies exhibit binding affinity to FcγRIIIa_F158 in a descending order of: VP101(hG1WT), bevacizumab.

Example 7: Affinity constant assay of C1q to VP101(hG1WT) and VP101(hG1DM)

Serum complement C1q can bind to the Fc fragment of IgG antibodies and mediate CDC effects. The binding capacity of a therapeutic monoclonal antibody to C1q will influence the safety and efficacy of the antibody.

The affinity constants of VP101(hG1WT) and VP101(hG1DM) to C1q were measured using a Fortebio Octet system to evaluate the CDC activity of the antibodies.

The method for determining the affinity constant of the antibodies to C1q by the Fortebio Octet system is briefly described as follows: the sample dilution buffer was a solution of PBS, 0.02% Tween-20 and 0.1% BSA, pH 7.4. 50 μg/mL antibody was immobilized on the FAB2G sensor at an immobilization height of about 2.0 nm. The sensor was equilibrated in a buffer for 60 s for blocking, and the binding of the immobilized antibody on the sensor to the C1q at concentrations of 0.625-10 nM (two-fold dilution) was determined for 60 s. The antigen and antibody were dissociated in the buffer for 60 s. The sensor was refreshed 4 times in 10 mM glycine pH 1.7, each for 5 s. The shaking speed of the sample plate was 1000 rpm, the temperature was 30 °C and the frequency was 0.6 Hz. The data were analyzed by 1:1 model fitting to obtain affinity constants. The data acquisition software was Fortebio Data Acquisition 7.0, and the data analysis software was Fortebio Data Analysis 7.0.

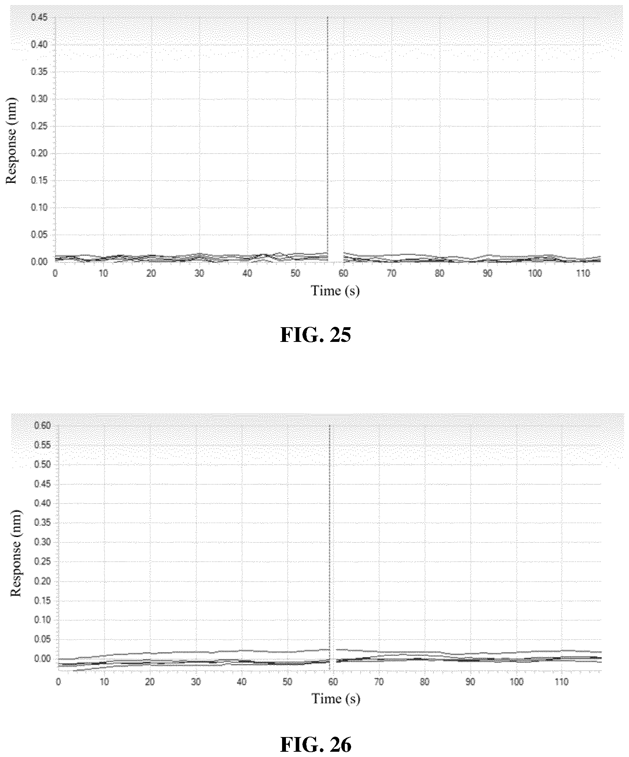

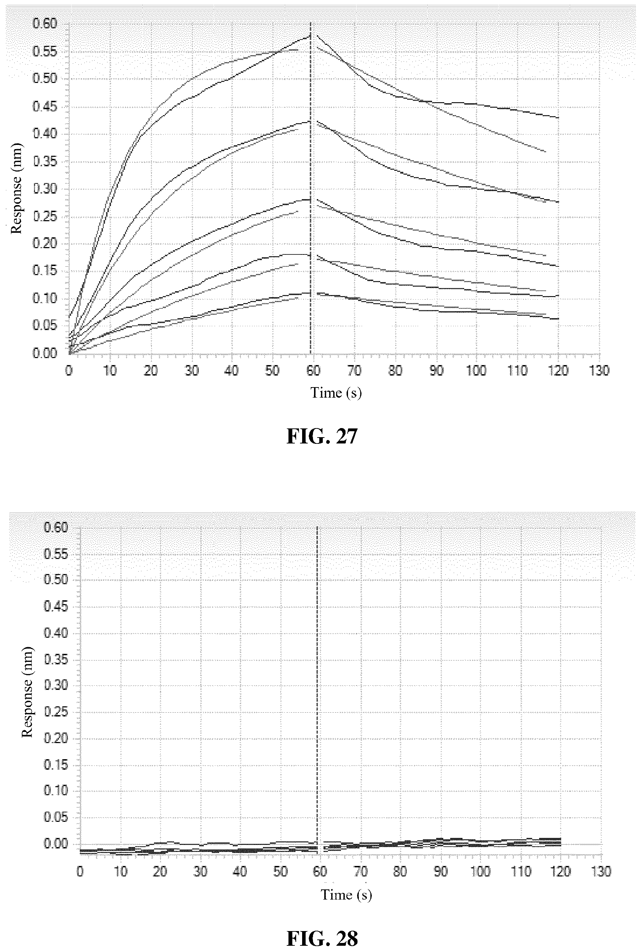

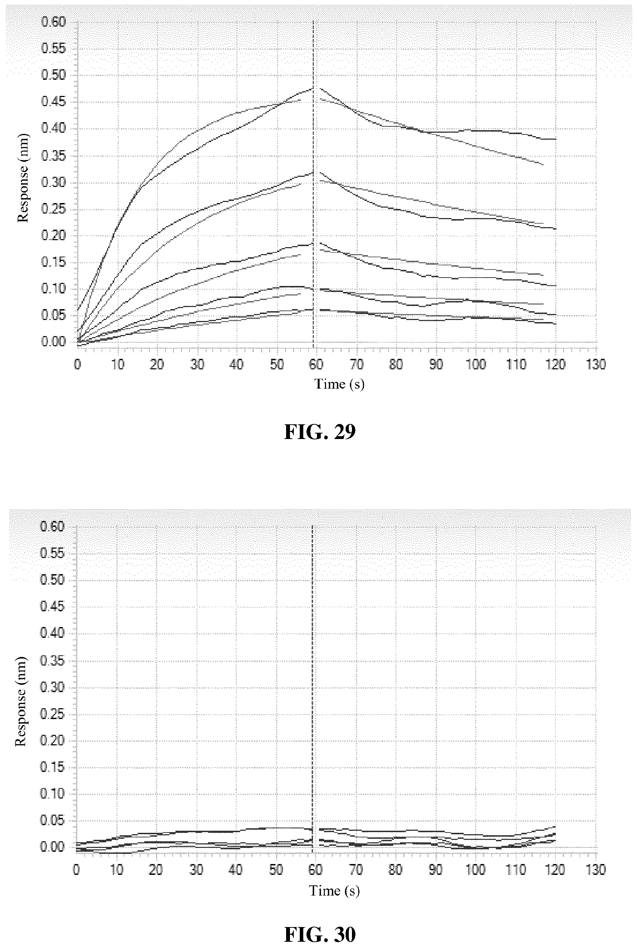

The results of affinity constant assay of C1q to VP101(hG1WT), VP101(hG4WT) and VP101(hG1DM), and reference antibodies nivolumab and bevacizumab are shown in Table 6 and FIGs. 26 to 30.

Table 6: Kinetics for binding of VP101(hG1WT), VP101(hG1DM) and subtypes thereof to C1q

| Antibody | $K_D$ (M) | kon(1/Ms) | SE (kon) | kdis(1/s) | SE(kdis) | Rmax(nm) |
|---|---|---|---|---|---|---|
| VP101(hG1DM) | N/A | N/A | N/A | N/A | N/A | N/A |
| Bevacizumab | 1.14E-09 | 6.52E+06 | 5.64E+05 | 7.40E-03 | 5.58E-04 | 0.51-0.63 |
| Nivolumab | N/A | N/A | N/A | N/A | N/A | N/A |
| VP101(hG1WT) | 9.76E-10 | 5.73E+06 | 5.49E+05 | 5.59E-03 | 6.12E-04 | 0.32-0.51 |
| VP101(hG4WT) | N/A | N/A | N/A | N/A | N/A | N/A |
| N/A indicates that the antibody had no binding or an extremely weak binding signal to the antigen. Thus the results were not analyzed and no corresponding data was obtained. | | | | | | |

The results showed that VP101(hG1WT) bound to C1q with an affinity constant of 9.76E-10M; bevacizumab bound to C1q with an affinity constant of 1.14E-09M; nivolumab, VP101(hG4WT) and VP101(hG1DM) had no binding or an extremely weak binding signal to C1q, and thus the results were not analyzed and no corresponding data was obtained.

The results demonstrated that other than nivolumab, VP101(hG4WT) and VP101(hG1DM) that have no binding to FcγRIIIa_F158, the antibodies exhibit binding affinity to C1q, and VP101(hG1WT) and bevacizumab have similar affinities.

Example 8: ADCC activity assay of VP101(hG1WT) and VP101(hG1DM) in CHO-K1-PD1 cells expressing PD-1 antigen

To test the ADCC effect of the antibodies VP101(hG1WT) and VP101(hG1DM) at the cytological level, the inventors constructed a CHO-K1-PD1 cell expressing PD-1 and a mixed lymphocyte reaction system of normal human PBMCs and target cells.

The method for the ADCC activity assay of VP101(hG1WT) and VP101(hG1DM) on CHO-K1-PD1 cells expressing PD-1 antigen is as follows:

Firstly a human PD-1 overexpression vector pCDH-CMV-PD1FL-Puro was constructed (pCDH-CMV-Puro was purchased from Youbio), and the expression vector was packaged by virus before the CHO-K1 cells were infected. Puromycin ($2 \mu g/mL$) was added for screening, and a CHO-K1-PD1 stable cell line with drug resistance stably expressing membrane PD-1 protein was obtained. Normal human PBMCs were obtained according to the manual of Ficoll peripheral blood mononuclear cell separating medium. The isolated PBMCs were resuspended in 1640 complete culture medium. After trypan blue staining, the cells were counted, analyzed for viability, and incubated overnight in an incubator at 37 °C, 5% $CO_2$ and saturated humidity. The next day, CHO-K1-PD1 cells and PBMCs were separately collected and centrifuged to remove the supernatant. The cell pellet was resuspended in RPMI-1640 (containing 1% BSA) (hereinafter referred to as analytic medium), centrifuged and washed 2 times. The cells were counted, analyzed for viability, and adjusted a proper cell density range using the analytic medium. According to the study design, CHO-K1-PD1 cell suspension was added to a 96-well plate (30000/well), and 50 $\mu L$ of the antibody was added. The mixture was uniformly mixed, and pre-incubating for 1 h at room temperature. After the pre-incubation, PBMCs were added at a concentration of 900,000 cells/50 $\mu L$/well. The mixture was well mixed and incubated for 4 h in an incubator at 37 °C and 5% $CO_2$. After 4 h, the 96-well plate was taken out and centrifuged at $250\times$ g for 5 min. 100 $\mu L$ of supernatant was carefully transferred to a new 96-well flat-bottom microplate (do not pipette the cell precipitate). 100 $\mu L$ of freshly prepared reaction solution was added to each well according to the Cytotoxicity Detection Kit instruction. The cells were incubated for 30 min at room temperature in the dark. OD values were measured at 490 nm and 650 nm, and calculated by: OD = $OD_{490nm}$ - $OD_{650nm}$. The ADCC activity was calculated for each group by: ADCC (%) = (treatment group - negative control group)/(target cell LDH maximum release - target cell LDH spontaneous release) $\times$ 100%.

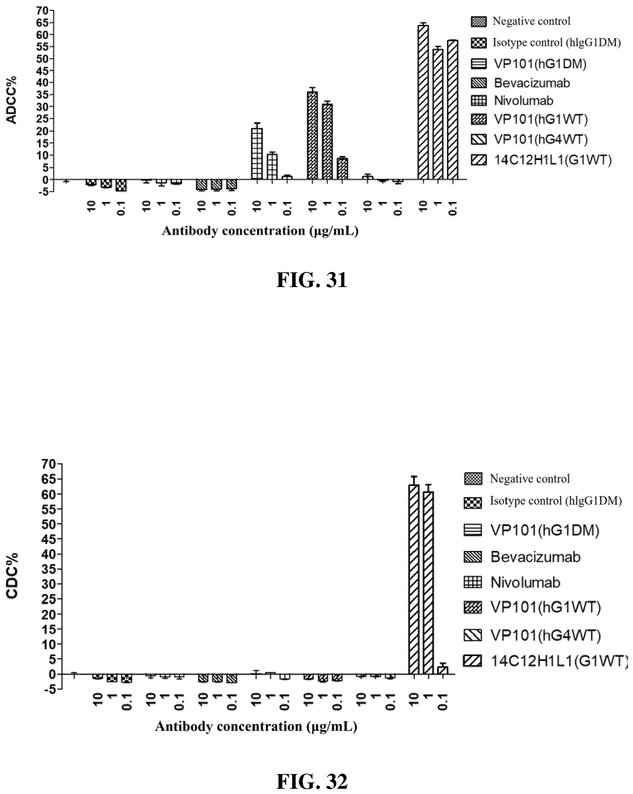

The results of ADCC activity assay of VP101(hG1WT) and VP101(hG1DM) in CHO-K1-PD1 cells expressing PD-1 antigen are expressed as ADCC%, and are shown in FIG. 31.

The results showed that the positive control 14C12H1L1(G1WT) has significant ADCC activity in the mixed lymphocyte reaction of PBMCs and CHO-K1-PD1 cells, indicating that the ADCC system is normal. Compared to isotype control antibody hIgGIDM, VP101(hG1WT) showed significant ADCC activity with dose-dependence, while VP101(G1DM) did not exhibit ADCC activity. The results suggested that VP101(hG1DM) produced by mutation based on VP101(hG1WT) has no ADCC activity at the cytological level, and ADCC effect is eliminated.

Example 9: CDC activity assay of VP101(hG1WT) and VP101(hG1DM) in CHO-K1-PD1 cells expressing PD-1 antigen

To test the CDC effect of the antibodies VP101(hG1WT) and VP101(hG1DM) at the cytological level, the inventors constructed a CHO-K1-PD1 cell expressing PD-1 (see Example 8 for the construction method) and a mixed lymphocyte reaction system of target cells and normal human complement serum.

The method for the CDC activity assay of VP101(hG1WT) and VP101(hG1DM) on CHO-K1-PD1 cells expressing PD-1 antigen is as follows:

On the day of detection, CHO-K1-PD1 cells were collected by digestion using trypsin, centrifuged at $170\times$ g for 5 min, then resuspended in RPMI-1640 (containing 1% BSA) (hereinafter referred to as analytical medium), centrifuged and washed 2 times. The cells were counted, analyzed for viability, and adjusted a proper cell density range using the analytic medium. According to the study design, CHO-K1-PD1 cell suspension was added to a 96-well plate (30000/well), and 50 $\mu L$ of the antibody was added. The mixture was uniformly mixed, and pre-incubating for 10 min at room temperature. After the pre-incubation, normal human complement serum (final concentration: 2%) was added at 50 $\mu L$/well. The mixture was well mixed and incubated for 4 h in an incubator at 37 °C and 5% $CO_2$. After 4 h, the mixture was centrifuged at $250\times$ g for 5 min. 100 $\mu L$ of supernatant was carefully pipetted and transferred to a new flat-bottom 96-well plate (do not pipette the cell precipitate). 100 $\mu L$ of freshly prepared reaction solution was added to each well according to the Cytotoxicity Detection Kit instruction. The cells were incubated for 30 min at room temperature in the dark. OD values were measured at 490 nm and 650 nm, and calculated by: OD = $OD_{490nm}$ - $OD_{650nm}$. The CDC activity was calculated for each group by: CDC (%) = (treatment group - negative control group)/(target cell LDH maximum release - target cell LDH spontaneous release) $\times$ 100%.

The results of CDC activity assay of VP101(hG1WT) and VP101(hG1DM) in CHO-K1-PD1 cells expressing PD-1 antigen are expressed as CDC%, and are shown in FIG. 32.

The results showed that compared with isotype control antibody hIgGIDM, the positive control antibody 14C12H1L1(G1WT) has significant difference in CDC% in the mixed lymphocyte reaction of normal human complement serum and CHO-K1-PD1 cells, indicating that the CDC system is normal. At equivalent dose levels, VP101(hG1WT) and VP101(hG1DM) have no significant difference in CDC% compared with the isotype control.

Example 10: Pharmacodynamic activity of VP101(hG1DM) measured by mixed lymphocyte reaction (MLR) of peripheral blood mononuclear cells and Raji-PDL1 cells

Firstly a human PD-L1 overexpression vector plenti6.3-PD-L1-BSD was constructed (plenti6.3-BSD was purchased from Invitrogen), and the expression vector was packaged by virus before the Raji cells were infected. BSD (10 $\mu$g/mL) was added for screening, and a Raji-PDL1 stable cell line stably expressing membrane PD-L1 protein was obtained. Normal human PBMCs were isolated according to the Ficoll peripheral blood mononuclear cell isolation instruction. The isolated PBMCs were resuspended in 1640 complete medium, counted and frozen. The PBMCs were thawed and co-incubated with *Staphylococcus aureus* enterotoxin B (SEB) for two days. After two days, the Raji-PDL1 cells in logarithmic phase were collected, and mitomycin C (Sigma, working concentration: 25 $\mu$g/mL) was added. The Raji-PDL1 cells were incubated in an incubator for 60 min, centrifuged and washed. PBMCs after two days of SEB stimulation were collected and washed. The mixed lymphocyte reaction was conducted in a cell number ratio of 1:1 with or without antibodies. 3 days later, the supernatant was collected by centrifugation, and IL-2 and IFN-$\gamma$ concentrations in the supernatant were detected by ELISA.

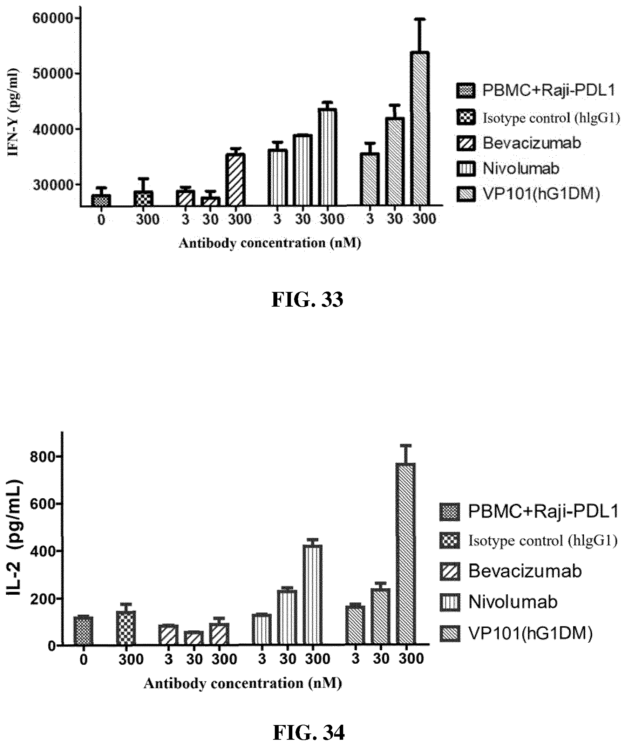

The results of IFN-$\gamma$ secretion are shown in FIG. 33. The results showed that VP101(hG1DM) can effectively promote secretion of IFN-$\gamma$ with significantly superior activity to nivolumab.

The results of IL-2 secretion are shown in FIG. 34. The results showed that VP101(hG1DM) can effectively promote secretion of IL-2 in a dose-dependent manner with significantly superior activity to nivolumab.

Example 11: Absence of antibody dependent cellular phagoxytosis activity of VP101(hG1DM) to PD-1-positive cells

Antibody dependent cellular phagoxytosis (ADCP) refers to the binding of the Fc fragment of an antibody bound to a cell surface antigen to the Fc receptor of a phagocytically active cell, such as a macrophage, which in turn mediates phagocytosis of the antibody-bound cell. For immune checkpoint inhibitor antibodies, such as PD-1 antibodies, the presence of ADCP activity will cause damage to immune cells expressing PD-1 that exert an anti-tumor killing effect, thereby affecting their anti-tumor activity.

In this experiment, murine macrophages were used as the effector cells and CHO-K1-PD1 cells overexpressing PD-1 (see Example 8 for the construction method) were used as the target cells to test the mediated ADCP effect. The ADCP activity of VP101(hG1DM) on cells expressing PD-1 was determined by flow cytometry, and the results showed that the antibody has no ADCP activity, while the approved PD-1 antibody nivolumab with the same target has significant ADCP activity. The method is as follows:

The femoral bone marrow of C57 mice (purchased from Guangdong Medical Laboratory Animal Center) was first aseptically collected and lysed by erythrocyte lysis buffer on ice for 5 min. The lysis was terminated with DMEM complete medium (containing 10% FBS), and the lysate was centrifuged at 1000 rpm and washed twice. The cell pellet was resuspended in 10 mL of DMEM complete medium and M-CSF was added at a working concentration of 100 ng/mL. The cells were cultured for 7 days at 37 °C and 5% $CO_2$ in a cell culture chamber for induction. Half of the medium was exchanged and M-CSF was added on Days 3 and 5. The induction of cells was completed on day 7. The cells were digested with 0.25% trypsin. Macrophages were collected, and centrifuged at 750$\times$ g for 5 min. The supernatant was discarded and the cells were suspended in DMEM complete medium (containing 10% FBS) and counted. The cell was adjusted to a proper density and filled into a 96-well plate for further use.

CHO-K1-PD1 cells were collected by conventional methods, centrifuged at 170$\times$ g for 5 min, resuspended, counted, analyzed for viability and washed once with PBS. Carboxyfluorescein diacetate succinimidyl ester (CFSE) was diluted to 2.5 $\mu$M with PBS to resuspend the cells (staining density: 10 million cells/mL). A proper amount of the cells were incubated in a cell incubator for 20 min. 6 mL of DMEM complete medium (containing 10% of FBS) to stop the staining. The cells were centrifuged at 170$\times$ g for 5 min, and the supernatant was discarded. 1 mL of DMEM complete medium was added and the cells were incubated in an incubator for 10 min. Antibodies were diluted with DMEM complete medium to 20 $\mu$g/mL, 2 $\mu$g/mL and 0.2 $\mu$g/mL (with working concentrations of 10 $\mu$g/mL, 1 $\mu$g/mL and 0.1 $\mu$g/mL) and isotype control antibodies hIgGIDM and hIgG4 were designed. Fresh induced mature macrophages were collected and centrifuged at 750$\times$ g for 5 min, and the supernatant was discarded. The cells were counted, transferred to a 96-well plate and centrifuged at 1000$\times$ g for 5 min, and the supernatant was discarded. The CHO-K1-PD1-CFSE cell density was adjusted. According to the design, the diluted antibodies and the target cells were added into wells of the 96-well plate containing macrophages in a ratio of 50 $\mu$L:50 $\mu$L, resuspended, well mixed, and incubated in an incubator at 37 °C for 2 h. 150 $\mu$L of 1% PBSA was added into each well at room temperature. The mixture was centrifuged for 5 min at 1000$\times$ g, and the supernatant was discarded. The cells were washed once with 200 $\mu$L of PBSA. APC anti-mouse/human CD11b antibody (500-fold diluted with PBSA) was added to the corresponding samples at 100 $\mu$L/sample, and the

mixture was well mixed and incubated on ice for 40 min. 150 $\mu$L of 1% PBSA was added into each well, centrifuged at 1000× g for 5 min, and the supernatant was discarded. Each well was washed once with 200 $\mu$L of PBSA. 200 $\mu$L of 1% PBSA was added to each well for resuspension and loaded on a Beckman flow cytometer.

Macrophages in the system were APC[+] positive, and macrophages involved in phagocytosis were APC and CFSE dual positive. The phagocytosis rate was determined as the ratio of the number of dual positive cells to the number of APC positive cells, and the antibody-mediated ADCP activity was evaluated. The ADCP activity of each group, represented by P%, was calculated according to the following formula:

$$\mathbf{P\%} = \frac{\textbf{Number of macrophages involved in phagocytosis}}{\textbf{Total number of macrophages}} \times \mathbf{100\%}$$

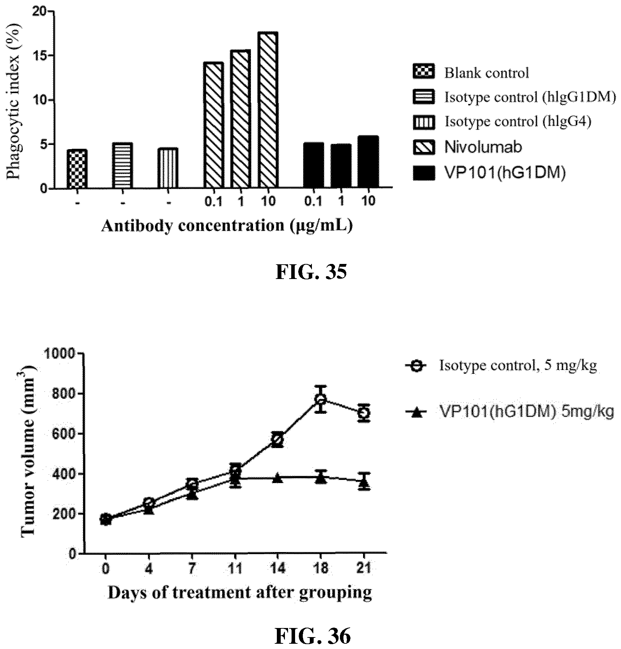

The results are shown in FIG. 35.

The results showed that nivolumab has significant ADCP effect in the macrophage + CHO-K1-PD1 system; the phagocytic rate of VP101(hG1DM) was comparable to the isotype control antibody, indicating that VP101(hG1DM) has no ADCP effect. The results suggested that VP101(hG1DM) is likely to have superior anti-tumor efficacy.

Example 12: Animal study of VP101(hG1DM) monotherapy for treating breast cancer

This study investigated the anti-tumor activity of VP101(hG1DM) against *in vivo* cell proliferation of breast cancer xenograft. The protocol is shown in Table 7 below.

Femal NCG mice with serious immune deficiency aged 5 weeks purchased from GemPharmatech were used in the study. The isotype control antibody hIgG4 was constructed by Akeso Biopharma, Inc., with the method described above. PBMCs were isolated and activated by Akeso Biopharma, Inc., with the method described above.

The collected breast cancer MDA-MB-231 cells were inoculated at the breast pads in 12 mice at 2 million cells/50 $\mu$L/mouse. 30 days after inoculation, the mice were divided by tumor volume into 2 groups, a negative control group and a VP101(hG1DM) group. PBMCs were administered at a dose of 1 million cells/50 $\mu$L/mouse subcutaneously on the same day, and the day of PBMC administration was recorded as day 0. The treatment was given on day 0, day 7, day 14 and day 21. Tumor volume was measured by vernier caliper. Tumor dimensions were measured twice weekly after grouping using vernier caliper, and the tumor volume was calculated according to the formula TV = 0.5 × ab$^2$, wherein a is the longest diameter of the tumor, b is the shortest diameter of the tumor, and TV is the volume of the tumor.

Table 7: Protocol of study on VP101(hG1DM) monotherapy effectively inhibiting breast cancer cell proliferation

| Group | Number of animals | Protocol for tumor xenografting | Dosing regimen |
|---|---|---|---|
| Isotype control Antibody | 6 | The breast cancer MDA-MB-231 cells, 2 million cells/50 $\mu$L/mouse, inoculated at breast pads | hIgG4, 5 mg/kg, intravenously administered, once a week for 4 doses |
| VP101 (hG1DM) | 6 | PBMCs, 1 million cells/50 $\mu$L/mouse, inoculated subcutaneously | VP101(hG1DM), 5 mg/kg, intravenously administered, once a week for 4 doses |

The results are shown in FIG. 36. The results showed that VP101(hG1DM) can effectively inhibit the volume increase of the breast cancer MDA-MB-231 cells, exhibiting good anti-tumor efficacy, especially anti-breast cancer efficacy, compared with the isotype control antibody.

Example 13: Study of PARPi and VP101(hG1DM) combination therapy for inhibiting ovarian cancer cells

SNU-251 ovarian cancer cells (Otwo Biotech, Cat. No.: HTX2700C) in the logarithmic growth phase were prepared into a single-cell suspension by digestion with trypsin. The cells were counted, adjusted to a proper cell density, seeded into 6-well plates at 2 × 10$^5$ per well, and incubated in RPMI 1640 medium (Gibco, Cat. No.: 22400-089) at 37 °C for 24 h.

After the cells grew to 60%-70%, the cells were divided for administration into groups, including control groups and treatment groups, and incubated for 24 h in an incubator at 37 °C and 5% CO$_2$.

The Transwell chambers were placed in a 24-well plate, 100 μL of RPMI 1640 medium was added to the upper chamber, and the mixture was equilibrated for 1 h in an incubator. The cells in the 6-well plates were digested using trypsin, and prepared into a single-cell suspension using a serum-free culture medium. The cells were counted, and the cell density was adjusted to $4 \times 10^4$ cells/100 μL. The cells were seeded into the upper chamber, and 600 μL of RPMI 1640 culture medium containing 10% fetal bovine serum was added into the lower chamber. The plates were incubated in an incubator at 37 °C and 5% $CO_2$ for 24 h.

After the incubation, the Transwell chambers were taken out, and the culture medium was removed. The lower chamber cells were immobilized with PBS for 2 min, and immobilized with 4% paraformaldehyde for 20 min. The chambers were washed with PBS twice.

Cells in the lower chamber were stained with 500 μL of 0.1% crystal violet dye for 15 min. The residual dye was washed off with PBS. Cells in the upper chamber were carefully wiped out with a cotton swab. The chambers were placed on a slide and 3 fields of view were selected under a microscope to observe the cells moved to the lower chamber and photographed. The number of cells was calculated using the ImgeJ software and the migration of the cells was calculated according to the formula.

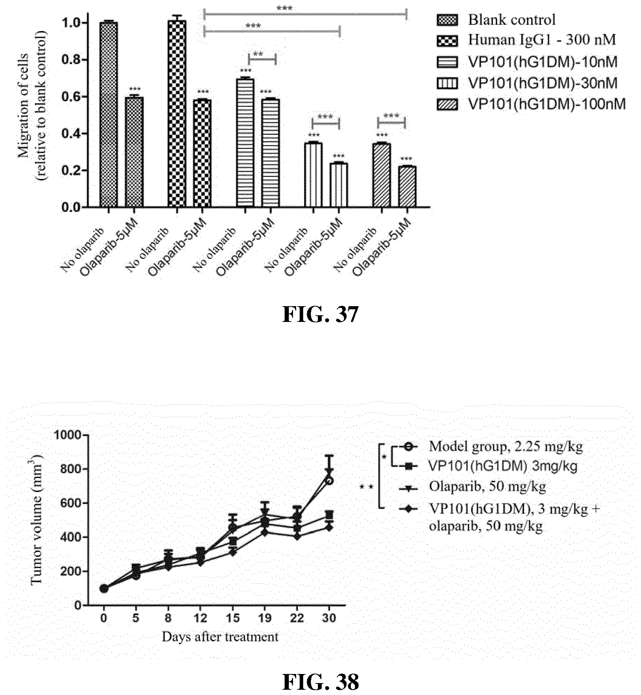

The results are shown in FIG. 37. The results showed that compared with the olaparib monotherapy or the VP101(hG1DM) monotherapy at the same concentration, the PARP inhibitor olaparib and VP101(hG1DM) combination therapy has significantly enhanced efficacy in inhibiting the migration of ovarian cancer cells.

Example 14: Animal study of PARPi and VP101(hG1DM) combination therapy for treating breast cancer

To test the *in vivo* anti-tumor activity of the PARP inhibitor olaparib and anti-PD-1/anti-VEGFA bifunctional antibody VP101(hG1DM) combination therapy, MDA-MB-231 cells (human breast cancer cells, purchased from ATCC) were subcutaneously grafted into NCG mice aged 5-7 weeks (purchased from GemPharmatech). 23 days after grafting, the mice were randomly divided into 4 groups of 6 animals by tumor volume. The day of grouping was defined as D0, and olaparib dosing was started on day D0. On D2, 4 million activated PBMCs were intraperitoneally injected and VP101(hG1DM) treatment was started. The regimen of the combination therapy group is as follows: the drugs were formulated separately and administered sequentially (no certain order or time interval was required, and one treatment should be given after the completed administration of the other). The modeling and specific regimen are shown in Table 8. After the administration, the length and width of tumors in each group were measured, and the tumor volume was calculated.

Table 8: Dose regimen of the PARP inhibitor olaparib and anti-PD-1/anti-VEGFA bifunctional antibody combination therapy for treating MDA-MB-231 xenograft tumor in NCG mouse model

| Serial number | Group | Number of animals | Modeling | Regimen |
|---|---|---|---|---|
| 1 | Model group | 6 | Each mouse was subcutaneouslygrafted with 8 million MDA-MB-231 cells, and intraperitoneally injected with 4 million CD3-activated PBMCs 25 days after grafting | hIgGIDM, 2.25 mg/kg, administered at the tail vein, once weekly for 4doses 10% hydroxypropyl-β-cyclodextrin, intragastrically administered every day for 23 days |
| 2 | VP101 (hG1DM) | 6 | | VP101(hG1DM), 3 mg/kg, administered at the tail vein, once weekly for 4 doses |
| 3 | Olaparib | 6 | | Olaparib, 50 mg/kg, intragastrically administered every day for 23 days |
| 4 | VP101 (hG1DM) + olaparib | 6 | | VP101(hG1DM), 3 mg/kg, administered at the tail vein, once weekly for 4 doses Olaparib, 50 mg/kg, intragastrically administered every day for 23 days |

The results are shown in FIG. 38. The results showed that the VP101(hG1DM) and PARP inhibitor olaparib combination therapy exhibits a synergistic anti-tumor effect in the mouse breast cancer model compared with the olaparib monotherapy group and the VP101(hG1DM) monotherapy group, and the combination has superior tumor inhibition to the monotherapy groups.

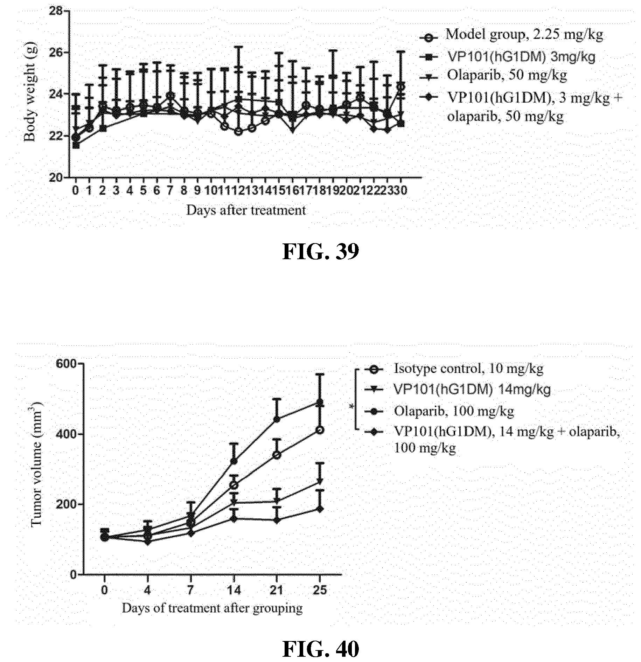

In addition, as shown in FIG. 39, both VP101(hG1DM) and PARP inhibitor olaparib were well tolerated by the tumor-bearing mice, either alone or in combination, and no effect on the body weight of the tumor-bearing mice was found in the groups.

Example 15: Animal study of PARPi and VP101(hG1DM) combination therapy for treating ovarian cancer

To test the *in vivo* anti-tumor activity of the PARP inhibitor olaparib and anti-PD-1/anti-VEGFA bifunctional antibody VP101(hG1DM) combination therapy, SK-OV-3 human ovarian cancer cells (purchased from ATCC) were subcutaneously grafted into NCG mice aged 5-7 weeks (purchased from GemPharmatech). 39 days after grafting, the mice were intraperitoneally injected with 3 million activated PBMCs and randomly divided into 4 groups of 6 animals by tumor volume. The day of grouping was defined as D0, and dosing was started on the day of grouping D0. The regimen of the combination therapy group is as follows: the drugs were formulated separately and administered sequentially (no certain order or time interval was required, and one treatment should be given after the completed administration of the other). The modeling and specific regimen are shown in Table 9. After the administration, the length and width of tumors in each group were measured, and the tumor volume was calculated.

Table 9: Dose regimen of the PARP inhibitor olaparib and anti-PD-1/anti-VEGFA bifunctional antibody combination therapy for treating SK-OV-3 xenograft tumor in NCG mouse model

| Serial number | Group | Number of animals | Modeling | Regimen |
|---|---|---|---|---|
| 1 | Isotype control | 6 | Each mouse was subcutaneously grafted with 5 million SK-OV-3 cells, | hIgG1DM, 10 mg/kg, administered at the tail vein, once weekly for 3 doses (the first dose was concurrently given with PBMCs intraperitoneally) |
| 2 | VP101 (hG1DM) | 6 | and intraperitoneally injected with 3 million CD3-activated PBMCs 39 days after grafting | VP101(hG1DM), 14 mg/kg, administered at the tail vein, once weekly for 3 doses (the first dose was concurrently given with PBMCs intraperitoneally) |
| 3 | Olaparib | 6 | | Olaparib, 100 mg/kg, intragastrically administered every day for 20 doses (the first dose was intraperitoneally administered alone) |
| 4 | VP101 (hG1DM) + olaparib | 6 | | VP101(hG1DM), 14 mg/kg, administered at the tail vein, once weekly for 3 doses (the first dose was concurrently given with PBMCs intraperitoneally) Olaparib, 100 mg/kg, intragastrically administered every day for 20 doses |

The results are shown in FIG. 40. The results showed that the VP101(hG1DM) and PARP inhibitor olaparib combination therapy exhibits a synergistic anti-tumor effect in the mouse ovarian cancer model compared with the olaparib monotherapy group and the VP101(hG1DM) monotherapy group, and the combination has superior tumor inhibition to the monotherapy groups.

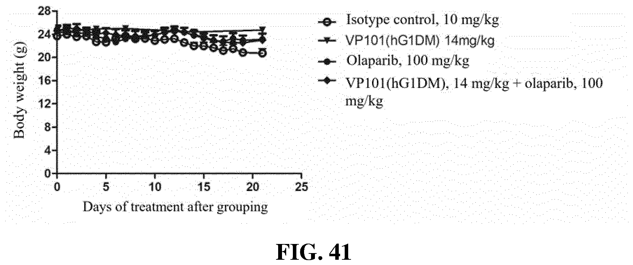

In addition, as shown in FIG. 41, both VP101(hG1DM) and PARP inhibitor olaparib were well tolerated by the tumor-bearing mice, either alone or in combination, and no effect on the body weight of the tumor-bearing mice was found in the groups.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

**Claims**

1. A therapeutic combination comprising at least one bispecific antibody and at least one PARP inhibitor,

   wherein, the bispecific antibody comprises:
   a first protein functional region targeting PD-1, and
   a second protein functional region targeting VEGFA;
   wherein:

   the first protein functional region is an immunoglobulin, and the second protein functional region is a single-chain antibody; wherein the immunoglobulin comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the single chain antibody comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively;
   or,
   the first protein functional region is a single-chain antibody, and the second protein functional region is an immunoglobulin; wherein the immunoglobulin comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively; the single chain antibody comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively;
   the immunoglobulin is of human IgG1 subtype;
   according to the EU numbering system, the immunoglobulin comprises a heavy chain constant region having mutations at any 2 or 3 of positions 234, 235 and 237, and the affinity constant of the bispecific antibody for FcγRIIIa and/or C1q is reduced after the mutation as compared to that before the mutation; preferably, the affinity constant is measured by a Fortebio Octet system.

2. The therapeutic combination according to claim 1, wherein, according to the EU numbering system, the heavy chain constant region of the immunoglobulin has the following mutations:

   L234A and L235A; or
   L234A and G237A; or
   L235A and G237A;
   or
   L234A, L235A and G237A.

3. A therapeutic combination comprising at least one bispecific antibody and at least one PARP inhibitor,

   wherein, the bispecific antibody comprises:

   a first protein functional region targeting PD-1, and
   a second protein functional region targeting VEGFA;

   wherein:

   the first protein functional region is an immunoglobulin, and the second protein functional region is a single-chain antibody; wherein the immunoglobulin comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the single chain antibody comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively;
   or,
   the first protein functional region is a single-chain antibody, and the second protein functional region is an immunoglobulin; wherein the immunoglobulin comprises a heavy chain variable region comprising HCDR1-HCDR3 having amino acid sequences set forth in SEQ ID NOs: 28-30, respectively, and a light chain variable region comprising LCDR1-LCDR3 having amino acid sequences set forth in SEQ ID NOs: 31-33, respectively; the single chain antibody comprises a heavy chain variable region comprising HCDR1-HCDR3 having amino acid sequences set forth in SEQ ID NOs: 34-36, respectively, and a light chain variable region comprising LCDR1-LCDR3 having amino acid sequences set forth in SEQ ID NOs: 37-39, respectively; the immunoglobulin is of human IgG1 subtype;

according to an EU numbering system, the immunoglobulin comprises a heavy chain constant region having the following mutations:

L234A and L235A; or
L234A and G237A; or
L235A and G237A; or
L234A, L235A and G237A.

4. The therapeutic combination according to any of claims 1 to 3, wherein, according to the EU numbering system, the heavy chain constant region of the immunoglobulin has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

5. The therapeutic combination according to any of claims 1 to 4, wherein the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence selected from SEQ ID NO: 5 and SEQ ID NO: 9, and the light chain variable region of the single chain antibody has an amino acid sequence selected from SEQ ID NO: 7, SEQ ID NO: 11 and SEQ ID NO: 17;
or,
the heavy chain variable region of the immunoglobulin has an amino acid sequence selected from SEQ ID NO: 5 and SEQ ID NO: 9, and the light chain variable region of the immunoglobulin has an amino acid sequence selected from SEQ ID NO: 7, SEQ ID NO: 11 and SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3.

6. The therapeutic combination according to any of claims 1 to 5, wherein the therapeutic combination is selected from any one of the following (1)-(12):

(1) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 7;
(2) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 11;
(3) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 5, and the light chain variable region of the single chain antibody has an amino acid sequence set forth in SEQ ID NO: 17;
(4) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 9, and the light chain variable region of the single chain antibody has an amino acid sequence set forth in SEQ ID NO: 7;
(5) the heavy chain variable region of the immunoglobulin has an amino acid sequence set forth in SEQ ID NO:

1, and the light chain variable region of the immunoglobulin has an amino acid sequence set forth in SEQ ID NO: 3; the heavy chain variable region of the single chain antibody has an amino acid sequence set forth in SEQ ID NO: 9, and the light chain variable region of the single chain antibody has an amino acid sequence set forth in SEQ ID NO: 11;

(6) the heavy chain variable region of the immunoglobulin has an amino acid sequence set forth in SEQ ID NO: 1, and the light chain variable region of the immunoglobulin has an amino acid sequence set forth in SEQ ID NO: 3; the heavy chain variable region of the single chain antibody has an amino acid sequence set forth in SEQ ID NO: 9, and the light chain variable region of the single chain antibody has an amino acid sequence set forth in SEQ ID NO: 17;

(7) the heavy chain variable region of the immunoglobulin has an amino acid sequence set forth in SEQ ID NO: 5, and the light chain variable region of the immunoglobulin has an amino acid sequence set forth in SEQ ID NO: 7; the heavy chain variable region of the single chain antibody has an amino acid sequence set forth in SEQ ID NO: 1, and the light chain variable region of the single chain antibody has an amino acid sequence set forth in SEQ ID NO: 3;

(8) the heavy chain variable region of the immunoglobulin has an amino acid sequence set forth in SEQ ID NO: 5, and the light chain variable region of the immunoglobulin has an amino acid sequence set forth in SEQ ID NO: 11; the heavy chain variable region of the single chain antibody has an amino acid sequence set forth in SEQ ID NO: 1, and the light chain variable region of the single chain antibody has an amino acid sequence set forth in SEQ ID NO: 3;

(9) the heavy chain variable region of the immunoglobulin has an amino acid sequence set forth in SEQ ID NO: 5, and the light chain variable region of the immunoglobulin has an amino acid sequence set forth in SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3;

(10) the heavy chain variable region of the immunoglobulin has an amino acid sequence set forth in SEQ ID NO: 9, and the light chain variable region of the immunoglobulin has an amino acid sequence set forth in SEQ ID NO: 7; the heavy chain variable region of the single chain antibody has an amino acid sequence set forth in SEQ ID NO: 1, and the light chain variable region of the single chain antibody has an amino acid sequence set forth in SEQ ID NO: 3;

(11) the heavy chain variable region of the immunoglobulin has an amino acid sequence set forth in SEQ ID NO: 9, and the light chain variable region of the immunoglobulin has an amino acid sequence set forth in SEQ ID NO: 11; the heavy chain variable region of the single chain antibody has an amino acid sequence set forth in SEQ ID NO: 1, and the light chain variable region of the single chain antibody has an amino acid sequence set forth in SEQ ID NO: 3; and

(12) the heavy chain variable region of the immunoglobulin has an amino acid sequence set forth in SEQ ID NO: 9, and the light chain variable region of the immunoglobulin has an amino acid sequence set forth in SEQ ID NO: 17; the heavy chain variable region of the single chain antibody has an amino acid sequence set forth in SEQ ID NO: 1, and the light chain variable region of the single chain antibody has an amino acid sequence set forth in SEQ ID NO: 3.

7. The therapeutic combination according to any of claims 1 to 6, wherein the immunoglobulin comprises a heavy chain having an amino acid sequence set forth in SEQ ID NO: 24, and a light chain having an amino acid sequence set forth in SEQ ID NO: 26.

8. The therapeutic combination according to any of claims 1 to 7, wherein the bispecific antibody or an antigen-binding fragment thereof binds to FcγRI with an affinity constant less than about $10^{-6}$ M, for example, less than about $10^{-7}$ M, $10^{-8}$ M, $10^{-9}$ M or less; preferably, the affinity constant is measured by a Fortebio Octet system.

9. The therapeutic combination according to any of claims 1 to 8, wherein the bispecific antibody or an antigen-binding fragment thereof binds to Clq with an affinity constant less than about $10^{-9}$ M, for example, less than about $10^{-7}$ M, $10^{-8}$ M, $10^{-9}$ M or less; preferably, the affinity constant is measured by a Fortebio Octet system.

10. The therapeutic combination according to any of claims 1 to 9, wherein the first protein functional region is linked to the second protein functional region either directly or via a linker fragment; and/or the heavy chain variable region of the single chain antibody is linked to the light chain variable region of the single chain antibody either directly or via a linker fragment.

11. The therapeutic combination according to claim 10, wherein the linker fragment is (GGGGS)n; n is a positive integer, and preferably, n is 1, 2, 3, 4, 5 or 6.

12. The therapeutic combination according to any of claims 1 to 11, wherein the numbers of the first protein functional region and the second protein functional region are each independently 1, 2 or more.

13. The therapeutic combination according to any of claims 1 to 12, wherein the single chain antibody is linked to the C terminus of the heavy chain of the immunoglobulin.

14. The method according to any of claims 1 to 13, wherein the heavy chain constant region of the immunoglobulin is selected from a heavy chain constant region of human IgG1, IgG2, IgG3 or IgG4, and the immunoglobulin comprises a light chain constant region selected from a light chain constant region of human IgG1, IgG2, IgG3 or IgG4;

preferably, the heavy chain constant region of the immunoglobulin is human Ig gamma-1 chain C region or human Ig gamma-4 chain C region, and the light chain constant region of the immunoglobulin is human Ig kappa chain C region;
preferably, the heavy chain constant region of the immunoglobulin is human Ig gamma-1 chain C region, ACCESSION: P01857; the light chain constant region of the immunoglobulin is human Ig kappa chain C region, ACCESSION: P01834; or
preferably, the heavy chain constant region of the immunoglobulin is human Ig gamma-4 chain C region, ACCESSION: P01861.1; the light chain constant region of the immunoglobulin is human Ig kappa chain C region, ACCESSION: P01834.

15. A therapeutic combination comprising at least one bispecific antibody and at least one PARP inhibitor, wherein, the bispecific antibody comprises:

a first protein functional region targeting PD-1, and
a second protein functional region targeting VEGFA;
the number of the first protein functional region is 1, and the number of the second protein functional region is 2;
wherein the first protein functional region is an immunoglobulin, and the second protein functional region is a single chain antibody;
the immunoglobulin comprises a heavy chain having an amino acid sequence set forth in SEQ ID NO: 24, and a light chain having an amino acid sequence set forth in SEQ ID NO: 26;
the single chain antibody comprises a heavy chain variable region having an amino acid sequence set forth in SEQ ID NO: 9, and a light chain variable region having an amino acid sequence set forth in SEQ ID NO: 17; or
the single chain antibody is linked to the C terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, amino acid sequences of the first linker fragment and the second linker fragment are independently selected from SEQ ID NO: 18 and SEQ ID NO: 19;
preferably, amino acid sequences of the first linker fragment and the second linker fragment are set forth in SEQ ID NO: 18.

16. The therapeutic combination according to any of claims 1 to 15, wherein the PARP inhibitor is selected from one or more of olaparib, rucaparib, niraparib, talazoparib, fluzoparib, veliparib ER, ABT-472, ABT-767, stenoparib, AST-6828, AG-PD, ANG-2864, ANG-3038, ANG-3186, AZD-5305, AZ-0108, AZD-2461, AMXI-5001, AMXI-2001, AMXI-3001, AMXI-7001, AMXI-9001, pamiparib, ZYTP-1, CK-102, XZ-120312, YHP-743, iobenguane I 131, rucaparib camsylate, CVL-218, CPH-101, CPH-102, CBX-11, CBX-15, minocycline, DB-207, DPS-102, E-7016, iobenguane I 131, MK-2512, HCX-014, HWH-340, IDX-1197, IDX-1197, senaparib, IMP-04100, IMP-04111, IMP-04149, IMP-04249, IMP-04307, IMP-04356, JPI-289, JPI-547, JPI-283, fluzoparib, GT-1620, iobenguane I 131, DR-2313, MP-124, H-10, NT-125, BGP-15, NMSP-293, NMSP-293, NMSP-118, NMSP-648, NMSP-914, DB-207, NUV-1156, NUV-1176, JPI-289, Stenoparib, OX-401, NU-1025, NU-1085, PLX-376, R-554, RBN-2397, RBN-012759, PJ-34, INO-1001, WW-46, BSI-401, iniparib, SOMCL-9112, SC-10914, HTMC-0435, SRX-3128, TSL-1502, PJ-34, CEP-8983, CK-102, THG-009, talazoparib SR, L-2286, mitoparib and WB-1340.

17. The therapeutic combination according to any of claims 1 to 16, further comprising one or more anti-tumor chemotherapeutics, wherein, preferably, the anti-tumor chemotherapeutic is selected from a topoisomerase II (TOP2) inhibitor, and preferably, the topoisomerase II (TOP2) inhibitor is etoposide;

> preferably, the anti-tumor chemotherapeutic is selected from a taxane, and preferably, the taxane is selected from paclitaxel, albumin-bound paclitaxel, liposome paclitaxel and docetaxel;
> preferably, the anti-tumor chemotherapeutic is selected from a platinum-based drug, preferably, the platinum-based drug is selected from cisplatin, carboplatin, and oxaliplatin;
> preferably, the anti-tumor chemotherapeutic is selected from one or more of gemcitabine, pemetrexed and capecitabine.

18. The therapeutic combination according to any of claims 1 to 17, wherein

> the therapeutic combination is a fixed combination, e.g., in the form of a solid pharmaceutical composition or a liquid pharmaceutical composition; or
> the therapeutic combination is a non-fixed combination, e.g., the bispecific antibody and the PARP inhibitor are each in the form of a pharmaceutical composition.

19. The therapeutic combination according to claim 18, wherein the pharmaceutical composition further comprises a pharmaceutically acceptable carrier and/or excipient.

20. A kit product, comprising: the therapeutic combination according to any of claims 1 to 19, and a package insert.

21. Use of the therapeutic combination according to any of claims 1 to 19 or the kit product according to claim 20 in preparing a medicament for treating and/or preventing a malignant tumor, wherein,

> preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;
> preferably, the tumor is a tumor with deficient repair function mediated by homologous recombination (e.g., a BRCA1 mutation and/or a BRCA2 mutation);
> preferably, the tumor is a tumor without deficient repair function mediated by homologous recombination;
> preferably, the lung cancer is non-small cell lung cancer or small cell lung cancer;
> preferably, the non-small cell lung cancer is a non-small cell lung cancer with EGFR and/or ALK-sensitive mutation;
> preferably, the non-small cell lung cancer is a non-small cell lung cancer without EGFR and/or ALK-sensitive mutation;
> preferably, the liver cancer is hepatocellular carcinoma;
> preferably, the renal cancer is renal cell carcinoma;
> preferably, the breast cancer is triple negative breast cancer;
> preferably, the urothelial carcinoma is bladder cancer;
> preferably, the peritoneal cancer is primary peritoneal cancer;
> preferably, the ovarian cancer is an ovarian cancer with deficient repair function mediated by homologous recombination (e.g., a BRCA1 mutation and/or a BRCA2 mutation);
> preferably, the fallopian tube cancer is a fallopian tube cancer with deficient repair function mediated by homologous recombination (e.g., a BRCA1 mutation and/or a BRCA2 mutation);
> preferably, the peritoneal cancer is a peritoneal cancer with deficient repair function mediated by homologous recombination (e.g., a BRCA1 mutation and/or a BRCA2 mutation);
> preferably, the breast cancer is a breast cancer with deficient repair function mediated by homologous recombination (e.g., a BRCA1 mutation and/or a BRCA2 mutation).

22. The therapeutic combination according to any of claims 1 to 19 or the kit product according to claim 20 for use in treating and/or preventing a malignant tumor, wherein,

> preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;

preferably, the tumor is a tumor with deficient repair function mediated by homologous recombination (e.g., a BRCA1 mutation and/or a BRCA2 mutation);

preferably, the tumor is a tumor without deficient repair function mediated by homologous recombination;

preferably, the lung cancer is non-small cell lung cancer or small cell lung cancer;

preferably, the non-small cell lung cancer is a non-small cell lung cancer with EGFR and/or ALK-sensitive mutation;

preferably, the non-small cell lung cancer is a non-small cell lung cancer without EGFR and/or ALK-sensitive mutation;

preferably, the liver cancer is hepatocellular carcinoma;

preferably, the renal cancer is renal cell carcinoma;

preferably, the breast cancer is triple negative breast cancer;

preferably, the urothelial carcinoma is bladder cancer;

preferably, the peritoneal cancer is primary peritoneal cancer;

preferably, the ovarian cancer is an ovarian cancer with deficient repair function mediated by homologous recombination (e.g., a BRCA1 mutation and/or a BRCA2 mutation);

preferably, the fallopian tube cancer is a fallopian tube cancer with deficient repair function mediated by homologous recombination (e.g., a BRCA1 mutation and/or a BRCA2 mutation);

preferably, the peritoneal cancer is a peritoneal cancer with deficient repair function mediated by homologous recombination (e.g., a BRCA1 mutation and/or a BRCA2 mutation);

preferably, the breast cancer is a breast cancer with deficient repair function mediated by homologous recombination (e.g., a BRCA1 mutation and/or a BRCA2 mutation).

23. A method for treating and/or preventing a malignant tumor, comprising: administering to a subject in need an effective amount of the therapeutic combination according to any of claims 1 to 19 or the kit product according to claim 20, wherein,

preferably, the malignant tumor is selected from ovarian cancer, endometrial cancer, breast cancer, cervical cancer, fallopian tube cancer, peritoneal cancer, pancreatic cancer, colon cancer, rectal cancer, lung cancer, liver cancer, skin cancer, glioma, melanoma, lymphoma, renal tumor, prostate cancer, bladder cancer, gastrointestinal cancer, brain cancer, oesophageal cancer such as oesophageal squamous cancer, microsatellite instability-high (MSI-H) or mismatch repair deficient (dMMR) cancer, urothelial carcinoma, mesothelioma, gastric adenocarcinoma, gastroesophageal junction adenocarcinoma, leukemia, myeloma, thyroid cancer, head and neck cancer, bone cancer, biliary tract cancer and testicular cancer;

preferably, the tumor is a tumor with deficient repair function mediated by homologous recombination (e.g., a BRCA1 mutation and/or a BRCA2 mutation);

preferably, the tumor is a tumor without deficient repair function mediated by homologous recombination;

preferably, the lung cancer is non-small cell lung cancer or small cell lung cancer;

preferably, the non-small cell lung cancer is a non-small cell lung cancer with EGFR and/or ALK-sensitive mutation;

preferably, the non-small cell lung cancer is a non-small cell lung cancer without EGFR and/or ALK-sensitive mutation;

preferably, the liver cancer is hepatocellular carcinoma;

preferably, the renal cancer is renal cell carcinoma;

preferably, the breast cancer is triple negative breast cancer;

preferably, the urothelial carcinoma is bladder cancer;

preferably, the peritoneal cancer is primary peritoneal cancer;

preferably, the ovarian cancer is an ovarian cancer with deficient repair function mediated by homologous recombination (e.g., a BRCA1 mutation and/or a BRCA2 mutation);

preferably, the fallopian tube cancer is a fallopian tube cancer with deficient repair function mediated by homologous recombination (e.g., a BRCA1 mutation and/or a BRCA2 mutation);

preferably, the peritoneal cancer is a peritoneal cancer with deficient repair function mediated by homologous recombination (e.g., a BRCA1 mutation and/or a BRCA2 mutation);

preferably, the breast cancer is a breast cancer with deficient repair function mediated by homologous recombination (e.g., a BRCA1 mutation and/or a BRCA2 mutation).

24. The method according to claim 23, wherein the administration is performed before or after surgery and/or before or after radiotherapy.

25. The method according to any of claims 23 to 24, wherein

the bispecific antibody is administered at a unit dose of 0.1-100 mg per kg body weight, preferably 5-50 mg or 5-15 mg per kg body weight,
administered once every 3 days, 4 days, 5 days, 6 days, 10 days, 1 week, 2 weeks or 3 weeks,
and/or
administered by intravenous drip infusion or intravenous injection.

26. The method according to any of claims 23 to 25, wherein

the PARP inhibitor is administered at a unit dose of 0.1-100 mg per kg body weight, preferably 5-50 mg or 5-15 mg per kg body weight,
administered once every 3 days, 4 days, 5 days, 6 days, 10 days, 1 week, 2 weeks or 3 weeks,
and/or
administered by intravenous drip infusion or intravenous injection.

27. Use of the bispecific antibody in the therapeutic combination according to any of claims 1 to 19 in preparing a medicament for treating and/or preventing a malignant tumor, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

28. A method for treating and/or preventing a malignant tumor, comprising: administering to a subject in need an effective amount of the bispecific antibody in the therapeutic combination according to any of claims 1 to 19, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

29. The bispecific antibody in the therapeutic combination according to any of claims 1 to 19 for use in treating and/or preventing a malignant tumor, wherein the malignant tumor is selected from ovarian cancer, fallopian tube cancer, peritoneal cancer and breast cancer;

preferably, the ovarian cancer is an ovarian cancer with deficient repair function mediated by homologous recombination (e.g., a BRCA1 mutation and/or a BRCA2 mutation);
preferably, the fallopian tube cancer is a fallopian tube cancer with deficient repair function mediated by homologous recombination (e.g., a BRCA1 mutation and/or a BRCA2 mutation);
preferably, the peritoneal cancer is a peritoneal cancer with deficient repair function mediated by homologous recombination (e.g., a BRCA1 mutation and/or a BRCA2 mutation);
preferably, the breast cancer is a breast cancer with deficient repair function mediated by homologous recombination (e.g., a BRCA1 mutation and/or a BRCA2 mutation).

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

**FIG. 17**

**FIG. 18**

**FIG. 19**

**FIG. 20**

**FIG. 21**

**FIG. 22**

**FIG. 23**

**FIG. 24**

**FIG. 25**

**FIG. 26**

**FIG. 27**

**FIG. 28**

**FIG. 29**

**FIG. 30**

**FIG. 31**

**FIG. 32**

**FIG. 33**

**FIG. 34**

**FIG. 35**

**FIG. 36**

**FIG. 37**

**FIG. 38**

**FIG. 39**

**FIG. 40**

**FIG. 41**

**INTERNATIONAL SEARCH REPORT**

| International application No. |
| --- |
| **PCT/CN2022/080107** |

**A. CLASSIFICATION OF SUBJECT MATTER**

C07K 16/46(2006.01)i; C07K 16/30(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, CNKI, Web of Science, 百度学术, BAIDU SCHOLAR, Patentics, 中国生物序列检索系统, China Patents Biological Sequence Search System, NCBI, EBI, STNext: 申请人, 发明人, 抗体, 双特异, antibody, bispecific, PD-1, 程序性细胞死亡受体1, Programmed Cell Death 1, 血管内皮生长因子, VEGF, vascular endothelial growth factor, 聚ADP核糖聚合酶, PARP, 抑制剂, inhibitor, 序列1-42

**C. DOCUMENTS CONSIDERED TO BE RELEVANT**

| Category* | Citation of document, with indication, where appropriate, of the relevant passages | Relevant to claim No. |
| --- | --- | --- |
| PY | CN 112830972 A (AKESO BIOPHARMA, INC.) 25 May 2021 (2021-05-25) claims 1-22 | 1-29 |
| Y | CN 109053895 A (AKESO BIOPHARMA, INC.) 21 December 2018 (2018-12-21) claims 1-23, and description, paragraphs 22-168 | 1-29 |
| Y | CN 112300286 A (AKESO BIOPHARMA, INC.) 02 February 2021 (2021-02-02) claims 1-28 | 1-29 |
| Y | CN 110831580 A (TESARO INC. et al.) 21 February 2020 (2020-02-21) claims 46-93 | 1-29 |
| A | WO 2019152642 A1 (MERCK SHARP & DOHME CORP. et al.) 08 August 2019 (2019-08-08) entire document | 1-29 |

☑ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 April 2022** | **28 April 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/080107**

**C.    DOCUMENTS CONSIDERED TO BE RELEVANT**

| Category* | Citation of document, with indication, where appropriate, of the relevant passages | Relevant to claim No. |
|---|---|---|
| A | 崔语恒 等 (CUI, Yuheng et al.). "PD-1/PD-L1抑制剂联合其他免疫检查点抑制剂治疗三阴性乳腺癌的研究进展. (Research Progress of PD-1/PD-L1 Inhibitors Combined with Other Immune Checkpoint Inhibitors in the Treatment of Triple Negative Breast Cancer.)" 天津医药. *(Tianjin Medical Journal.)*, Vol. 48, No. 12, 31 December 2020 (2020-12-31), pp. 1230-1235 | 1-29 |
| A | GHONIM, M. A. et al. "Low Doses of PARP Inhibitors As A novel Therapeutic Approach to Enhance The Anti-Cancer Immunotherapy of PD-1 Immune Checkpoint Blockade." *THE FASEB JOURNAL.*, Vol. 33, No. S1, 01 April 2019 (2019-04-01), p. 680.15 | 1-29 |

Form PCT/ISA/210 (second sheet) (January 2015)

<table>
<tr><td>**INTERNATIONAL SEARCH REPORT**</td><td>International application No.<br>**PCT/CN2022/080107**</td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

          ☑ in the form of an Annex C/ST.25 text file.

          ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

          ☐ in the form of an Annex C/ST.25 text file (Rule 13*ter*.1(a)).

          ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
|---|
| **PCT/CN2022/080107** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **23-26、 28**
   because they relate to subject matter not required to be searched by this Authority, namely:

   [1]   Claims 23-26 and 28 relate to a method for treating and/or preventing malignant tumors, which falls within the subject matter as defined in PCT Rule 39.1(iv) that does not warrant a search conducted by the international searching authority. However, the search is made on the basis of a use of the pharmaceutical composition according to any one of claims 1-19 or a bispecific antibody therein, or the kit product according to claim 20 in the preparation of a drug for treating and/or preventing malignant tumors.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/080107**

| Patent document cited in search report | | | Publication date (day/month/year) | Patent family member(s) | | | Publication date (day/month/year) |
|---|---|---|---|---|---|---|---|
| CN | 112830972 | A | 25 May 2021 | WO | 2021104302 | A1 | 03 June 2021 |
| CN | 109053895 | A | 21 December 2018 | AU | 2019332708 | A1 | 15 April 2021 |
| | | | | SG | 11202101985 T | A | 29 April 2021 |
| | | | | US | 2021340239 | A1 | 04 November 2021 |
| | | | | JP | 2021536242 | A | 27 December 2021 |
| | | | | PH | 12021550407 | A1 | 20 September 2021 |
| | | | | CA | 3110749 | A1 | 05 March 2020 |
| | | | | BR | 112021003559 | A2 | 25 May 2021 |
| | | | | EA | 202190535 | A1 | 20 July 2021 |
| | | | | KR | 20210053912 | A | 12 May 2021 |
| | | | | WO | 2020043184 | A1 | 05 March 2020 |
| | | | | IL | 281116 | D0 | 29 April 2021 |
| | | | | EP | 3882275 | A1 | 22 September 2021 |
| CN | 112300286 | A | 02 February 2021 | WO | 2021023117 | A1 | 11 February 2021 |
| | | | | AU | 2020325350 | A1 | 03 March 2022 |
| | | | | CA | 3146780 | A1 | 11 February 2021 |
| CN | 110831580 | A | 21 February 2020 | AU | 2021218080 | A1 | 09 September 2021 |
| | | | | AU | 2018264992 | A1 | 05 December 2019 |
| | | | | JP | 2020519621 | A | 02 July 2020 |
| | | | | TW | 201906632 | A | 16 February 2019 |
| | | | | US | 2020289493 | A1 | 17 September 2020 |
| | | | | MA | 48637 | A | 17 March 2021 |
| | | | | CA | 3063201 | A1 | 15 November 2018 |
| | | | | KR | 20200018436 | A | 19 February 2020 |
| | | | | EP | 3621592 | A1 | 18 March 2020 |
| | | | | BR | 112019023591 | A2 | 26 May 2020 |
| | | | | WO | 2018208968 | A1 | 15 November 2018 |
| WO | 2019152642 | A1 | 08 August 2019 | JP | 2021511791 | A | 13 May 2021 |
| | | | | US | 2021363256 | A1 | 25 November 2021 |
| | | | | US | 2019233518 | A1 | 01 August 2019 |
| | | | | EP | 3746481 | A1 | 09 December 2020 |
| | | | | AU | 2019215013 | A1 | 20 August 2020 |
| | | | | US | 2021371530 | A1 | 02 December 2021 |
| | | | | CN | 111836829 | A | 27 October 2020 |
| | | | | CA | 3089721 | A1 | 08 August 2019 |
| | | | | WO | 2019148412 | A1 | 08 August 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5747654 A
- US 4816567 A
- CN 1259962 A
- CN 106967172 A

**Non-patent literature cited in the description**

- **MA LI.** *Chinese Journal of Birth Health and Heredity,* 2016, vol. 24 (5), 146-148
- **ROSKOSKI R JR. et al.** *Crit Rev Oncol Hematol,* 2007, vol. 62 (3), 179-213
- **TAKAHASHI T et al.** *Oncogene,* 1999, vol. 18 (13), 2221-2230
- **DONG HONGCHAO et al.** *Journal of Modern Oncology,* September 2014, vol. 22 (9), 2231-3
- **HOMET M. B. ; PARISI G. et al.** Anti-PD-1 Therapy in Melanoma. *Semin Oncol.,* June 2015, vol. 42 (3), 466-473
- **HELD S.A. ; HEINE A. et al.** Advances in immunology of biliary muscular tissue CML. *Curr. Cancer Drug Targets,* September 2013, vol. 13 (7), 768-74
- **JOYCE F. LIU et al.** *JAMA Oncol.,* 2019, vol. 5 (12), 1731-1738
- **MANEGOLD C et al.** *J Thorac Oncol.,* February 2017, vol. 12 (2), 194-207
- **DUDEK AZ et al.** *J Clin Oncol.,* 2018, vol. 36
- **STEIN S et al.** *J Clin Oncol,* 2018, vol. 36 (15), 4074
- **BENDELL JC et al.** *American Society of Clinical Oncology,* 29 May 2015
- **HOCHSTER HS et al.** *American Society of Clinical Oncology Gastrointestinal Cancers Symposium,* 19 January 2017
- **YUKINORI OZAKI et al.** *Cancer Res,* 2020, vol. 80 (4
- **HERBST RS et al.** *Lancet Oncol.,* 2019, vol. 20 (8), 1109-1123
- **KRISHNANSU S. et al.** *N Engl J Med,* 2014, vol. 370, 734-743
- **ANTONARAKIS ES et al.** *J Clin Oncol.,* 10 February 2020, vol. 38 (5), 395-405
- **JOAQUIM BELLMUNT et al.** *N Engl J Med,* 2017, vol. 376, 1015-1026
- **KATO K et al.** *Lancet Oncol.,* 2019, vol. 20 (11), 1506-17
- **SCHERPEREEL A et al.** *Lancet Oncol.,* 2019, vol. 20 (2), 239-253
- **LORD CJ ; ASHWORTH A.** *Science,* 2017, vol. 355 (6330), 1152-1158
- **TURNER NC.** *N Engl J Med.,* 2017, vol. 377 (25), 2490-2492
- **J. MATEO et al.** *Ann Oncol.,* 01 September 2019, vol. 30 (9), 1437-1447
- **MIRZA MR et al.** *N Engl J Med,* 2016, vol. 375 (22), 2154-2164
- **MÜLLER D ; KONTERMANN RE.** Bispecific antibodies for cancer immunotherapy: current perspectives. *BioDrugs,* 2010, vol. 24, 89-98
- **COLOMA M. J. ; MORRISON S. L.** Design and production of novel tetravalent bispecific antibodies. *Nat Biotechnol.,* 1997, vol. 15, 159-163
- **MILLER B. R. ; DEMAREST S. J. et al.** *Protein Eng Des Sel,* 2010, vol. 23, 549-57
- **FITZGERALD J ; LUGOVSKOY A.** *MAbs,* 2011, vol. 3, 299-309
- **MATEO J et al.** *Ann Oncol.,* 2019, vol. 30 (9), 1437-1447
- **JIAO S et al.** *Clin Cancer Res.,* 2017, vol. 23 (14), 3711-3720
- **RAJAGOPAL et al.** *Prot. Engin.,* 1997, vol. 10, 1453-1459
- **REITER et al.** *Nat. Biotechnol.,* 1996, vol. 14, 1239-1245
- **REITER et al.** *Protein Engineering,* 1995, vol. 8, 1323-1331
- **WEBBER et al.** *Molecular Immunology,* 1995, vol. 32, 249-258
- **REITER et al.** *Immunity,* 1995, vol. 2, 281-287
- **REITER et al.** *JBC,* 1994, vol. 269, 18327-18331
- **REITER et al.** *Inter. J. of Cancer,* 1994, vol. 58, 142-149
- **REITER et al.** *Cancer Res.,* 1994, vol. 54, 2714-2718
- **KABAT et al.** Sequences of Proteins of Immunological Interest. 1991, vol. 1-3, 91-3242
- **EHRENMANN, FRANCOIS ; QUENTIN KAAS ; MARIE-PAULE LEFRANC.** IMGT/3Dstructure-DB and IMGT/DomainGapAlign: a database and a tool for immunoglobulins or antibodies, T cell receptors, MHC, IgSF and MhcSF. *Nucleic acids research,* 2009, vol. 38 (1), D301-D307

- Kabat Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883
- **EHRENMANN, FRANCOIS ; QUENTIN KAAS ; MARIE-PAULE LEFRANC.** IMGT/3Dstructure-DB and IMGT/DomainGapAlign: a database and a tool for immunoglobulins or antibodies, T cell receptors, MHC, IgSF and MhcSF. *Nucleic acids research,* 2009, vol. 38 (1), D301-D307
- Fundamental Immunology. Raven Press, 1989
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **HOLLIGER P. et al.** *Proc. Natl. Acad. Sci. USA,* 1993, vol. 90, 6444-6448
- **POLJAK R.J. et al.** *Structure,* 1994, vol. 2, 1121-1123
- **KOHLER et al.** *Nature,* 1975, vol. 256, 495
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402
- Epitope Mapping Protocols. Methods in Molecular Biology. 1996, vol. 66
- Remington's Pharmaceutical Sciences. Mack Publishing Company, 1995
- **J. SAMBROOK et al.** Guide to Molecular Cloning Experiments. Science Press
- **ACIERNO et al.** Affinity maturation increases the stability and plasticity of the Fv domain of anti-protein antibodies. *J Mol Biol.,* 2007, vol. 374 (1), 130-46